(19) Europäisches Patentamt / European Patent Office / Office européen des brevets

(11) **EP 3 560 505 B1**

(12) **EUROPEAN PATENT SPECIFICATION**

(45) Date of publication and mention
of the grant of the patent:
**13.10.2021 Bulletin 2021/41**

(21) Application number: **17877435.2**

(22) Date of filing: **06.12.2017**

(51) Int Cl.:
*A61K 35/76* (2015.01)       *A61K 39/395* (2006.01)
*A61K 45/00* (2006.01)       *A61P 3/10* (2006.01)
*A61P 9/00* (2006.01)       *A61P 21/00* (2006.01)
*A61P 25/00* (2006.01)       *A61P 25/16* (2006.01)
*A61P 25/28* (2006.01)       *A61P 27/02* (2006.01)
*A61P 43/00* (2006.01)

(86) International application number:
**PCT/JP2017/043725**

(87) International publication number:
**WO 2018/105630 (14.06.2018 Gazette 2018/24)**

(54) **NOVEL THERAPEUTIC AGENT FOR PRIONOID DISEASES**

NEUARTIGES THERAPEUTIKUM FÜR PRIONOIDE ERKRANKUNGEN

NOUVEL AGENT THÉRAPEUTIQUE POUR MALADIES DE TYPE PRIONOÏDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.12.2016 JP 2016236536**

(43) Date of publication of application:
**30.10.2019 Bulletin 2019/44**

(73) Proprietors:
• **Osaka University**
**Suita-shi, Osaka 565-0871 (JP)**
• **Foundation for Biomedical Research and
Innovation
at Kobe
Kobe-shi,
Hyogo 650-0047 (JP)**
• **GenomIdea Inc.**
**Osaka-shi, Osaka 550-0002 (JP)**

(72) Inventors:
• **KANEDA Yasufumi**
**Suita-shi**
**Osaka 565-0871 (JP)**

• **FUKUSHIMA Masanori**
**Kobe-shi**
**Hyogo 650-004 (JP)**
• **NAKAJIMA Toshihiro**
**Ikeda-shi**
**Osaka 563-8577 (JP)**

(74) Representative: **J A Kemp LLP**
**14 South Square
Gray's Inn
London WC1R 5JJ (GB)**

(56) References cited:
EP-A1- 1 449 542       EP-A1- 3 536 329
WO-A1-2006/011600       WO-A2-2015/136541

• YOSHIMURA S-I ET AL: "Gene transfer of
hepatocyte growth factor to subarachnoid space
in cerebral hypoperfusion model",
HYPERTENSION, LIPPINCOTT WILLIAMS &
WILKINS, US, vol. 39, no. 5, 1 May 2002
(2002-05-01), pages 1028-1034, XP002351609,
ISSN: 0194-911X, DOI:
10.1161/01.HYP.0000017553.67732.E1

SEQUENCE LISTING

<110> Osaka University GenomIdea Inc. Foundation for Biomedical Research and Innovation

<120> Novel Therapeutic Agent for Prionoid Disease

<130> PS86-9001WO

<150> JP2016-236536
<151> 2016-12-06

<160> 32

<170> PatentIn version 3.5

<210> 1
<211> 867
<212> DNA
<213> Homo sapiens

<220>
<221> CDS
<222> (1)..(867)

<400> 1

```
atg cag atc cca cag gcg ccc tgg cca gtc gtc tgg gcg gtg cta caa        48
Met Gln Ile Pro Gln Ala Pro Trp Pro Val Val Trp Ala Val Leu Gln
1               5                   10                  15

ctg ggc tgg cgg cca gga tgg ttc tta gac tcc cca gac agg ccc tgg        96
Leu Gly Trp Arg Pro Gly Trp Phe Leu Asp Ser Pro Asp Arg Pro Trp
            20                  25                  30

aac ccc ccc acc ttc tcc cca gcc ctg ctc gtg gtg acc gaa ggg gac       144
Asn Pro Pro Thr Phe Ser Pro Ala Leu Leu Val Val Thr Glu Gly Asp
        35                  40                  45

aac gcc acc ttc acc tgc agc ttc tcc aac aca tcg gag agc ttc gtg       192
Asn Ala Thr Phe Thr Cys Ser Phe Ser Asn Thr Ser Glu Ser Phe Val
    50                  55                  60

cta aac tgg tac cgc atg agc ccc agc aac cag acg gac aag ctg gcc       240
Leu Asn Trp Tyr Arg Met Ser Pro Ser Asn Gln Thr Asp Lys Leu Ala
65                  70                  75                  80

gcc ttc ccc gag gac cgc agc cag ccc ggc cag gac tgc cgc ttc cgt       288
Ala Phe Pro Glu Asp Arg Ser Gln Pro Gly Gln Asp Cys Arg Phe Arg
                85                  90                  95

gtc aca caa ctg ccc aac ggg cgt gac ttc cac atg agc gtg gtc agg       336
Val Thr Gln Leu Pro Asn Gly Arg Asp Phe His Met Ser Val Val Arg
            100                 105                 110

gcc cgg cgc aat gac agc ggc acc tac ctc tgt ggg gcc atc tcc ctg       384
Ala Arg Arg Asn Asp Ser Gly Thr Tyr Leu Cys Gly Ala Ile Ser Leu
        115                 120                 125

gcc ccc aag gcg cag atc aaa gag agc ctg cgg gca gag ctc agg gtg       432
Ala Pro Lys Ala Gln Ile Lys Glu Ser Leu Arg Ala Glu Leu Arg Val
    130                 135                 140
```

```
aca gag aga agg gca gaa gtg ccc aca gcc cac ccc agc ccc tca ccc        480
Thr Glu Arg Arg Ala Glu Val Pro Thr Ala His Pro Ser Pro Ser Pro
145             150             155             160

agg cca gcc ggc cag ttc caa acc ctg gtg gtt ggt gtc gtg ggc ggc        528
Arg Pro Ala Gly Gln Phe Gln Thr Leu Val Val Gly Val Val Gly Gly
            165             170             175

ctg ctg ggc agc ctg gtg ctg cta gtc tgg gtc ctg gcc gtc atc tgc        576
Leu Leu Gly Ser Leu Val Leu Leu Val Trp Val Leu Ala Val Ile Cys
            180             185             190

tcc cgg gcc gca cga ggg aca ata gga gcc agg cgc acc ggc cag ccc        624
Ser Arg Ala Ala Arg Gly Thr Ile Gly Ala Arg Arg Thr Gly Gln Pro
            195             200             205

ctg aag gag gac ccc tca gcc gtg cct gtg ttc tct gtg gac tat ggg        672
Leu Lys Glu Asp Pro Ser Ala Val Pro Val Phe Ser Val Asp Tyr Gly
            210             215             220

gag ctg gat ttc cag tgg cga gag aag acc ccg gag ccc ccc gtg ccc        720
Glu Leu Asp Phe Gln Trp Arg Glu Lys Thr Pro Glu Pro Pro Val Pro
225             230             235             240

tgt gtc cct gag cag acg gag tat gcc acc att gtc ttt cct agc gga        768
Cys Val Pro Glu Gln Thr Glu Tyr Ala Thr Ile Val Phe Pro Ser Gly
            245             250             255

atg ggc acc tca tcc ccc gcc cgc agg ggc tca gct gac ggc cct cgg        816
Met Gly Thr Ser Ser Pro Ala Arg Arg Gly Ser Ala Asp Gly Pro Arg
            260             265             270

agt gcc cag cca ctg agg cct gag gat gga cac tgc tct tgg ccc ctc        864
Ser Ala Gln Pro Leu Arg Pro Glu Asp Gly His Cys Ser Trp Pro Leu
            275             280             285

tga                                                                    867
```

<210> 2
<211> 288
<212> PRT
<213> Homo sapiens

<400> 2

```
Met Gln Ile Pro Gln Ala Pro Trp Pro Val Val Trp Ala Val Leu Gln
1               5               10              15

Leu Gly Trp Arg Pro Gly Trp Phe Leu Asp Ser Pro Asp Arg Pro Trp
                20              25              30

Asn Pro Pro Thr Phe Ser Pro Ala Leu Leu Val Val Thr Glu Gly Asp
                35              40              45

Asn Ala Thr Phe Thr Cys Ser Phe Ser Asn Thr Ser Glu Ser Phe Val
                50              55              60
```

```
Leu Asn Trp Tyr Arg Met Ser Pro Ser Asn Gln Thr Asp Lys Leu Ala
65              70              75              80


Ala Phe Pro Glu Asp Arg Ser Gln Pro Gly Gln Asp Cys Arg Phe Arg
            85              90              95


Val Thr Gln Leu Pro Asn Gly Arg Asp Phe His Met Ser Val Val Arg
        100             105             110


Ala Arg Arg Asn Asp Ser Gly Thr Tyr Leu Cys Gly Ala Ile Ser Leu
    115             120             125


Ala Pro Lys Ala Gln Ile Lys Glu Ser Leu Arg Ala Glu Leu Arg Val
    130             135             140


Thr Glu Arg Arg Ala Glu Val Pro Thr Ala His Pro Ser Pro Ser Pro
145             150             155             160


Arg Pro Ala Gly Gln Phe Gln Thr Leu Val Val Gly Val Val Gly Gly
            165             170             175


Leu Leu Gly Ser Leu Val Leu Leu Val Trp Val Leu Ala Val Ile Cys
        180             185             190


Ser Arg Ala Ala Arg Gly Thr Ile Gly Ala Arg Arg Thr Gly Gln Pro
    195             200             205


Leu Lys Glu Asp Pro Ser Ala Val Pro Val Phe Ser Val Asp Tyr Gly
    210             215             220


Glu Leu Asp Phe Gln Trp Arg Glu Lys Thr Pro Glu Pro Pro Val Pro
225             230             235             240


Cys Val Pro Glu Gln Thr Glu Tyr Ala Thr Ile Val Phe Pro Ser Gly
            245             250             255


Met Gly Thr Ser Ser Pro Ala Arg Arg Gly Ser Ala Asp Gly Pro Arg
        260             265             270


Ser Ala Gln Pro Leu Arg Pro Glu Asp Gly His Cys Ser Trp Pro Leu
        275             280             285
```

<210> 3
<211> 531
<212> DNA
<213> Homo sapiens

<220>

<221> CDS
<222> (1)..(531)

<400> 3

```
atg agg ata ttt gct gtc ttt ata ttc atg acc tac tgg cat ttg ctg       48
Met Arg Ile Phe Ala Val Phe Ile Phe Met Thr Tyr Trp His Leu Leu
1               5                   10                  15

aac gcc cca tac aac aaa atc aac caa aga att ttg gtt gtg gat cca       96
Asn Ala Pro Tyr Asn Lys Ile Asn Gln Arg Ile Leu Val Val Asp Pro
                20                  25                  30

gtc acc tct gaa cat gaa ctg aca tgt cag gct gag ggc tac ccc aag      144
Val Thr Ser Glu His Glu Leu Thr Cys Gln Ala Glu Gly Tyr Pro Lys
            35                  40                  45

gcc gaa gtc atc tgg aca agc agt gac cat caa gtc ctg agt ggt aag      192
Ala Glu Val Ile Trp Thr Ser Ser Asp His Gln Val Leu Ser Gly Lys
        50                  55                  60

acc acc acc acc aat tcc aag aga gag gag aag ctt ttc aat gtg acc      240
Thr Thr Thr Thr Asn Ser Lys Arg Glu Glu Lys Leu Phe Asn Val Thr
65                  70                  75                  80

agc aca ctg aga atc aac aca aca act aat gag att ttc tac tgc act      288
Ser Thr Leu Arg Ile Asn Thr Thr Thr Asn Glu Ile Phe Tyr Cys Thr
                85                  90                  95

ttt agg aga tta gat cct gag gaa aac cat aca gct gaa ttg gtc atc      336
Phe Arg Arg Leu Asp Pro Glu Glu Asn His Thr Ala Glu Leu Val Ile
                100                 105                 110

cca gaa cta cct ctg gca cat cct cca aat gaa agg act cac ttg gta      384
Pro Glu Leu Pro Leu Ala His Pro Pro Asn Glu Arg Thr His Leu Val
            115                 120                 125

att ctg gga gcc atc tta tta tgc ctt ggt gta gca ctg aca ttc atc      432
Ile Leu Gly Ala Ile Leu Leu Cys Leu Gly Val Ala Leu Thr Phe Ile
        130                 135                 140

ttc cgt tta aga aaa ggg aga atg atg gat gtg aaa aaa tgt ggc atc      480
Phe Arg Leu Arg Lys Gly Arg Met Met Asp Val Lys Lys Cys Gly Ile
145                 150                 155                 160

caa gat aca aac tca aag aag caa agt gat aca cat ttg gag gag acg      528
Gln Asp Thr Asn Ser Lys Lys Gln Ser Asp Thr His Leu Glu Glu Thr
                165                 170                 175

taa                                                                  531
```

<210> 4
<211> 176
<212> PRT
<213> Homo sapiens

<400> 4

```
Met Arg Ile Phe Ala Val Phe Ile Phe Met Thr Tyr Trp His Leu Leu
1               5                   10              15

Asn Ala Pro Tyr Asn Lys Ile Asn Gln Arg Ile Leu Val Val Asp Pro
            20                  25                  30

Val Thr Ser Glu His Glu Leu Thr Cys Gln Ala Glu Gly Tyr Pro Lys
        35                  40                  45

Ala Glu Val Ile Trp Thr Ser Ser Asp His Gln Val Leu Ser Gly Lys
        50                  55                  60

Thr Thr Thr Thr Asn Ser Lys Arg Glu Glu Lys Leu Phe Asn Val Thr
65                  70                  75                  80

Ser Thr Leu Arg Ile Asn Thr Thr Thr Asn Glu Ile Phe Tyr Cys Thr
                85                  90                  95

Phe Arg Arg Leu Asp Pro Glu Glu Asn His Thr Ala Glu Leu Val Ile
            100                 105                 110

Pro Glu Leu Pro Leu Ala His Pro Pro Asn Glu Arg Thr His Leu Val
            115                 120                 125

Ile Leu Gly Ala Ile Leu Leu Cys Leu Gly Val Ala Leu Thr Phe Ile
        130                 135                 140

Phe Arg Leu Arg Lys Gly Arg Met Met Asp Val Lys Lys Cys Gly Ile
145                 150                 155                 160

Gln Asp Thr Asn Ser Lys Lys Gln Ser Asp Thr His Leu Glu Glu Thr
                165                 170                 175
```

<210> 5
<211> 822
<212> DNA
<213> Homo sapiens

<220>
<221> CDS
<222> (1)..(822)

<400> 5

```
atg atc ttc ctc ctg cta atg ttg agc ctg gaa ttg cag ctt cac cag        48
Met Ile Phe Leu Leu Leu Met Leu Ser Leu Glu Leu Gln Leu His Gln
1               5                   10                  15

ata gca gct tta ttc aca gtg aca gtc cct aag gaa ctg tac ata ata        96
Ile Ala Ala Leu Phe Thr Val Thr Val Pro Lys Glu Leu Tyr Ile Ile
            20                  25                  30

gag cat ggc agc aat gtg acc ctg gaa tgc aac ttt gac act gga agt       144
Glu His Gly Ser Asn Val Thr Leu Glu Cys Asn Phe Asp Thr Gly Ser
        35                  40                  45

cat gtg aac ctt gga gca ata aca gcc agt ttg caa aag gtg gaa aat       192
```

```
                                      His Val Asn Leu Gly Ala Ile Thr Ala Ser Leu Gln Lys Val Glu Asn
                                          50                  55                  60

gat aca tcc cca cac cgt gaa aga gcc act ttg ctg gag gag cag ctg       240
Asp Thr Ser Pro His Arg Glu Arg Ala Thr Leu Leu Glu Glu Gln Leu
65                  70                  75                  80

ccc cta ggg aag gcc tcg ttc cac ata cct caa gtc caa gtg agg gac       288
Pro Leu Gly Lys Ala Ser Phe His Ile Pro Gln Val Gln Val Arg Asp
                    85                  90                  95

gaa gga cag tac caa tgc ata atc atc tat ggg gtc gcc tgg gac tac       336
Glu Gly Gln Tyr Gln Cys Ile Ile Ile Tyr Gly Val Ala Trp Asp Tyr
                100                 105                 110

aag tac ctg act ctg aaa gtc aaa gct tcc tac agg aaa ata aac act       384
Lys Tyr Leu Thr Leu Lys Val Lys Ala Ser Tyr Arg Lys Ile Asn Thr
            115                 120                 125

cac atc cta aag gtt cca gaa aca gat gag gta gag ctc acc tgc cag       432
His Ile Leu Lys Val Pro Glu Thr Asp Glu Val Glu Leu Thr Cys Gln
        130                 135                 140

gct aca ggt tat cct ctg gca gaa gta tcc tgg cca aac gtc agc gtt       480
Ala Thr Gly Tyr Pro Leu Ala Glu Val Ser Trp Pro Asn Val Ser Val
145                 150                 155                 160

cct gcc aac acc agc cac tcc agg acc cct gaa ggc ctc tac cag gtc       528
Pro Ala Asn Thr Ser His Ser Arg Thr Pro Glu Gly Leu Tyr Gln Val
                    165                 170                 175

acc agt gtt ctg cgc cta aag cca ccc cct ggc aga aac ttc agc tgt       576
Thr Ser Val Leu Arg Leu Lys Pro Pro Pro Gly Arg Asn Phe Ser Cys
                180                 185                 190

gtg ttc tgg aat act cac gtg agg gaa ctt act ttg gcc agc att gac       624
Val Phe Trp Asn Thr His Val Arg Glu Leu Thr Leu Ala Ser Ile Asp
            195                 200                 205

ctt caa agt cag atg gaa ccc agg acc cat cca act tgg ctg ctt cac       672
Leu Gln Ser Gln Met Glu Pro Arg Thr His Pro Thr Trp Leu Leu His
        210                 215                 220

att ttc atc ccc ttc tgc atc att gct ttc att ttc ata gcc aca gtg       720
Ile Phe Ile Pro Phe Cys Ile Ile Ala Phe Ile Phe Ile Ala Thr Val
225                 230                 235                 240

ata gcc cta aga aaa caa ctc tgt caa aag ctg tat tct tca aaa gac       768
Ile Ala Leu Arg Lys Gln Leu Cys Gln Lys Leu Tyr Ser Ser Lys Asp
                    245                 250                 255

aca aca aaa aga cct gtc acc aca aca aag agg gaa gtg aac agt gct       816
Thr Thr Lys Arg Pro Val Thr Thr Thr Lys Arg Glu Val Asn Ser Ala
                260                 265                 270

atc tga       822
Ile
```

<210> 6
<211> 273
<212> PRT

<213> Homo sapiens

<400> 6

```
Met Ile Phe Leu Leu Leu Met Leu Ser Leu Glu Leu Gln Leu His Gln
1               5               10              15

Ile Ala Ala Leu Phe Thr Val Thr Val Pro Lys Glu Leu Tyr Ile Ile
        20              25              30

Glu His Gly Ser Asn Val Thr Leu Glu Cys Asn Phe Asp Thr Gly Ser
        35              40              45

His Val Asn Leu Gly Ala Ile Thr Ala Ser Leu Gln Lys Val Glu Asn
    50              55              60

Asp Thr Ser Pro His Arg Glu Arg Ala Thr Leu Leu Glu Glu Gln Leu
65              70              75              80

Pro Leu Gly Lys Ala Ser Phe His Ile Pro Gln Val Gln Val Arg Asp
            85              90              95

Glu Gly Gln Tyr Gln Cys Ile Ile Ile Tyr Gly Val Ala Trp Asp Tyr
            100             105             110

Lys Tyr Leu Thr Leu Lys Val Lys Ala Ser Tyr Arg Lys Ile Asn Thr
        115             120             125

His Ile Leu Lys Val Pro Glu Thr Asp Glu Val Glu Leu Thr Cys Gln
    130             135             140

Ala Thr Gly Tyr Pro Leu Ala Glu Val Ser Trp Pro Asn Val Ser Val
145             150             155             160

Pro Ala Asn Thr Ser His Ser Arg Thr Pro Glu Gly Leu Tyr Gln Val
            165             170             175

Thr Ser Val Leu Arg Leu Lys Pro Pro Pro Gly Arg Asn Phe Ser Cys
            180             185             190

Val Phe Trp Asn Thr His Val Arg Glu Leu Thr Leu Ala Ser Ile Asp
            195             200             205

Leu Gln Ser Gln Met Glu Pro Arg Thr His Pro Thr Trp Leu Leu His
    210             215             220

Ile Phe Ile Pro Phe Cys Ile Ile Ala Phe Ile Phe Ile Ala Thr Val
225                 230             235             240
```

```
Ile Ala Leu Arg Lys Gln Leu Cys Gln Lys Leu Tyr Ser Ser Lys Asp
                245                 250                 255


Thr Thr Lys Arg Pro Val Thr Thr Thr Lys Arg Glu Val Asn Ser Ala
            260                 265                 270


Ile
```

<210> 7
<211> 525
<212> DNA
<213> Homo sapiens

<220>
<221> CDS
<222> (1)..(525)

<400> 7

```
atg gct tgc ctt gga ttt cag cgg cac aag gct cag ctg aac ctg gct      48
Met Ala Cys Leu Gly Phe Gln Arg His Lys Ala Gln Leu Asn Leu Ala
1               5                   10                  15

acc agg acc tgg ccc tgc act ctc ctg ttt ttt ctt ctc ttc atc cct      96
Thr Arg Thr Trp Pro Cys Thr Leu Leu Phe Phe Leu Leu Phe Ile Pro
            20                  25                  30

gtc ttc tgc aaa gca atg cac gtg gcc cag cct gct gtg gta ctg gcc     144
Val Phe Cys Lys Ala Met His Val Ala Gln Pro Ala Val Val Leu Ala
        35                  40                  45

agc agc cga ggc atc gcc agc ttt gtg tgt gag tat gca tct cca ggc     192
Ser Ser Arg Gly Ile Ala Ser Phe Val Cys Glu Tyr Ala Ser Pro Gly
        50                  55                  60

aaa gcc act gag gtc cgg gtg aca gtg ctt cgg cag gct gac agc cag     240
Lys Ala Thr Glu Val Arg Val Thr Val Leu Arg Gln Ala Asp Ser Gln
65                  70                  75                  80

gtg act gaa gtc tgt gcg gca acc tac atg atg ggg aat gag ttg acc     288
Val Thr Glu Val Cys Ala Ala Thr Tyr Met Met Gly Asn Glu Leu Thr
                85                  90                  95

ttc cta gat gat tcc atc tgc acg ggc acc tcc agt gga aat caa gtg     336
Phe Leu Asp Asp Ser Ile Cys Thr Gly Thr Ser Ser Gly Asn Gln Val
            100                 105                 110

aac ctc act atc caa gga ctg agg gcc atg gac acg gga ctc tac atc     384
Asn Leu Thr Ile Gln Gly Leu Arg Ala Met Asp Thr Gly Leu Tyr Ile
        115                 120                 125

tgc aag gtg gag ctc atg tac cca ccg cca tac tac ctg ggc ata ggc     432
Cys Lys Val Glu Leu Met Tyr Pro Pro Pro Tyr Tyr Leu Gly Ile Gly
        130                 135                 140

aac gga acc cag att tat gta att gct aaa gaa aag aag ccc tct tac     480
Asn Gly Thr Gln Ile Tyr Val Ile Ala Lys Glu Lys Lys Pro Ser Tyr
145                 150                 155                 160
```

```
aac agg ggt cta tgt gaa aat gcc ccc aac aga gcc aga atg tga        525
Asn Arg Gly Leu Cys Glu Asn Ala Pro Asn Arg Ala Arg Met
            165                 170
```

<210> 8
<211> 174
<212> PRT
<213> Homo sapiens

<400> 8

```
Met Ala Cys Leu Gly Phe Gln Arg His Lys Ala Gln Leu Asn Leu Ala
1               5               10                  15


Thr Arg Thr Trp Pro Cys Thr Leu Leu Phe Phe Leu Leu Phe Ile Pro
            20                  25                  30


Val Phe Cys Lys Ala Met His Val Ala Gln Pro Ala Val Val Leu Ala
            35                  40                  45


Ser Ser Arg Gly Ile Ala Ser Phe Val Cys Glu Tyr Ala Ser Pro Gly
        50                  55                  60


Lys Ala Thr Glu Val Arg Val Thr Val Leu Arg Gln Ala Asp Ser Gln
65                  70                  75                  80


Val Thr Glu Val Cys Ala Ala Thr Tyr Met Met Gly Asn Glu Leu Thr
                85                  90                  95


Phe Leu Asp Asp Ser Ile Cys Thr Gly Thr Ser Ser Gly Asn Gln Val
            100                 105                 110


Asn Leu Thr Ile Gln Gly Leu Arg Ala Met Asp Thr Gly Leu Tyr Ile
        115                 120                 125


Cys Lys Val Glu Leu Met Tyr Pro Pro Pro Tyr Tyr Leu Gly Ile Gly
    130                 135                 140


Asn Gly Thr Gln Ile Tyr Val Ile Ala Lys Glu Lys Lys Pro Ser Tyr
145                 150                 155                 160


Asn Arg Gly Leu Cys Glu Asn Ala Pro Asn Arg Ala Arg Met
            165                 170
```

<210> 9
<211> 867
<212> DNA
<213> Homo sapiens

<220>

<221> CDS
<222> (1)..(867)

<400> 9

```
atg ggc cac aca cgg agg cag gga aca tca cca tcc aag tgt cca tac        48
Met Gly His Thr Arg Arg Gln Gly Thr Ser Pro Ser Lys Cys Pro Tyr
1               5                   10                  15

ctc aat ttc ttt cag ctc ttg gtg ctg gct ggt ctt tct cac ttc tgt        96
Leu Asn Phe Phe Gln Leu Leu Val Leu Ala Gly Leu Ser His Phe Cys
            20                  25                  30

tca ggt gtt atc cac gtg acc aag gaa gtg aaa gaa gtg gca acg ctg       144
Ser Gly Val Ile His Val Thr Lys Glu Val Lys Glu Val Ala Thr Leu
            35                  40                  45

tcc tgt ggt cac aat gtt tct gtt gaa gag ctg gca caa act cgc atc       192
Ser Cys Gly His Asn Val Ser Val Glu Glu Leu Ala Gln Thr Arg Ile
        50                  55                  60

tac tgg caa aag gag aag aaa atg gtg ctg act atg atg tct ggg gac       240
Tyr Trp Gln Lys Glu Lys Lys Met Val Leu Thr Met Met Ser Gly Asp
65                  70                  75                  80

atg aat ata tgg ccc gag tac aag aac cgg acc atc ttt gat atc act       288
Met Asn Ile Trp Pro Glu Tyr Lys Asn Arg Thr Ile Phe Asp Ile Thr
                85                  90                  95

aat aac ctc tcc att gtg atc ctg gct ctg cgc cca tct gac gag ggc       336
Asn Asn Leu Ser Ile Val Ile Leu Ala Leu Arg Pro Ser Asp Glu Gly
            100                 105                 110

aca tac gag tgt gtt gtt ctg aag tat gaa aaa gac gct ttc aag cgg       384
Thr Tyr Glu Cys Val Val Leu Lys Tyr Glu Lys Asp Ala Phe Lys Arg
            115                 120                 125

gaa cac ctg gct gaa gtg acg tta tca gtc aaa gct gac ttc cct aca       432
Glu His Leu Ala Glu Val Thr Leu Ser Val Lys Ala Asp Phe Pro Thr
        130                 135                 140

cct agt ata tct gac ttt gaa att cca act tct aat att aga agg ata       480
Pro Ser Ile Ser Asp Phe Glu Ile Pro Thr Ser Asn Ile Arg Arg Ile
145                 150                 155                 160

att tgc tca acc tct gga ggt ttt cca gag cct cac ctc tcc tgg ttg       528
Ile Cys Ser Thr Ser Gly Gly Phe Pro Glu Pro His Leu Ser Trp Leu
                165                 170                 175

gaa aat gga gaa gaa tta aat gcc atc aac aca aca gtt tcc caa gat       576
Glu Asn Gly Glu Glu Leu Asn Ala Ile Asn Thr Thr Val Ser Gln Asp
                180                 185                 190

cct gaa act gag ctc tat gct gtt agc agc aaa ctg gat ttc aat atg       624
Pro Glu Thr Glu Leu Tyr Ala Val Ser Ser Lys Leu Asp Phe Asn Met
            195                 200                 205

aca acc aac cac agc ttc atg tgt ctc atc aag tat gga cat tta aga       672
Thr Thr Asn His Ser Phe Met Cys Leu Ile Lys Tyr Gly His Leu Arg
            210                 215                 220

gtg aat cag acc ttc aac tgg aat aca acc aag caa gag cat ttt cct       720
Val Asn Gln Thr Phe Asn Trp Asn Thr Thr Lys Gln Glu His Phe Pro
```

```
       225                    230                    235                    240

gat aac ctg ctc cca tcc tgg gcc att acc tta atc tca gta aat gga                 768
Asp Asn Leu Leu Pro Ser Trp Ala Ile Thr Leu Ile Ser Val Asn Gly
            245                    250                    255

att ttt gtg ata tgc tgc ctg acc tac tgc ttt gcc cca aga tgc aga                 816
Ile Phe Val Ile Cys Cys Leu Thr Tyr Cys Phe Ala Pro Arg Cys Arg
            260                    265                    270

gag aga agg agg aat gag aga ttg aga agg gaa agt gta cgc cct gta                 864
Glu Arg Arg Arg Asn Glu Arg Leu Arg Arg Glu Ser Val Arg Pro Val
            275                    280                    285

taa                                                                             867
```

<210> 10
<211> 288
<212> PRT
<213> Homo sapiens

<400> 10

```
Met Gly His Thr Arg Arg Gln Gly Thr Ser Pro Ser Lys Cys Pro Tyr
1               5               10              15

Leu Asn Phe Phe Gln Leu Leu Val Leu Ala Gly Leu Ser His Phe Cys
            20              25              30

Ser Gly Val Ile His Val Thr Lys Glu Val Lys Glu Val Ala Thr Leu
        35              40              45

Ser Cys Gly His Asn Val Ser Val Glu Glu Leu Ala Gln Thr Arg Ile
    50              55              60

Tyr Trp Gln Lys Glu Lys Lys Met Val Leu Thr Met Met Ser Gly Asp
65              70              75              80

Met Asn Ile Trp Pro Glu Tyr Lys Asn Arg Thr Ile Phe Asp Ile Thr
            85              90              95

Asn Asn Leu Ser Ile Val Ile Leu Ala Leu Arg Pro Ser Asp Glu Gly
        100             105             110

Thr Tyr Glu Cys Val Val Leu Lys Tyr Glu Lys Asp Ala Phe Lys Arg
    115             120             125

Glu His Leu Ala Glu Val Thr Leu Ser Val Lys Ala Asp Phe Pro Thr
    130             135             140

Pro Ser Ile Ser Asp Phe Glu Ile Pro Thr Ser Asn Ile Arg Arg Ile
145             150             155             160
```

```
Ile Cys Ser Thr Ser Gly Gly Phe Pro Glu Pro His Leu Ser Trp Leu
            165             170             175

Glu Asn Gly Glu Glu Leu Asn Ala Ile Asn Thr Thr Val Ser Gln Asp
            180             185             190

Pro Glu Thr Glu Leu Tyr Ala Val Ser Ser Lys Leu Asp Phe Asn Met
            195             200             205

Thr Thr Asn His Ser Phe Met Cys Leu Ile Lys Tyr Gly His Leu Arg
    210             215             220

Val Asn Gln Thr Phe Asn Trp Asn Thr Thr Lys Gln Glu His Phe Pro
225             230             235             240

Asp Asn Leu Leu Pro Ser Trp Ala Ile Thr Leu Ile Ser Val Asn Gly
                245             250             255

Ile Phe Val Ile Cys Cys Leu Thr Tyr Cys Phe Ala Pro Arg Cys Arg
            260             265             270

Glu Arg Arg Arg Asn Glu Arg Leu Arg Arg Glu Ser Val Arg Pro Val
            275             280             285
```

<210> 11
<211> 654
<212> DNA
<213> Homo sapiens

<220>
<221> CDS
<222> (1)..(654)

<400> 11

35

```
atg gat ccc cag tgc act atg gga ctg agt aac att ctc ttt gtg atg        48
Met Asp Pro Gln Cys Thr Met Gly Leu Ser Asn Ile Leu Phe Val Met
1               5                   10                  15

gcc ttc ctg ctc tct gct aac ttc agt caa cct gaa ata gta cca att        96
Ala Phe Leu Leu Ser Ala Asn Phe Ser Gln Pro Glu Ile Val Pro Ile
            20                  25                  30

tct aat ata aca gaa aat gtg tac ata aat ttg acc tgc tca tct ata       144
Ser Asn Ile Thr Glu Asn Val Tyr Ile Asn Leu Thr Cys Ser Ser Ile
            35                  40                  45

cac ggt tac cca gaa cct aag aag atg agt gtt ttg cta aga acc aag       192
His Gly Tyr Pro Glu Pro Lys Lys Met Ser Val Leu Leu Arg Thr Lys
        50                  55                  60

aat tca act atc gag tat gat ggt att atg cag aaa tct caa gat aat       240
Asn Ser Thr Ile Glu Tyr Asp Gly Ile Met Gln Lys Ser Gln Asp Asn
65                  70                  75                  80

gtc aca gaa ctg tac gac gtt tcc atc agc ttg tct gtt tca ttc cct       288
Val Thr Glu Leu Tyr Asp Val Ser Ile Ser Leu Ser Val Ser Phe Pro
                85                  90                  95

gat gtt acg agc aat atg acc atc ttc tgt att ctg gaa act gac aag       336
Asp Val Thr Ser Asn Met Thr Ile Phe Cys Ile Leu Glu Thr Asp Lys
            100                 105                 110

acg cgg ctt tta tct tca cct ttc tct ata gag ctt gag gac cct cag       384
Thr Arg Leu Leu Ser Ser Pro Phe Ser Ile Glu Leu Glu Asp Pro Gln
        115                 120                 125

cct ccc cca gac cac att cct tgg att aca gct gta ctt cca aca gtt       432
Pro Pro Pro Asp His Ile Pro Trp Ile Thr Ala Val Leu Pro Thr Val
        130                 135                 140

att ata tgt gtg atg gtt ttc tgt cta att cta tgg aaa tgg aag aag       480
Ile Ile Cys Val Met Val Phe Cys Leu Ile Leu Trp Lys Trp Lys Lys
145                 150                 155                 160

aag aag cgg cct cgc aac tct tat aaa tgt gga acc aac aca atg gag       528
Lys Lys Arg Pro Arg Asn Ser Tyr Lys Cys Gly Thr Asn Thr Met Glu
                165                 170                 175

agg gaa gag agt gaa cag acc aag aaa aga gaa aaa atc cat ata cct       576
Arg Glu Glu Ser Glu Gln Thr Lys Lys Arg Glu Lys Ile His Ile Pro
            180                 185                 190

gaa aga tct gat gaa gcc cag cgt gtt ttt aaa agt tcg aag aca tct       624
Glu Arg Ser Asp Glu Ala Gln Arg Val Phe Lys Ser Ser Lys Thr Ser
        195                 200                 205

tca tgc gac aaa agt gat aca tgt ttt taa                               654
Ser Cys Asp Lys Ser Asp Thr Cys Phe
        210                 215
```

<210> 12

<211> 217

<212> PRT

<213> Homo sapiens

36

<400> 12

```
Met Asp Pro Gln Cys Thr Met Gly Leu Ser Asn Ile Leu Phe Val Met
1               5                   10                  15

Ala Phe Leu Leu Ser Ala Asn Phe Ser Gln Pro Glu Ile Val Pro Ile
            20                  25                  30

Ser Asn Ile Thr Glu Asn Val Tyr Ile Asn Leu Thr Cys Ser Ser Ile
            35                  40                  45

His Gly Tyr Pro Glu Pro Lys Lys Met Ser Val Leu Leu Arg Thr Lys
        50                  55                  60

Asn Ser Thr Ile Glu Tyr Asp Gly Ile Met Gln Lys Ser Gln Asp Asn
65                  70                  75                  80

Val Thr Glu Leu Tyr Asp Val Ser Ile Ser Leu Ser Val Ser Phe Pro
                85                  90                  95

Asp Val Thr Ser Asn Met Thr Ile Phe Cys Ile Leu Glu Thr Asp Lys
            100                 105                 110

Thr Arg Leu Leu Ser Ser Pro Phe Ser Ile Glu Leu Glu Asp Pro Gln
        115                 120                 125

Pro Pro Pro Asp His Ile Pro Trp Ile Thr Ala Val Leu Pro Thr Val
    130                 135                 140

Ile Ile Cys Val Met Val Phe Cys Leu Ile Leu Trp Lys Trp Lys Lys
145                 150                 155                 160

Lys Lys Arg Pro Arg Asn Ser Tyr Lys Cys Gly Thr Asn Thr Met Glu
                165                 170                 175

Arg Glu Glu Ser Glu Gln Thr Lys Lys Arg Glu Lys Ile His Ile Pro
            180                 185                 190

Glu Arg Ser Asp Glu Ala Gln Arg Val Phe Lys Ser Ser Lys Thr Ser
        195                 200                 205

Ser Cys Asp Lys Ser Asp Thr Cys Phe
    210                 215
```

<210> 13
<211> 1605
<212> DNA
<213> Homo sapiens

<220>
<221> CDS
<222> (1)..(1605)

<400> 13

```
atg ctg cgt cgg cgg ggc agc cct ggc atg ggt gtg cat gtg ggt gca    48
Met Leu Arg Arg Arg Gly Ser Pro Gly Met Gly Val His Val Gly Ala
1               5                   10                  15

gcc ctg gga gca ctg tgg ttc tgc ctc aca gga gcc ctg gag gtc cag    96
Ala Leu Gly Ala Leu Trp Phe Cys Leu Thr Gly Ala Leu Glu Val Gln
            20                  25                  30

gtc cct gaa gac cca gtg gtg gca ctg gtg ggc acc gat gcc acc ctg   144
Val Pro Glu Asp Pro Val Val Ala Leu Val Gly Thr Asp Ala Thr Leu
        35                  40                  45

tgc tgc tcc ttc tcc cct gag cct ggc ttc agc ctg gca cag ctc aac   192
Cys Cys Ser Phe Ser Pro Glu Pro Gly Phe Ser Leu Ala Gln Leu Asn
```

```
                50                     55                     60

     ctc atc tgg cag ctg aca gat acc aaa cag ctg gtg cac agc ttt gct      240
     Leu Ile Trp Gln Leu Thr Asp Thr Lys Gln Leu Val His Ser Phe Ala
     65              70                     75                     80

     gag ggc cag gac cag ggc agc gcc tat gcc aac cgc acg gcc ctc ttc      288
     Glu Gly Gln Asp Gln Gly Ser Ala Tyr Ala Asn Arg Thr Ala Leu Phe
                     85                     90                     95

     ccg gac ctg ctg gca cag ggc aac gca tcc ctg agg ctg cag cgc gtg      336
     Pro Asp Leu Leu Ala Gln Gly Asn Ala Ser Leu Arg Leu Gln Arg Val
                     100                    105                    110

     cgt gtg gcg gac gag ggc agc ttc acc tgc ttc gtg agc atc cgg gat      384
     Arg Val Ala Asp Glu Gly Ser Phe Thr Cys Phe Val Ser Ile Arg Asp
                     115                    120                    125

     ttc ggc agc gct gcc gtc agc ctg cag gtg gcc gct ccc tac tcg aag      432
     Phe Gly Ser Ala Ala Val Ser Leu Gln Val Ala Ala Pro Tyr Ser Lys
                     130                    135                    140

     ccc agc atg acc ctg gag ccc aac aag gac ctg cgg cca ggg gac acg      480
     Pro Ser Met Thr Leu Glu Pro Asn Lys Asp Leu Arg Pro Gly Asp Thr
     145                    150                    155                    160

     gtg acc atc acg tgc tcc agc tac cag ggc tac cct gag gct gag gtg      528
     Val Thr Ile Thr Cys Ser Ser Tyr Gln Gly Tyr Pro Glu Ala Glu Val
                     165                    170                    175

     ttc tgg cag gat ggg cag ggt gtg ccc ctg act ggc aac gtg acc acg      576
     Phe Trp Gln Asp Gly Gln Gly Val Pro Leu Thr Gly Asn Val Thr Thr
                     180                    185                    190

     tcg cag atg gcc aac gag cag ggc ttg ttt gat gtg cac agc atc ctg      624
     Ser Gln Met Ala Asn Glu Gln Gly Leu Phe Asp Val His Ser Ile Leu
                     195                    200                    205

     cgg gtg gtg ctg ggt gca aat ggc acc tac agc tgc ctg gtg cgc aac      672
     Arg Val Val Leu Gly Ala Asn Gly Thr Tyr Ser Cys Leu Val Arg Asn
                     210                    215                    220

     ccc gtg ctg cag cag gat gcg cac agc tct gtc acc atc aca ccc cag      720
     Pro Val Leu Gln Gln Asp Ala His Ser Ser Val Thr Ile Thr Pro Gln
     225                    230                    235                    240

     aga agc ccc aca gga gcc gtg gag gtc cag gtc cct gag gac ccg gtg      768
     Arg Ser Pro Thr Gly Ala Val Glu Val Gln Val Pro Glu Asp Pro Val
                     245                    250                    255

     gtg gcc cta gtg ggc acc gat gcc acc ctg cgc tgc tcc ttc tcc ccc      816
     Val Ala Leu Val Gly Thr Asp Ala Thr Leu Arg Cys Ser Phe Ser Pro
                     260                    265                    270

     gag cct ggc ttc agc ctg gca cag ctc aac ctc atc tgg cag ctg aca      864
     Glu Pro Gly Phe Ser Leu Ala Gln Leu Asn Leu Ile Trp Gln Leu Thr
                     275                    280                    285

     gac acc aaa cag ctg gtg cac agt ttc acc gaa ggc cgg gac cag ggc      912
     Asp Thr Lys Gln Leu Val His Ser Phe Thr Glu Gly Arg Asp Gln Gly
                     290                    295                    300

     agc gcc tat gcc aac cgc acg gcc ctc ttc ccg gac ctg ctg gca caa      960
```

```
            Ser Ala Tyr Ala Asn Arg Thr Ala Leu Phe Pro Asp Leu Leu Ala Gln
            305             310             315                         320

ggc aat gca tcc ctg agg ctg cag cgc gtg cgt gtg gcg gac gag ggc         1008
Gly Asn Ala Ser Leu Arg Leu Gln Arg Val Arg Val Ala Asp Glu Gly
                325             330             335

agc ttc acc tgc ttc gtg agc atc cgg gat ttc ggc agc gct gcc gtc         1056
Ser Phe Thr Cys Phe Val Ser Ile Arg Asp Phe Gly Ser Ala Ala Val
                340             345             350

agc ctg cag gtg gcc gct ccc tac tcg aag ccc agc atg acc ctg gag         1104
Ser Leu Gln Val Ala Ala Pro Tyr Ser Lys Pro Ser Met Thr Leu Glu
                355             360             365

ccc aac aag gac ctg cgg cca ggg gac acg gtg acc atc acg tgc tcc         1152
Pro Asn Lys Asp Leu Arg Pro Gly Asp Thr Val Thr Ile Thr Cys Ser
            370             375             380

agc tac cgg ggc tac cct gag gct gag gtg ttc tgg cag gat ggg cag         1200
Ser Tyr Arg Gly Tyr Pro Glu Ala Glu Val Phe Trp Gln Asp Gly Gln
385             390             395             400

ggt gtg ccc ctg act ggc aac gtg acc acg tcg cag atg gcc aac gag         1248
Gly Val Pro Leu Thr Gly Asn Val Thr Thr Ser Gln Met Ala Asn Glu
                405             410             415

cag ggc ttg ttt gat gtg cac agc gtc ctg cgg gtg gtg ctg ggt gcg         1296
Gln Gly Leu Phe Asp Val His Ser Val Leu Arg Val Val Leu Gly Ala
                420             425             430

aat ggc acc tac agc tgc ctg gtg cgc aac ccc gtg ctg cag cag gat         1344
Asn Gly Thr Tyr Ser Cys Leu Val Arg Asn Pro Val Leu Gln Gln Asp
            435             440             445

gcg cac ggc tct gtc acc atc aca ggg cag cct atg aca ttc ccc cca         1392
Ala His Gly Ser Val Thr Ile Thr Gly Gln Pro Met Thr Phe Pro Pro
        450             455             460

gag gcc ctg tgg gtg acc gtg ggg ctg tct gtc tgt ctc att gca ctg         1440
Glu Ala Leu Trp Val Thr Val Gly Leu Ser Val Cys Leu Ile Ala Leu
465             470             475             480

ctg gtg gcc ctg gct ttc gtg tgc tgg aga aag atc aaa cag agc tgt         1488
Leu Val Ala Leu Ala Phe Val Cys Trp Arg Lys Ile Lys Gln Ser Cys
                485             490             495

gag gag gag aat gca gga gct gag gac cag gat ggg gag gga gaa ggc         1536
Glu Glu Glu Asn Ala Gly Ala Glu Asp Gln Asp Gly Glu Gly Glu Gly
            500             505             510

tcc aag aca gcc ctg cag cct ctg aaa cac tct gac agc aaa gaa gat         1584
Ser Lys Thr Ala Leu Gln Pro Leu Lys His Ser Asp Ser Lys Glu Asp
            515             520             525

gat gga caa gaa ata gcc tga                                             1605
Asp Gly Gln Glu Ile Ala
            530
```

<210> 14
<211> 534

<212> PRT
<213> Homo sapiens

<400> 14

<212> PRT
<213> Homo sapiens

<400> 14

Met Leu Arg Arg Arg Gly Ser Pro Gly Met Gly Val His Val Gly Ala
1                    5                10                15

Ala Leu Gly Ala Leu Trp Phe Cys Leu Thr Gly Ala Leu Glu Val Gln
            20                25                30

Val Pro Glu Asp Pro Val Val Ala Leu Val Gly Thr Asp Ala Thr Leu
            35                40                45

Cys Cys Ser Phe Ser Pro Glu Pro Gly Phe Ser Leu Ala Gln Leu Asn
    50                55                60

Leu Ile Trp Gln Leu Thr Asp Thr Lys Gln Leu Val His Ser Phe Ala
65                70                75                80

Glu Gly Gln Asp Gln Gly Ser Ala Tyr Ala Asn Arg Thr Ala Leu Phe
            85                90                95

Pro Asp Leu Leu Ala Gln Gly Asn Ala Ser Leu Arg Leu Gln Arg Val
            100               105               110

Arg Val Ala Asp Glu Gly Ser Phe Thr Cys Phe Val Ser Ile Arg Asp
        115               120               125

Phe Gly Ser Ala Ala Val Ser Leu Gln Val Ala Ala Pro Tyr Ser Lys
    130               135               140

Pro Ser Met Thr Leu Glu Pro Asn Lys Asp Leu Arg Pro Gly Asp Thr
145               150               155               160

Val Thr Ile Thr Cys Ser Ser Tyr Gln Gly Tyr Pro Glu Ala Glu Val
            165               170               175

Phe Trp Gln Asp Gly Gln Gly Val Pro Leu Thr Gly Asn Val Thr Thr
        180               185               190

Ser Gln Met Ala Asn Glu Gln Gly Leu Phe Asp Val His Ser Ile Leu
        195               200               205

Arg Val Val Leu Gly Ala Asn Gly Thr Tyr Ser Cys Leu Val Arg Asn
    210               215               220

Pro Val Leu Gln Gln Asp Ala His Ser Ser Val Thr Ile Thr Pro Gln
225               230               235               240

```
Arg Ser Pro Thr Gly Ala Val Glu Val Gln Val Pro Glu Asp Pro Val
            245             250             255

Val Ala Leu Val Gly Thr Asp Ala Thr Leu Arg Cys Ser Phe Ser Pro
            260             265             270

Glu Pro Gly Phe Ser Leu Ala Gln Leu Asn Leu Ile Trp Gln Leu Thr
            275             280             285

Asp Thr Lys Gln Leu Val His Ser Phe Thr Glu Gly Arg Asp Gln Gly
            290             295             300

Ser Ala Tyr Ala Asn Arg Thr Ala Leu Phe Pro Asp Leu Leu Ala Gln
305             310             315             320

Gly Asn Ala Ser Leu Arg Leu Gln Arg Val Arg Val Ala Asp Glu Gly
            325             330             335

Ser Phe Thr Cys Phe Val Ser Ile Arg Asp Phe Gly Ser Ala Ala Val
            340             345             350

Ser Leu Gln Val Ala Ala Pro Tyr Ser Lys Pro Ser Met Thr Leu Glu
            355             360             365

Pro Asn Lys Asp Leu Arg Pro Gly Asp Thr Val Thr Ile Thr Cys Ser
            370             375             380

Ser Tyr Arg Gly Tyr Pro Glu Ala Glu Val Phe Trp Gln Asp Gly Gln
385             390             395             400

Gly Val Pro Leu Thr Gly Asn Val Thr Thr Ser Gln Met Ala Asn Glu
            405             410             415

Gln Gly Leu Phe Asp Val His Ser Val Leu Arg Val Val Leu Gly Ala
            420             425             430

Asn Gly Thr Tyr Ser Cys Leu Val Arg Asn Pro Val Leu Gln Gln Asp
            435             440             445

Ala His Gly Ser Val Thr Ile Thr Gly Gln Pro Met Thr Phe Pro Pro
            450             455             460

Glu Ala Leu Trp Val Thr Val Gly Leu Ser Val Cys Leu Ile Ala Leu
465             470             475             480

Leu Val Ala Leu Ala Phe Val Cys Trp Arg Lys Ile Lys Gln Ser Cys
            485             490             495
```

```
            Glu Glu Glu Asn Ala Gly Ala Glu Asp Gln Asp Gly Glu Gly Glu Gly
                    500                 505                 510


            Ser Lys Thr Ala Leu Gln Pro Leu Lys His Ser Asp Ser Lys Glu Asp
                    515                 520                 525


            Asp Gly Gln Glu Ile Ala
                    530


<210> 15
<211> 564
<212> DNA
<213> Homo sapiens


<220>
<221> CDS
<222> (1)..(564)


<400> 15


    atg ttc aga ggc cgg aca gca gtg ttt gct gat caa gtg ata gtt ggc       48
    Met Phe Arg Gly Arg Thr Ala Val Phe Ala Asp Gln Val Ile Val Gly
    1                   5                   10                  15


    aat gcc tct ttg cgg ctg aaa aac gtg caa ctc aca gat gct ggc acc       96
    Asn Ala Ser Leu Arg Leu Lys Asn Val Gln Leu Thr Asp Ala Gly Thr
                    20                  25                  30


    tac aaa tgt tat atc atc act tct aaa ggc aag ggg aat gct aac ctt      144
    Tyr Lys Cys Tyr Ile Ile Thr Ser Lys Gly Lys Gly Asn Ala Asn Leu
                35                  40                  45


    gag tat aaa act gga gcc ttc agc atg ccg gaa gtg aat gtg gac tat      192
    Glu Tyr Lys Thr Gly Ala Phe Ser Met Pro Glu Val Asn Val Asp Tyr
            50                  55                  60


    aat gcc agc tca gag acc ttg cgg tgt gag gct ccc cga tgg ttc ccc      240
    Asn Ala Ser Ser Glu Thr Leu Arg Cys Glu Ala Pro Arg Trp Phe Pro
    65                  70                  75                  80


    cag ccc aca gtg gtc tgg gca tcc caa gtt gac cag gga gcc aac ttc      288
    Gln Pro Thr Val Val Trp Ala Ser Gln Val Asp Gln Gly Ala Asn Phe
                    85                  90                  95


    tcg gaa gtc tcc aat acc agc ttt gag ctg aac tct gag aat gtg acc      336
    Ser Glu Val Ser Asn Thr Ser Phe Glu Leu Asn Ser Glu Asn Val Thr
                    100                 105                 110


    atg aag gtt gtg tct gtg ctc tac aat gtt acg atc aac aac aca tac      384
    Met Lys Val Val Ser Val Leu Tyr Asn Val Thr Ile Asn Asn Thr Tyr
                115                 120                 125


    tcc tgt atg att gaa aat gac att gcc aaa gca aca ggg gat atc aaa      432
    Ser Cys Met Ile Glu Asn Asp Ile Ala Lys Ala Thr Gly Asp Ile Lys
            130                 135                 140


    gtg aca gaa tcg gag atc aaa agg cgg agt cac cta cag ctg cta aac      480
    Val Thr Glu Ser Glu Ile Lys Arg Arg Ser His Leu Gln Leu Leu Asn
```

```
                    145                      150                      155                      160

                    tca aag gct tct ctg tgt gtc tct tct ttc ttt gcc atc agc tgg gca          528
                    Ser Lys Ala Ser Leu Cys Val Ser Ser Phe Phe Ala Ile Ser Trp Ala
                                    165                      170                      175

                    ctt ctg cct ctc agc cct tac ctg atg cta aaa taa                          564
                    Leu Leu Pro Leu Ser Pro Tyr Leu Met Leu Lys
                                    180                      185
```

<210> 16
<211> 187
<212> PRT
<213> Homo sapiens

<400> 16

```
Met Phe Arg Gly Arg Thr Ala Val Phe Ala Asp Gln Val Ile Val Gly
1               5               10              15

Asn Ala Ser Leu Arg Leu Lys Asn Val Gln Leu Thr Asp Ala Gly Thr
        20              25              30

Tyr Lys Cys Tyr Ile Ile Thr Ser Lys Gly Lys Gly Asn Ala Asn Leu
            35              40              45

Glu Tyr Lys Thr Gly Ala Phe Ser Met Pro Glu Val Asn Val Asp Tyr
    50              55              60

Asn Ala Ser Ser Glu Thr Leu Arg Cys Glu Ala Pro Arg Trp Phe Pro
65              70              75              80

Gln Pro Thr Val Val Trp Ala Ser Gln Val Asp Gln Gly Ala Asn Phe
            85              90              95

Ser Glu Val Ser Asn Thr Ser Phe Glu Leu Asn Ser Glu Asn Val Thr
            100             105             110

Met Lys Val Val Ser Val Leu Tyr Asn Val Thr Ile Asn Asn Thr Tyr
        115             120             125

Ser Cys Met Ile Glu Asn Asp Ile Ala Lys Ala Thr Gly Asp Ile Lys
    130             135             140

Val Thr Glu Ser Glu Ile Lys Arg Arg Ser His Leu Gln Leu Leu Asn
145             150             155             160

Ser Lys Ala Ser Leu Cys Val Ser Ser Phe Phe Ala Ile Ser Trp Ala
            165             170             175

Leu Leu Pro Leu Ser Pro Tyr Leu Met Leu Lys
                    180             185
```

<210> 17
<211> 936
<212> DNA
<213> Homo sapiens

<220>
<221> CDS
<222> (1)..(936)

<400> 17

```
atg ggc gtc ccc acg gcc ctg gag gcc ggc agc tgg cgc tgg gga tcc      48
Met Gly Val Pro Thr Ala Leu Glu Ala Gly Ser Trp Arg Trp Gly Ser
1               5                   10                  15

ctg ctc ttc gct ctc ttc ctg gct gcg tcc cta ggt ccg gtg gca gcc      96
Leu Leu Phe Ala Leu Phe Leu Ala Ala Ser Leu Gly Pro Val Ala Ala
                20                  25                  30

ttc aag gtc gcc acg ccg tat tcc ctg tat gtc tgt ccc gag ggg cag     144
Phe Lys Val Ala Thr Pro Tyr Ser Leu Tyr Val Cys Pro Glu Gly Gln
            35                  40                  45

aac gtc acc ctc acc tgc agg ctc ttg ggc cct gtg gac aaa ggg cac     192
Asn Val Thr Leu Thr Cys Arg Leu Leu Gly Pro Val Asp Lys Gly His
        50                  55                  60

gat gtg acc ttc tac aag acg tgg tac cgc agc tcg agg ggc gag gtg     240
Asp Val Thr Phe Tyr Lys Thr Trp Tyr Arg Ser Ser Arg Gly Glu Val
65                  70                  75                  80

cag acc tgc tca gag cgc cgg ccc atc cgc aac ctc acg ttc cag gac     288
Gln Thr Cys Ser Glu Arg Arg Pro Ile Arg Asn Leu Thr Phe Gln Asp
                85                  90                  95

ctt cac ctg cac cat gga ggc cac cag gct gcc aac acc agc cac gac     336
Leu His Leu His His Gly Gly His Gln Ala Ala Asn Thr Ser His Asp
            100                 105                 110

ctg gct cag cgc cac ggg ctg gag tcg gcc tcc gac cac cat ggc aac     384
Leu Ala Gln Arg His Gly Leu Glu Ser Ala Ser Asp His His Gly Asn
        115                 120                 125

ttc tcc atc acc atg cgc aac ctg acc ctg ctg gat agc ggc ctc tac     432
Phe Ser Ile Thr Met Arg Asn Leu Thr Leu Leu Asp Ser Gly Leu Tyr
        130                 135                 140

tgc tgc ctg gtg gtg gag atc agg cac cac cac tcg gag cac agg gtc     480
Cys Cys Leu Val Val Glu Ile Arg His His His Ser Glu His Arg Val
145                 150                 155                 160

cat ggt gcc atg gag ctg cag gtg cag aca ggc aaa gat gca cca tcc     528
His Gly Ala Met Glu Leu Gln Val Gln Thr Gly Lys Asp Ala Pro Ser
                165                 170                 175

aac tgt gtg gtg tac cca tcc tcc tcc cag gat agt gaa aac atc acg     576
Asn Cys Val Val Tyr Pro Ser Ser Ser Gln Asp Ser Glu Asn Ile Thr
                180                 185                 190

gct gca gcc ctg gct acg ggt gcc tgc atc gta gga atc ctc tgc ctc     624
```

```
        Ala Ala Ala Leu Ala Thr Gly Ala Cys Ile Val Gly Ile Leu Cys Leu
                195                 200                 205

        ccc ctc atc ctg ctc ctg gtc tac aag caa agg cag gca gcc tcc aac    672
        Pro Leu Ile Leu Leu Leu Val Tyr Lys Gln Arg Gln Ala Ala Ser Asn
                210                 215                 220

        cgc cgt gcc cag gag ctg gtg cgg atg gac agc aac att caa ggg att    720
        Arg Arg Ala Gln Glu Leu Val Arg Met Asp Ser Asn Ile Gln Gly Ile
        225                 230                 235                 240

        gaa aac ccc ggc ttt gaa gcc tca cca cct gcc cag ggg ata ccc gag    768
        Glu Asn Pro Gly Phe Glu Ala Ser Pro Pro Ala Gln Gly Ile Pro Glu
                        245                 250                 255

        gcc aaa gtc agg cac ccc ctg tcc tat gtg gcc cag cgg cag cct tct    816
        Ala Lys Val Arg His Pro Leu Ser Tyr Val Ala Gln Arg Gln Pro Ser
                        260                 265                 270

        gag tct ggg cgg cat ctg ctt tcg gag ccc agc acc ccc ctg tct cct    864
        Glu Ser Gly Arg His Leu Leu Ser Glu Pro Ser Thr Pro Leu Ser Pro
                        275                 280                 285

        cca ggc ccc gga gac gtc ttc ttc cca tcc ctg gac cct gtc cct gac    912
        Pro Gly Pro Gly Asp Val Phe Phe Pro Ser Leu Asp Pro Val Pro Asp
                290                 295                 300

        tct cca aac ttt gag gtc atc tag                                    936
        Ser Pro Asn Phe Glu Val Ile
        305                 310
```

<210> 18
<211> 311
<212> PRT
<213> Homo sapiens

<400> 18

```
        Met Gly Val Pro Thr Ala Leu Glu Ala Gly Ser Trp Arg Trp Gly Ser
        1                 5                   10                  15

        Leu Leu Phe Ala Leu Phe Leu Ala Ala Ser Leu Gly Pro Val Ala Ala
                        20                  25                  30

        Phe Lys Val Ala Thr Pro Tyr Ser Leu Tyr Val Cys Pro Glu Gly Gln
                        35                  40                  45

        Asn Val Thr Leu Thr Cys Arg Leu Leu Gly Pro Val Asp Lys Gly His
                50                  55                  60

        Asp Val Thr Phe Tyr Lys Thr Trp Tyr Arg Ser Ser Arg Gly Glu Val
        65                  70                  75                  80

        Gln Thr Cys Ser Glu Arg Arg Pro Ile Arg Asn Leu Thr Phe Gln Asp
                        85                  90                  95
```

```
Leu His Leu His His Gly Gly His Gln Ala Ala Asn Thr Ser His Asp
        100                 105                 110

Leu Ala Gln Arg His Gly Leu Glu Ser Ala Ser Asp His His Gly Asn
        115                 120                 125

Phe Ser Ile Thr Met Arg Asn Leu Thr Leu Leu Asp Ser Gly Leu Tyr
        130                 135                 140

Cys Cys Leu Val Val Glu Ile Arg His His His Ser Glu His Arg Val
145                 150                 155                 160

His Gly Ala Met Glu Leu Gln Val Gln Thr Gly Lys Asp Ala Pro Ser
                165                 170                 175

Asn Cys Val Val Tyr Pro Ser Ser Ser Gln Asp Ser Glu Asn Ile Thr
        180                 185                 190

Ala Ala Ala Leu Ala Thr Gly Ala Cys Ile Val Gly Ile Leu Cys Leu
        195                 200                 205

Pro Leu Ile Leu Leu Leu Val Tyr Lys Gln Arg Gln Ala Ala Ser Asn
        210                 215                 220

Arg Arg Ala Gln Glu Leu Val Arg Met Asp Ser Asn Ile Gln Gly Ile
225                 230                 235                 240

Glu Asn Pro Gly Phe Glu Ala Ser Pro Pro Ala Gln Gly Ile Pro Glu
                245                 250                 255

Ala Lys Val Arg His Pro Leu Ser Tyr Val Ala Gln Arg Gln Pro Ser
                260                 265                 270

Glu Ser Gly Arg His Leu Leu Ser Glu Pro Ser Thr Pro Leu Ser Pro
        275                 280                 285

Pro Gly Pro Gly Asp Val Phe Phe Pro Ser Leu Asp Pro Val Pro Asp
        290                 295                 300

Ser Pro Asn Phe Glu Val Ile
305                 310
```

<210> 19
<211> 1335
<212> DNA
<213> Homo sapiens

<220>

<221> CDS
<222> (1)..(1335)

<400> 19

```
atg ttg ctc atg gtc gtc agc atg gcg tgt gtt ggg ttc ttc ttg gtc        48
Met Leu Leu Met Val Val Ser Met Ala Cys Val Gly Phe Phe Leu Val
1               5                   10                  15

cag agg gcc ggt cca cac gtg ggt ggt cag gac aag ccc ttc ctg tct        96
Gln Arg Ala Gly Pro His Val Gly Gly Gln Asp Lys Pro Phe Leu Ser
            20                  25                  30

gcc tgg ccc agc gct gtg gtg cct cga gga gga cac gtg act ctt cgg       144
Ala Trp Pro Ser Ala Val Val Pro Arg Gly Gly His Val Thr Leu Arg
        35                  40                  45

tgt cac tat cgt cat agg ttt aac aat ttc atg cta tac aaa gaa gac       192
Cys His Tyr Arg His Arg Phe Asn Asn Phe Met Leu Tyr Lys Glu Asp
    50                  55                  60

aga atc cac gtt ccc atc ttc cat ggc aga tta ttc cag gag agc ttc       240
Arg Ile His Val Pro Ile Phe His Gly Arg Leu Phe Gln Glu Ser Phe
65                  70                  75                  80

aac atg agc cct gtg acc aca gca cat gca ggg aac tac aca tgt cgg       288
Asn Met Ser Pro Val Thr Thr Ala His Ala Gly Asn Tyr Thr Cys Arg
                85                  90                  95

ggt tca cac cca cac tcc ccc act ggg tgg tcg gca ccc agc aac ccc       336
Gly Ser His Pro His Ser Pro Thr Gly Trp Ser Ala Pro Ser Asn Pro
            100                 105                 110

gtg gtg atc atg gtc aca gga aac cac aga aaa cct tcc ctc ctg gcc       384
Val Val Ile Met Val Thr Gly Asn His Arg Lys Pro Ser Leu Leu Ala
            115                 120                 125

cac cca ggt ccc ctg gtg aaa tca gga gag aga gtc atc ctg caa tgt       432
His Pro Gly Pro Leu Val Lys Ser Gly Glu Arg Val Ile Leu Gln Cys
        130                 135                 140

tgg tca gat atc atg ttt gag cac ttc ttt ctg cac aaa gag ggg atc       480
Trp Ser Asp Ile Met Phe Glu His Phe Phe Leu His Lys Glu Gly Ile
145                 150                 155                 160

tct aag gac ccc tca cgc ctc gtt gga cag atc cat gat ggg gtc tcc       528
Ser Lys Asp Pro Ser Arg Leu Val Gly Gln Ile His Asp Gly Val Ser
                165                 170                 175

aag gcc aat ttc tcc atc ggt ccc atg atg ctt gcc ctt gca ggg acc       576
Lys Ala Asn Phe Ser Ile Gly Pro Met Met Leu Ala Leu Ala Gly Thr
            180                 185                 190

tac aga tgc tac ggt tct gtt act cac acc ccc tat cag ttg tca gct       624
Tyr Arg Cys Tyr Gly Ser Val Thr His Thr Pro Tyr Gln Leu Ser Ala
        195                 200                 205

ccc agt gat ccc ctg gac atc gtg gtc aca ggt cca tat gag aaa cct       672
Pro Ser Asp Pro Leu Asp Ile Val Val Thr Gly Pro Tyr Glu Lys Pro
    210                 215                 220

tct ctc tca gcc cag ccg ggc ccc aag gtt cag gca gga gag agc gtg       720
Ser Leu Ser Ala Gln Pro Gly Pro Lys Val Gln Ala Gly Glu Ser Val
225                 230                 235                 240
```

```
acc ttg tcc tgc agc tcc cgg agc tcc tat gac atg tac cat cta tcc          768
Thr Leu Ser Cys Ser Ser Arg Ser Ser Tyr Asp Met Tyr His Leu Ser
            245                 250                 255

agg gag ggg gga gcc cat gaa cgt agg ctc cct gca gtg cgc aag gtc          816
Arg Glu Gly Gly Ala His Glu Arg Arg Leu Pro Ala Val Arg Lys Val
            260                 265                 270

aac aga aca ttc cag gca gat ttc cct ctg ggc cct gcc acc cac gga          864
Asn Arg Thr Phe Gln Ala Asp Phe Pro Leu Gly Pro Ala Thr His Gly
            275                 280                 285

ggg acc tac aga tgc ttc ggc tct ttc cgt cac tct ccc tac gag tgg          912
Gly Thr Tyr Arg Cys Phe Gly Ser Phe Arg His Ser Pro Tyr Glu Trp
            290                 295                 300

tca gac ccg agt gac cca ctg ctt gtt tct gtc aca gga aac cct tca          960
Ser Asp Pro Ser Asp Pro Leu Leu Val Ser Val Thr Gly Asn Pro Ser
305                 310                 315                 320

agt agt tgg cct tca ccc aca gaa cca agc tcc aaa tct ggt aac ccc         1008
Ser Ser Trp Pro Ser Pro Thr Glu Pro Ser Ser Lys Ser Gly Asn Pro
                325                 330                 335

aga cac ctg cac att ctg att ggg acc tca gtg gtc atc atc ctc ttc         1056
Arg His Leu His Ile Leu Ile Gly Thr Ser Val Val Ile Ile Leu Phe
            340                 345                 350

atc ctc ctc ctc ttc ttt ctc ctt cat ctc tgg tgc tcc aac aaa aaa         1104
Ile Leu Leu Leu Phe Phe Leu Leu His Leu Trp Cys Ser Asn Lys Lys
            355                 360                 365

aat gct gct gta atg gac caa gag cct gca ggg aac aga aca gcc aac         1152
Asn Ala Ala Val Met Asp Gln Glu Pro Ala Gly Asn Arg Thr Ala Asn
            370                 375                 380

agc gag gac tct gat gaa caa gac cct gag gag gtg aca tac gca cag         1200
Ser Glu Asp Ser Asp Glu Gln Asp Pro Glu Glu Val Thr Tyr Ala Gln
385                 390                 395                 400

ttg gat cac tgc gtt ttc aca cag aga aaa atc act cgc cct tct cag         1248
Leu Asp His Cys Val Phe Thr Gln Arg Lys Ile Thr Arg Pro Ser Gln
            405                 410                 415

agg ccc aag aca ccc cct aca gat acc atc ttg tac acg gaa ctt cca         1296
Arg Pro Lys Thr Pro Pro Thr Asp Thr Ile Leu Tyr Thr Glu Leu Pro
            420                 425                 430

aat gct aag ccc aga tcc aaa gtt gtc tcc tgc cca tga                     1335
Asn Ala Lys Pro Arg Ser Lys Val Val Ser Cys Pro
            435                 440
```

<210> 20
<211> 444
<212> PRT
<213> Homo sapiens

<400> 20

```
        Met Leu Leu Met Val Val Ser Met Ala Cys Val Gly Phe Phe Leu Val
        1               5                   10                  15
```

```
Gln Arg Ala Gly Pro His Val Gly Gly Gln Asp Lys Pro Phe Leu Ser
            20                  25                  30

Ala Trp Pro Ser Ala Val Val Pro Arg Gly Gly His Val Thr Leu Arg
            35                  40                  45

Cys His Tyr Arg His Arg Phe Asn Asn Phe Met Leu Tyr Lys Glu Asp
            50                  55                  60

Arg Ile His Val Pro Ile Phe His Gly Arg Leu Phe Gln Glu Ser Phe
65                  70                  75                  80

Asn Met Ser Pro Val Thr Thr Ala His Ala Gly Asn Tyr Thr Cys Arg
                85                  90                  95

Gly Ser His Pro His Ser Pro Thr Gly Trp Ser Ala Pro Ser Asn Pro
            100                 105                 110

Val Val Ile Met Val Thr Gly Asn His Arg Lys Pro Ser Leu Leu Ala
            115                 120                 125

His Pro Gly Pro Leu Val Lys Ser Gly Glu Arg Val Ile Leu Gln Cys
    130                 135                 140

Trp Ser Asp Ile Met Phe Glu His Phe Phe Leu His Lys Glu Gly Ile
145                 150                 155                 160

Ser Lys Asp Pro Ser Arg Leu Val Gly Gln Ile His Asp Gly Val Ser
            165                 170                 175

Lys Ala Asn Phe Ser Ile Gly Pro Met Met Leu Ala Leu Ala Gly Thr
            180                 185                 190

Tyr Arg Cys Tyr Gly Ser Val Thr His Thr Pro Tyr Gln Leu Ser Ala
    195                 200                 205

Pro Ser Asp Pro Leu Asp Ile Val Val Thr Gly Pro Tyr Glu Lys Pro
    210                 215                 220

Ser Leu Ser Ala Gln Pro Gly Pro Lys Val Gln Ala Gly Glu Ser Val
225                 230                 235                 240

Thr Leu Ser Cys Ser Ser Arg Ser Ser Tyr Asp Met Tyr His Leu Ser
            245                 250                 255

Arg Glu Gly Gly Ala His Glu Arg Arg Leu Pro Ala Val Arg Lys Val
```

                    260                    265                    270

Asn Arg Thr Phe Gln Ala Asp Phe Pro Leu Gly Pro Ala Thr His Gly
         275             280             285

Gly Thr Tyr Arg Cys Phe Gly Ser Phe Arg His Ser Pro Tyr Glu Trp
         290             295             300

Ser Asp Pro Ser Asp Pro Leu Leu Val Ser Val Thr Gly Asn Pro Ser
305             310             315             320

Ser Ser Trp Pro Ser Pro Thr Glu Pro Ser Ser Lys Ser Gly Asn Pro
             325             330             335

Arg His Leu His Ile Leu Ile Gly Thr Ser Val Val Ile Ile Leu Phe
         340             345             350

Ile Leu Leu Leu Phe Phe Leu Leu His Leu Trp Cys Ser Asn Lys Lys
         355             360             365

Asn Ala Ala Val Met Asp Gln Glu Pro Ala Gly Asn Arg Thr Ala Asn
         370             375             380

Ser Glu Asp Ser Asp Glu Gln Asp Pro Glu Glu Val Thr Tyr Ala Gln
385             390             395             400

Leu Asp His Cys Val Phe Thr Gln Arg Lys Ile Thr Arg Pro Ser Gln
             405             410             415

Arg Pro Lys Thr Pro Pro Thr Asp Thr Ile Leu Tyr Thr Glu Leu Pro
             420             425             430

Asn Ala Lys Pro Arg Ser Lys Val Val Ser Cys Pro
             435             440

<210> 21
<211> 768
<212> DNA
<213> Homo sapiens

<220>
<221> CDS
<222> (1)..(768)

<400> 21

```
atg gga aac agc tgt tac aac ata gta gcc act ctg ttg ctg gtc ctc        48
Met Gly Asn Ser Cys Tyr Asn Ile Val Ala Thr Leu Leu Leu Val Leu
1               5                   10                  15


aac ttt gag agg aca aga tca ttg cag gat cct tgt agt aac tgc cca        96
```

```
      Asn Phe Glu Arg Thr Arg Ser Leu Gln Asp Pro Cys Ser Asn Cys Pro
                  20              25              30

      gct ggt aca ttc tgt gat aat aac agg aat cag att tgc agt ccc tgt          144
      Ala Gly Thr Phe Cys Asp Asn Asn Arg Asn Gln Ile Cys Ser Pro Cys
                  35              40              45

      cct cca aat agt ttc tcc agc gca ggt gga caa agg acc tgt gac ata          192
      Pro Pro Asn Ser Phe Ser Ser Ala Gly Gly Gln Arg Thr Cys Asp Ile
                  50              55              60

      tgc agg cag tgt aaa ggt gtt ttc agg acc agg aag gag tgt tcc tcc          240
      Cys Arg Gln Cys Lys Gly Val Phe Arg Thr Arg Lys Glu Cys Ser Ser
      65              70              75              80

      acc agc aat gca gag tgt gac tgc act cca ggg ttt cac tgc ctg ggg          288
      Thr Ser Asn Ala Glu Cys Asp Cys Thr Pro Gly Phe His Cys Leu Gly
                  85              90              95

      gca gga tgc agc atg tgt gaa cag gat tgt aaa caa ggt caa gaa ctg          336
      Ala Gly Cys Ser Met Cys Glu Gln Asp Cys Lys Gln Gly Gln Glu Leu
                  100             105             110

      aca aaa aaa ggt tgt aaa gac tgt tgc ttt ggg aca ttt aac gat cag          384
      Thr Lys Lys Gly Cys Lys Asp Cys Cys Phe Gly Thr Phe Asn Asp Gln
                  115             120             125

      aaa cgt ggc atc tgt cga ccc tgg aca aac tgt tct ttg gat gga aag          432
      Lys Arg Gly Ile Cys Arg Pro Trp Thr Asn Cys Ser Leu Asp Gly Lys
                  130             135             140

      tct gtg ctt gtg aat ggg acg aag gag agg gac gtg gtc tgt gga cca          480
      Ser Val Leu Val Asn Gly Thr Lys Glu Arg Asp Val Val Cys Gly Pro
      145             150             155             160

      tct cca gcc gac ctc tct ccg gga gca tcc tct gtg acc ccg cct gcc          528
      Ser Pro Ala Asp Leu Ser Pro Gly Ala Ser Ser Val Thr Pro Pro Ala
                  165             170             175

      cct gcg aga gag cca gga cac tct ccg cag atc atc tcc ttc ttt ctt          576
      Pro Ala Arg Glu Pro Gly His Ser Pro Gln Ile Ile Ser Phe Phe Leu
                  180             185             190

      gcg ctg acg tcg act gcg ttg ctc ttc ctg ctg ttc ttc ctc acg ctc          624
      Ala Leu Thr Ser Thr Ala Leu Leu Phe Leu Leu Phe Phe Leu Thr Leu
                  195             200             205

      cgt ttc tct gtt gtt aaa cgg ggc aga aag aaa ctc ctg tat ata ttc          672
      Arg Phe Ser Val Val Lys Arg Gly Arg Lys Lys Leu Leu Tyr Ile Phe
                  210             215             220

      aaa caa cca ttt atg aga cca gta caa act act caa gag gaa gat ggc          720
      Lys Gln Pro Phe Met Arg Pro Val Gln Thr Thr Gln Glu Glu Asp Gly
      225             230             235             240

      tgt agc tgc cga ttt cca gaa gaa gaa gaa gga gga tgt gaa ctg tga          768
      Cys Ser Cys Arg Phe Pro Glu Glu Glu Glu Gly Gly Cys Glu Leu
                  245             250             255
```

<210> 22
<211> 255

56

EP 3 560 505 B1

<212> PRT
<213> Homo sapiens

<400> 22

```
Met Gly Asn Ser Cys Tyr Asn Ile Val Ala Thr Leu Leu Val Leu
1               5               10              15

Asn Phe Glu Arg Thr Arg Ser Leu Gln Asp Pro Cys Ser Asn Cys Pro
            20              25              30

Ala Gly Thr Phe Cys Asp Asn Asn Arg Asn Gln Ile Cys Ser Pro Cys
            35              40              45

Pro Pro Asn Ser Phe Ser Ser Ala Gly Gly Gln Arg Thr Cys Asp Ile
    50              55              60

Cys Arg Gln Cys Lys Gly Val Phe Arg Thr Arg Lys Glu Cys Ser Ser
65              70              75              80

Thr Ser Asn Ala Glu Cys Asp Cys Thr Pro Gly Phe His Cys Leu Gly
            85              90              95

Ala Gly Cys Ser Met Cys Glu Gln Asp Cys Lys Gln Gly Gln Glu Leu
            100             105             110

Thr Lys Lys Gly Cys Lys Asp Cys Cys Phe Gly Thr Phe Asn Asp Gln
            115             120             125

Lys Arg Gly Ile Cys Arg Pro Trp Thr Asn Cys Ser Leu Asp Gly Lys
    130             135             140

Ser Val Leu Val Asn Gly Thr Lys Glu Arg Asp Val Val Cys Gly Pro
145             150             155             160

Ser Pro Ala Asp Leu Ser Pro Gly Ala Ser Ser Val Thr Pro Pro Ala
            165             170             175

Pro Ala Arg Glu Pro Gly His Ser Pro Gln Ile Ile Ser Phe Phe Leu
            180             185             190

Ala Leu Thr Ser Thr Ala Leu Leu Phe Leu Leu Phe Phe Leu Thr Leu
            195             200             205

Arg Phe Ser Val Val Lys Arg Gly Arg Lys Lys Leu Leu Tyr Ile Phe
    210             215             220

Lys Gln Pro Phe Met Arg Pro Val Gln Thr Thr Gln Glu Glu Asp Gly
225             230             235             240

Cys Ser Cys Arg Phe Pro Glu Glu Glu Glu Gly Gly Cys Glu Leu
            245             250             255
```

58

<210> 23
<211> 1578
<212> DNA
<213> Homo sapiens

<220>
<221> CDS
<222> (1)..(1578)

<400> 23

```
atg tgg gag gct cag ttc ctg ggc ttg ctg ttt ctg cag ccg ctt tgg      48
Met Trp Glu Ala Gln Phe Leu Gly Leu Leu Phe Leu Gln Pro Leu Trp
1               5                   10                  15

gtg gct cca gtg aag cct ctc cag cca ggg gct gag gtc ccg gtg gtg      96
Val Ala Pro Val Lys Pro Leu Gln Pro Gly Ala Glu Val Pro Val Val
                20                  25                  30

tgg gcc cag gag ggg gct cct gcc cag ctc ccc tgc agc ccc aca atc     144
Trp Ala Gln Glu Gly Ala Pro Ala Gln Leu Pro Cys Ser Pro Thr Ile
            35                  40                  45

ccc ctc cag gat ctc agc ctt ctg cga aga gca ggg gtc act tgg cag     192
Pro Leu Gln Asp Leu Ser Leu Leu Arg Arg Ala Gly Val Thr Trp Gln
        50                  55                  60

cat cag cca gac agt ggc ccg ccc gct gcc gcc ccc ggc cat ccc ctg     240
His Gln Pro Asp Ser Gly Pro Pro Ala Ala Ala Pro Gly His Pro Leu
65                  70                  75                  80

gcc ccc ggc cct cac ccg gcg gcg ccc tcc tcc tgg ggg ccc agg ccc     288
Ala Pro Gly Pro His Pro Ala Ala Pro Ser Ser Trp Gly Pro Arg Pro
                85                  90                  95

cgc cgc tac acg gtg ctg agc gtg ggt ccc gga ggc ctg cgc agc ggg     336
Arg Arg Tyr Thr Val Leu Ser Val Gly Pro Gly Gly Leu Arg Ser Gly
                100                 105                 110

agg ctg ccc ctg cag ccc cgc gtc cag ctg gat gag cgc ggc cgg cag     384
Arg Leu Pro Leu Gln Pro Arg Val Gln Leu Asp Glu Arg Gly Arg Gln
            115                 120                 125

cgc ggg gac ttc tcg cta tgg ctg cgc cca gcc cgg cgc gcg gac gcc     432
Arg Gly Asp Phe Ser Leu Trp Leu Arg Pro Ala Arg Arg Ala Asp Ala
        130                 135                 140

ggc gag tac cgc gcc gcg gtg cac ctc agg gac cgc gcc ctc tcc tgc     480
Gly Glu Tyr Arg Ala Ala Val His Leu Arg Asp Arg Ala Leu Ser Cys
145                 150                 155                 160

cgc ctc cgt ctg cgc ctg ggc cag gcc tcg atg act gcc agc ccc cca     528
Arg Leu Arg Leu Arg Leu Gly Gln Ala Ser Met Thr Ala Ser Pro Pro
                165                 170                 175

gga tct ctc aga gcc tcc gac tgg gtc att ttg aac tgc tcc ttc agc     576
Gly Ser Leu Arg Ala Ser Asp Trp Val Ile Leu Asn Cys Ser Phe Ser
                180                 185                 190
```

```
cgc cct gac cgc cca gcc tct gtg cat tgg ttc cgg aac cgg ggc cag        624
Arg Pro Asp Arg Pro Ala Ser Val His Trp Phe Arg Asn Arg Gly Gln
        195                 200                 205

ggc cga gtc cct gtc cgg gag tcc ccc cat cac cac tta gcg gaa agc        672
Gly Arg Val Pro Val Arg Glu Ser Pro His His His Leu Ala Glu Ser
    210                 215                 220

ttc ctc ttc ctg ccc caa gtc agc ccc atg gac tct ggg ccc tgg ggc        720
Phe Leu Phe Leu Pro Gln Val Ser Pro Met Asp Ser Gly Pro Trp Gly
225                 230                 235                 240

tgc atc ctc acc tac aga gat ggc ttc aac gtc tcc atc atg tat aac        768
Cys Ile Leu Thr Tyr Arg Asp Gly Phe Asn Val Ser Ile Met Tyr Asn
                245                 250                 255

ctc act gtt ctg ggt ctg gag ccc cca act ccc ttg aca gtg tac gct        816
Leu Thr Val Leu Gly Leu Glu Pro Pro Thr Pro Leu Thr Val Tyr Ala
            260                 265                 270

gga gca ggt tcc agg gtg ggg ctg ccc tgc cgc ctg cct gct ggt gtg        864
Gly Ala Gly Ser Arg Val Gly Leu Pro Cys Arg Leu Pro Ala Gly Val
        275                 280                 285

ggg acc cgg tct ttc ctc act gcc aag tgg act cct cct ggg gga ggc        912
Gly Thr Arg Ser Phe Leu Thr Ala Lys Trp Thr Pro Pro Gly Gly Gly
    290                 295                 300

cct gac ctc ctg gtg act gga gac aat ggc gac ttt acc ctt cga cta        960
Pro Asp Leu Leu Val Thr Gly Asp Asn Gly Asp Phe Thr Leu Arg Leu
305                 310                 315                 320

gag gat gtg agc cag gcc cag gct ggg acc tac acc tgc cat atc cat       1008
Glu Asp Val Ser Gln Ala Gln Ala Gly Thr Tyr Thr Cys His Ile His
                325                 330                 335

ctg cag gaa cag cag ctc aat gcc act gtc aca ttg gca atc atc aca       1056
Leu Gln Glu Gln Gln Leu Asn Ala Thr Val Thr Leu Ala Ile Ile Thr
                340                 345                 350

gtg act ccc aaa tcc ttt ggg tca cct gga tcc ctg ggg aag ctg ctt       1104
Val Thr Pro Lys Ser Phe Gly Ser Pro Gly Ser Leu Gly Lys Leu Leu
            355                 360                 365

tgt gag gtg act cca gta tct gga caa gaa cgc ttt gtg tgg agc tct       1152
Cys Glu Val Thr Pro Val Ser Gly Gln Glu Arg Phe Val Trp Ser Ser
        370                 375                 380

ctg gac acc cca tcc cag agg agt ttc tca gga cct tgg ctg gag gca       1200
Leu Asp Thr Pro Ser Gln Arg Ser Phe Ser Gly Pro Trp Leu Glu Ala
385                 390                 395                 400

cag gag gcc cag ctc ctt tcc cag cct tgg caa tgc cag ctg tac cag       1248
Gln Glu Ala Gln Leu Leu Ser Gln Pro Trp Gln Cys Gln Leu Tyr Gln
                405                 410                 415

ggg gag agg ctt ctt gga gca gca gtg tac ttc aca gag ctg tct agc       1296
Gly Glu Arg Leu Leu Gly Ala Ala Val Tyr Phe Thr Glu Leu Ser Ser
            420                 425                 430

cca ggt gcc caa cgc tct ggg aga gcc cca ggt gcc ctc cca gca ggc       1344
Pro Gly Ala Gln Arg Ser Gly Arg Ala Pro Gly Ala Leu Pro Ala Gly
```

                435                         440                         445

cac ctc ctg ctg ttt ctc atc ctt ggt gtc ctt tct ctg ctc ctt ttg          1392
His Leu Leu Leu Phe Leu Ile Leu Gly Val Leu Ser Leu Leu Leu Leu
    450                     455                     460

gtg act gga gcc ttt ggc ttt cac ctt tgg aga aga cag tgg cga cca          1440
Val Thr Gly Ala Phe Gly Phe His Leu Trp Arg Arg Gln Trp Arg Pro
465                     470                     475                     480

aga cga ttt tct gcc tta gag caa ggg att cac cct ccg cag gct cag          1488
Arg Arg Phe Ser Ala Leu Glu Gln Gly Ile His Pro Pro Gln Ala Gln
            485                     490                     495

agc aag ata gag gag ctg gag caa gaa ccg gag ccg gag ccg gag ccg          1536
Ser Lys Ile Glu Glu Leu Glu Gln Glu Pro Glu Pro Glu Pro Glu Pro
            500                     505                     510

gaa ccg gag ccc gag ccc gag ccc gag ccg gag cag ctc tga              1578
Glu Pro Glu Pro Glu Pro Glu Pro Glu Pro Glu Gln Leu
            515                     520                     525

<210> 24
<211> 525
<212> PRT
<213> Homo sapiens

<400> 24

```
Met Trp Glu Ala Gln Phe Leu Gly Leu Leu Phe Leu Gln Pro Leu Trp
1               5               10              15

Val Ala Pro Val Lys Pro Leu Gln Pro Gly Ala Glu Val Pro Val Val
            20              25              30

Trp Ala Gln Glu Gly Ala Pro Ala Gln Leu Pro Cys Ser Pro Thr Ile
        35              40              45

Pro Leu Gln Asp Leu Ser Leu Leu Arg Arg Ala Gly Val Thr Trp Gln
    50              55              60

His Gln Pro Asp Ser Gly Pro Pro Ala Ala Ala Pro Gly His Pro Leu
65              70              75              80

Ala Pro Gly Pro His Pro Ala Ala Pro Ser Ser Trp Gly Pro Arg Pro
            85              90              95

Arg Arg Tyr Thr Val Leu Ser Val Gly Pro Gly Gly Leu Arg Ser Gly
        100             105             110

Arg Leu Pro Leu Gln Pro Arg Val Gln Leu Asp Glu Arg Gly Arg Gln
    115             120             125

Arg Gly Asp Phe Ser Leu Trp Leu Arg Pro Ala Arg Arg Ala Asp Ala
```

```
        130                    135                      140


        Gly Glu Tyr Arg Ala Ala Val His Leu Arg Asp Arg Ala Leu Ser Cys
        145                    150                  155                  160


        Arg Leu Arg Leu Arg Leu Gly Gln Ala Ser Met Thr Ala Ser Pro Pro
                        165                  170                  175


        Gly Ser Leu Arg Ala Ser Asp Trp Val Ile Leu Asn Cys Ser Phe Ser
                    180                  185                  190


        Arg Pro Asp Arg Pro Ala Ser Val His Trp Phe Arg Asn Arg Gly Gln
                    195                  200                  205


        Gly Arg Val Pro Val Arg Glu Ser Pro His His His Leu Ala Glu Ser
            210                  215                  220


        Phe Leu Phe Leu Pro Gln Val Ser Pro Met Asp Ser Gly Pro Trp Gly
        225                  230                  235                  240


        Cys Ile Leu Thr Tyr Arg Asp Gly Phe Asn Val Ser Ile Met Tyr Asn
                        245                  250                  255


        Leu Thr Val Leu Gly Leu Glu Pro Pro Thr Pro Leu Thr Val Tyr Ala
                    260                  265                  270


        Gly Ala Gly Ser Arg Val Gly Leu Pro Cys Arg Leu Pro Ala Gly Val
                    275                  280                  285


        Gly Thr Arg Ser Phe Leu Thr Ala Lys Trp Thr Pro Pro Gly Gly Gly
                    290                  295                  300


        Pro Asp Leu Leu Val Thr Gly Asp Asn Gly Asp Phe Thr Leu Arg Leu
        305                  310                  315                  320


        Glu Asp Val Ser Gln Ala Gln Ala Gly Thr Tyr Thr Cys His Ile His
                        325                  330                  335


        Leu Gln Glu Gln Gln Leu Asn Ala Thr Val Thr Leu Ala Ile Ile Thr
                    340                  345                  350


        Val Thr Pro Lys Ser Phe Gly Ser Pro Gly Ser Leu Gly Lys Leu Leu
                    355                  360                  365


        Cys Glu Val Thr Pro Val Ser Gly Gln Glu Arg Phe Val Trp Ser Ser
            370                  375                  380
```

```
Leu Asp Thr Pro Ser Gln Arg Ser Phe Ser Gly Pro Trp Leu Glu Ala
385             390             395             400

Gln Glu Ala Gln Leu Leu Ser Gln Pro Trp Gln Cys Gln Leu Tyr Gln
                405             410             415

Gly Glu Arg Leu Leu Gly Ala Ala Val Tyr Phe Thr Glu Leu Ser Ser
                420             425             430

Pro Gly Ala Gln Arg Ser Gly Arg Ala Pro Gly Ala Leu Pro Ala Gly
                435             440             445

His Leu Leu Leu Phe Leu Ile Leu Gly Val Leu Ser Leu Leu Leu Leu
                450             455             460

Val Thr Gly Ala Phe Gly Phe His Leu Trp Arg Arg Gln Trp Arg Pro
465             470             475             480

Arg Arg Phe Ser Ala Leu Glu Gln Gly Ile His Pro Pro Gln Ala Gln
                485             490             495

Ser Lys Ile Glu Glu Leu Glu Gln Glu Pro Glu Pro Glu Pro Glu Pro
                500             505             510

Glu Pro Glu Pro Glu Pro Glu Pro Glu Pro Glu Gln Leu
                515             520             525
```

<210> 25
<211> 906
<212> DNA
<213> Homo sapiens

<220>
<221> CDS
<222> (1)..(906)

<400> 25

```
atg ttt tca cat ctt ccc ttt gac tgt gtc ctg ctg ctg ctg ctg cta        48
Met Phe Ser His Leu Pro Phe Asp Cys Val Leu Leu Leu Leu Leu Leu
1               5                   10                  15

cta ctt aca agg tcc tca gaa gtg gaa tac aga gcg gag gtc ggt cag        96
Leu Leu Thr Arg Ser Ser Glu Val Glu Tyr Arg Ala Glu Val Gly Gln
            20                  25                  30

aat gcc tat ctg ccc tgc ttc tac acc cca gcc gcc cca ggg aac ctc       144
Asn Ala Tyr Leu Pro Cys Phe Tyr Thr Pro Ala Ala Pro Gly Asn Leu
            35                  40                  45

gtg ccc gtc tgc tgg ggc aaa gga gcc tgt cct gtg ttt gaa tgt ggc       192
Val Pro Val Cys Trp Gly Lys Gly Ala Cys Pro Val Phe Glu Cys Gly
        50                  55                  60
```

```
aac gtg gtg ctc agg act gat gaa agg gat gtg aat tat tgg aca tcc        240
Asn Val Val Leu Arg Thr Asp Glu Arg Asp Val Asn Tyr Trp Thr Ser
65              70              75              80

aga tac tgg cta aat ggg gat ttc cgc aaa gga gat gtg tcc ctg acc        288
Arg Tyr Trp Leu Asn Gly Asp Phe Arg Lys Gly Asp Val Ser Leu Thr
            85              90              95

ata gag aat gtg act cta gca gac agt ggg atc tac tgc tgc cgg atc        336
Ile Glu Asn Val Thr Leu Ala Asp Ser Gly Ile Tyr Cys Cys Arg Ile
            100             105             110

caa atc cca ggc ata atg aat gat gaa aaa ttt aac ctg aag ttg gtc        384
Gln Ile Pro Gly Ile Met Asn Asp Glu Lys Phe Asn Leu Lys Leu Val
            115             120             125

atc aaa cca gcc aag gtc acc cct gca ccg act cgg cag aga gac ttc        432
Ile Lys Pro Ala Lys Val Thr Pro Ala Pro Thr Arg Gln Arg Asp Phe
            130             135             140

act gca gcc ttt cca agg atg ctt acc acc agg gga cat ggc cca gca        480
Thr Ala Ala Phe Pro Arg Met Leu Thr Thr Arg Gly His Gly Pro Ala
145             150             155             160

gag aca cag aca ctg ggg agc ctc cct gat ata aat cta aca caa ata        528
Glu Thr Gln Thr Leu Gly Ser Leu Pro Asp Ile Asn Leu Thr Gln Ile
            165             170             175

tcc aca ttg gcc aat gag tta cgg gac tct aga ttg gcc aat gac tta        576
Ser Thr Leu Ala Asn Glu Leu Arg Asp Ser Arg Leu Ala Asn Asp Leu
            180             185             190

cgg gac tct gga gca acc atc aga ata ggc atc tac atc gga gca ggg        624
Arg Asp Ser Gly Ala Thr Ile Arg Ile Gly Ile Tyr Ile Gly Ala Gly
            195             200             205

atc tgt gct ggg ctg gct ctg gct ctt atc ttc ggc gct tta att ttc        672
Ile Cys Ala Gly Leu Ala Leu Ala Leu Ile Phe Gly Ala Leu Ile Phe
            210             215             220

aaa tgg tat tct cat agc aaa gag aag ata cag aat tta agc ctc atc        720
Lys Trp Tyr Ser His Ser Lys Glu Lys Ile Gln Asn Leu Ser Leu Ile
225             230             235             240

tct ttg gcc aac ctc cct ccc tca gga ttg gca aat gca gta gca gag        768
Ser Leu Ala Asn Leu Pro Pro Ser Gly Leu Ala Asn Ala Val Ala Glu
            245             250             255

gga att cgc tca gaa gaa aac atc tat acc att gaa gag aac gta tat        816
Gly Ile Arg Ser Glu Glu Asn Ile Tyr Thr Ile Glu Glu Asn Val Tyr
            260             265             270

gaa gtg gag gag ccc aat gag tat tat tgc tat gtc agc agc agg cag        864
Glu Val Glu Glu Pro Asn Glu Tyr Tyr Cys Tyr Val Ser Ser Arg Gln
            275             280             285

caa ccc tca caa cct ttg ggt tgt cgc ttt gca atg cca tag            906
Gln Pro Ser Gln Pro Leu Gly Cys Arg Phe Ala Met Pro
            290             295             300
```

<210> 26

66

<211> 301
<212> PRT
<213> Homo sapiens

<400> 26

<211> 301
<212> PRT
<213> Homo sapiens

```
Met Phe Ser His Leu Pro Phe Asp Cys Val Leu Leu Leu Leu Leu
1           5               10              15

Leu Leu Thr Arg Ser Ser Glu Val Glu Tyr Arg Ala Glu Val Gly Gln
         20              25              30

Asn Ala Tyr Leu Pro Cys Phe Tyr Thr Pro Ala Ala Pro Gly Asn Leu
         35              40              45

Val Pro Val Cys Trp Gly Lys Gly Ala Cys Pro Val Phe Glu Cys Gly
    50              55              60

Asn Val Val Leu Arg Thr Asp Glu Arg Asp Val Asn Tyr Trp Thr Ser
65              70              75              80

Arg Tyr Trp Leu Asn Gly Asp Phe Arg Lys Gly Asp Val Ser Leu Thr
             85              90              95

Ile Glu Asn Val Thr Leu Ala Asp Ser Gly Ile Tyr Cys Cys Arg Ile
         100             105             110

Gln Ile Pro Gly Ile Met Asn Asp Glu Lys Phe Asn Leu Lys Leu Val
         115             120             125

Ile Lys Pro Ala Lys Val Thr Pro Ala Pro Thr Arg Gln Arg Asp Phe
    130             135             140

Thr Ala Ala Phe Pro Arg Met Leu Thr Thr Arg Gly His Gly Pro Ala
145             150             155             160

Glu Thr Gln Thr Leu Gly Ser Leu Pro Asp Ile Asn Leu Thr Gln Ile
             165             170             175

Ser Thr Leu Ala Asn Glu Leu Arg Asp Ser Arg Leu Ala Asn Asp Leu
         180             185             190

Arg Asp Ser Gly Ala Thr Ile Arg Ile Gly Ile Tyr Ile Gly Ala Gly
         195             200             205

Ile Cys Ala Gly Leu Ala Leu Ala Leu Ile Phe Gly Ala Leu Ile Phe
    210             215             220

Lys Trp Tyr Ser His Ser Lys Glu Lys Ile Gln Asn Leu Ser Leu Ile
225             230             235             240
```

```
Ser Leu Ala Asn Leu Pro Pro Ser Gly Leu Ala Asn Ala Val Ala Glu
                245                 250                 255


Gly Ile Arg Ser Glu Glu Asn Ile Tyr Thr Ile Glu Glu Asn Val Tyr
                260                 265                 270


Glu Val Glu Glu Pro Asn Glu Tyr Tyr Cys Tyr Val Ser Ser Arg Gln
                275                 280                 285


Gln Pro Ser Gln Pro Leu Gly Cys Arg Phe Ala Met Pro
    290                 295                 300
```

<210> 27
<211> 735
<212> DNA
<213> Homo sapiens

<220>
<221> CDS
<222> (1)..(735)

<400> 27

```
atg cgc tgg tgt ctc ctc ctg atc tgg gcc cag ggg ctg agg cag gct       48
Met Arg Trp Cys Leu Leu Leu Ile Trp Ala Gln Gly Leu Arg Gln Ala
1               5                   10                  15

ccc ctc gcc tca gga atg atg aca ggc aca ata gaa aca acg ggg aac       96
Pro Leu Ala Ser Gly Met Met Thr Gly Thr Ile Glu Thr Thr Gly Asn
                20                  25                  30

att tct gca gag aaa ggt ggc tct atc atc tta caa tgt cac ctc tcc      144
Ile Ser Ala Glu Lys Gly Gly Ser Ile Ile Leu Gln Cys His Leu Ser
            35                  40                  45

tcc acc acg gca caa gtg acc cag gtc aac tgg gag cag cag gac cag      192
Ser Thr Thr Ala Gln Val Thr Gln Val Asn Trp Glu Gln Gln Asp Gln
        50                  55                  60

ctt ctg gcc att tgt aat gct gac ttg ggg tgg cac atc tcc cca tcc      240
Leu Leu Ala Ile Cys Asn Ala Asp Leu Gly Trp His Ile Ser Pro Ser
65                  70                  75                  80

ttc aag gat cga gtg gcc cca ggt ccc ggc ctg ggc ctc acc ctc cag      288
Phe Lys Asp Arg Val Ala Pro Gly Pro Gly Leu Gly Leu Thr Leu Gln
                85                  90                  95

tcg ctg acc gtg aac gat aca ggg gag tac ttc tgc atc tat cac acc      336
Ser Leu Thr Val Asn Asp Thr Gly Glu Tyr Phe Cys Ile Tyr His Thr
                100                 105                 110

tac cct gat ggg acg tac act ggg aga atc ttc ctg gag gtc cta gaa      384
Tyr Pro Asp Gly Thr Tyr Thr Gly Arg Ile Phe Leu Glu Val Leu Glu
            115                 120                 125

agc tca gtg gct gag cac ggt gcc agg ttc cag att cca ttg ctt gga      432
Ser Ser Val Ala Glu His Gly Ala Arg Phe Gln Ile Pro Leu Leu Gly
```

130               135               140

```
gcc atg gcc gcg acg ctg gtg gtc atc tgc aca gca gtc atc gtg gtg        480
Ala Met Ala Ala Thr Leu Val Val Ile Cys Thr Ala Val Ile Val Val
145             150                 155                 160

gtc gcg ttg act aga aag aag aaa gcc ctc aga atc cat tct gtg gaa        528
Val Ala Leu Thr Arg Lys Lys Lys Ala Leu Arg Ile His Ser Val Glu
                165                 170                 175

ggt gac ctc agg aga aaa tca gct gga cag gag gaa tgg agc ccc agt        576
Gly Asp Leu Arg Arg Lys Ser Ala Gly Gln Glu Glu Trp Ser Pro Ser
                180                 185                 190

gct ccc tca ccc cca gga agc tgt gtc cag gca gaa gct gca cct gct        624
Ala Pro Ser Pro Pro Gly Ser Cys Val Gln Ala Glu Ala Ala Pro Ala
                195                 200                 205

ggg ctc tgt gga gag cag cgg gga gag gac tgt gcc gag ctg cat gac        672
Gly Leu Cys Gly Glu Gln Arg Gly Glu Asp Cys Ala Glu Leu His Asp
    210                 215                 220

tac ttc aat gtc ctg agt tac aga agc ctg ggt aac tgc agc ttc ttc        720
Tyr Phe Asn Val Leu Ser Tyr Arg Ser Leu Gly Asn Cys Ser Phe Phe
225                 230                 235                 240

aca gag act ggt tag                                                    735
Thr Glu Thr Gly
```

<210> 28
<211> 244
<212> PRT
<213> Homo sapiens

<400> 28

```
Met Arg Trp Cys Leu Leu Leu Ile Trp Ala Gln Gly Leu Arg Gln Ala
1               5                   10                  15

Pro Leu Ala Ser Gly Met Met Thr Gly Thr Ile Glu Thr Thr Gly Asn
            20                  25                  30

Ile Ser Ala Glu Lys Gly Gly Ser Ile Ile Leu Gln Cys His Leu Ser
            35                  40                  45

Ser Thr Thr Ala Gln Val Thr Gln Val Asn Trp Glu Gln Gln Asp Gln
        50                  55                  60

Leu Leu Ala Ile Cys Asn Ala Asp Leu Gly Trp His Ile Ser Pro Ser
65                  70                  75                  80

Phe Lys Asp Arg Val Ala Pro Gly Pro Gly Leu Gly Leu Thr Leu Gln
                85                  90                  95

Ser Leu Thr Val Asn Asp Thr Gly Glu Tyr Phe Cys Ile Tyr His Thr
```

```
                100                        105                          110

        Tyr Pro Asp Gly Thr Tyr Thr Gly Arg Ile Phe Leu Glu Val Leu Glu
                115                 120                 125

        Ser Ser Val Ala Glu His Gly Ala Arg Phe Gln Ile Pro Leu Leu Gly
                130                 135                 140

        Ala Met Ala Ala Thr Leu Val Val Ile Cys Thr Ala Val Ile Val Val
        145                 150                 155                 160

        Val Ala Leu Thr Arg Lys Lys Lys Ala Leu Arg Ile His Ser Val Glu
                        165                 170                 175

        Gly Asp Leu Arg Arg Lys Ser Ala Gly Gln Glu Glu Trp Ser Pro Ser
                        180                 185                 190

        Ala Pro Ser Pro Pro Gly Ser Cys Val Gln Ala Glu Ala Ala Pro Ala
                195                 200                 205

        Gly Leu Cys Gly Glu Gln Arg Gly Glu Asp Cys Ala Glu Leu His Asp
                210                 215                 220

        Tyr Phe Asn Val Leu Ser Tyr Arg Ser Leu Gly Asn Cys Ser Phe Phe
        225                 230                 235                 240

        Thr Glu Thr Gly
```

<210> 29
<211> 834
<212> DNA
<213> Homo sapiens

<220>
<221> CDS
<222> (1)..(834)

<400> 29

```
atg tgc gtg ggg gct cgg cgg ctg ggc cgc ggg ccg tgt gcg gct ctg        48
Met Cys Val Gly Ala Arg Arg Leu Gly Arg Gly Pro Cys Ala Ala Leu
1               5                   10                  15

ctc ctc ctg ggc ctg ggg ctg agc acc gtg acg ggg ctc cac tgt gtc        96
Leu Leu Leu Gly Leu Gly Leu Ser Thr Val Thr Gly Leu His Cys Val
            20                  25                  30

ggg gac acc tac ccc agc aac gac cgg tgc tgc cac gag tgc agg cca       144
Gly Asp Thr Tyr Pro Ser Asn Asp Arg Cys Cys His Glu Cys Arg Pro
            35                  40                  45

ggc aac ggg atg gtg agc cgc tgc agc cgc tcc cag aac acg gtg tgc       192
```

Gly Asn Gly Met Val Ser Arg Cys Ser Arg Ser Gln Asn Thr Val Cys
    50              55              60

cgt ccg tgc ggg ccg ggc ttc tac aac gac gtg gtc agc tcc aag ccg     240
Arg Pro Cys Gly Pro Gly Phe Tyr Asn Asp Val Val Ser Ser Lys Pro
65              70              75              80

tgc aag ccc tgc acg tgg tgt aac ctc aga agt ggg agt gag cgg aag     288
Cys Lys Pro Cys Thr Trp Cys Asn Leu Arg Ser Gly Ser Glu Arg Lys
            85              90              95

cag ctg tgc acg gcc aca cag gac aca gtc tgc cgc tgc cgg gcg ggc     336
Gln Leu Cys Thr Ala Thr Gln Asp Thr Val Cys Arg Cys Arg Ala Gly
            100             105             110

acc cag ccc ctg gac agc tac aag cct gga gtt gac tgt gcc ccc tgc     384
Thr Gln Pro Leu Asp Ser Tyr Lys Pro Gly Val Asp Cys Ala Pro Cys
        115             120             125

cct cca ggg cac ttc tcc cca ggc gac aac cag gcc tgc aag ccc tgg     432
Pro Pro Gly His Phe Ser Pro Gly Asp Asn Gln Ala Cys Lys Pro Trp
        130             135             140

acc aac tgc acc ttg gct ggg aag cac acc ctg cag ccg gcc agc aat     480
Thr Asn Cys Thr Leu Ala Gly Lys His Thr Leu Gln Pro Ala Ser Asn
145             150             155             160

agc tcg gac gca atc tgt gag gac agg gac ccc cca gcc acg cag ccc     528
Ser Ser Asp Ala Ile Cys Glu Asp Arg Asp Pro Pro Ala Thr Gln Pro
            165             170             175

cag gag acc cag ggc ccc ccg gcc agg ccc atc act gtc cag ccc act     576
Gln Glu Thr Gln Gly Pro Pro Ala Arg Pro Ile Thr Val Gln Pro Thr
            180             185             190

gaa gcc tgg ccc aga acc tca cag gga ccc tcc acc cgg ccc gtg gag     624
Glu Ala Trp Pro Arg Thr Ser Gln Gly Pro Ser Thr Arg Pro Val Glu
            195             200             205

gtc ccc ggg ggc cgt gcg gtt gcc gcc atc ctg ggc ctg ggc ctg gtg     672
Val Pro Gly Gly Arg Ala Val Ala Ala Ile Leu Gly Leu Gly Leu Val
        210             215             220

ctg ggg ctg ctg ggc ccc ctg gcc atc ctg ctg gcc ctg tac ctg ctc     720
Leu Gly Leu Leu Gly Pro Leu Ala Ile Leu Leu Ala Leu Tyr Leu Leu
225             230             235             240

cgg agg gac cag agg ctg ccc ccc gat gcc cac aag ccc cct ggg gga     768
Arg Arg Asp Gln Arg Leu Pro Pro Asp Ala His Lys Pro Pro Gly Gly
            245             250             255

ggc agt ttc cgg acc ccc atc caa gag gag cag gcc gac gcc cac tcc     816
Gly Ser Phe Arg Thr Pro Ile Gln Glu Glu Gln Ala Asp Ala His Ser
            260             265             270

acc ctg gcc aag atc tga     834
Thr Leu Ala Lys Ile
            275

<210> 30
<211> 277

<212> PRT
<213> Homo sapiens

<400> 30

```
Met Cys Val Gly Ala Arg Arg Leu Gly Arg Gly Pro Cys Ala Ala Leu
1               5               10              15

Leu Leu Leu Gly Leu Gly Leu Ser Thr Val Thr Gly Leu His Cys Val
            20              25              30

Gly Asp Thr Tyr Pro Ser Asn Asp Arg Cys Cys His Glu Cys Arg Pro
            35              40              45

Gly Asn Gly Met Val Ser Arg Cys Ser Arg Ser Gln Asn Thr Val Cys
    50              55              60

Arg Pro Cys Gly Pro Gly Phe Tyr Asn Asp Val Val Ser Ser Lys Pro
65              70              75              80

Cys Lys Pro Cys Thr Trp Cys Asn Leu Arg Ser Gly Ser Glu Arg Lys
                85              90              95

Gln Leu Cys Thr Ala Thr Gln Asp Thr Val Cys Arg Cys Arg Ala Gly
            100             105             110

Thr Gln Pro Leu Asp Ser Tyr Lys Pro Gly Val Asp Cys Ala Pro Cys
        115             120             125

Pro Pro Gly His Phe Ser Pro Gly Asp Asn Gln Ala Cys Lys Pro Trp
    130             135             140

Thr Asn Cys Thr Leu Ala Gly Lys His Thr Leu Gln Pro Ala Ser Asn
145             150             155             160

Ser Ser Asp Ala Ile Cys Glu Asp Arg Asp Pro Pro Ala Thr Gln Pro
                165             170             175

Gln Glu Thr Gln Gly Pro Pro Ala Arg Pro Ile Thr Val Gln Pro Thr
        180             185             190

Glu Ala Trp Pro Arg Thr Ser Gln Gly Pro Ser Thr Arg Pro Val Glu
        195             200             205

Val Pro Gly Gly Arg Ala Val Ala Ala Ile Leu Gly Leu Gly Leu Val
    210             215             220

Leu Gly Leu Leu Gly Pro Leu Ala Ile Leu Leu Ala Leu Tyr Leu Leu
225             230             235             240
```

```
Arg Arg Asp Gln Arg Leu Pro Pro Asp Ala His Lys Pro Pro Gly Gly
            245                 250             255

Gly Ser Phe Arg Thr Pro Ile Gln Glu Glu Gln Ala Asp Ala His Ser
        260                 265             270

Thr Leu Ala Lys Ile
            275
```

<210> 31
<211> 726
<212> DNA
<213> Homo sapiens

<220>
<221> CDS
<222> (1)..(726)

<400> 31

```
atg aag aca ttg cct gcc atg ctt gga act ggg aaa tta ttt tgg gtc        48
Met Lys Thr Leu Pro Ala Met Leu Gly Thr Gly Lys Leu Phe Trp Val
1               5                   10                  15

ttc ttc tta atc cca tat ctg gac atc tgg aac atc cat ggg aaa gaa        96
Phe Phe Leu Ile Pro Tyr Leu Asp Ile Trp Asn Ile His Gly Lys Glu
            20                  25                  30

tca tgt gat gta cag ctt tat ata aag aga caa tct gaa cac tcc atc       144
Ser Cys Asp Val Gln Leu Tyr Ile Lys Arg Gln Ser Glu His Ser Ile
        35                  40                  45

tta gca gga gat ccc ttt gaa cta gaa tgc cct gtg aaa tac tgt gct       192
Leu Ala Gly Asp Pro Phe Glu Leu Glu Cys Pro Val Lys Tyr Cys Ala
    50                  55                  60

aac agg cct cat gtg act tgg tgc aag ctc aat gga aca aca tgt gta       240
Asn Arg Pro His Val Thr Trp Cys Lys Leu Asn Gly Thr Thr Cys Val
65                  70                  75                  80

aaa ctt gaa gat aga caa aca agt tgg aag gaa gag aag aac att tca       288
Lys Leu Glu Asp Arg Gln Thr Ser Trp Lys Glu Glu Lys Asn Ile Ser
                85                  90                  95

ttt ttc att cta cat ttt gaa cca gtg ctt cct aat gac aat ggg tca       336
Phe Phe Ile Leu His Phe Glu Pro Val Leu Pro Asn Asp Asn Gly Ser
            100                 105                 110

tac cgc tgt tct gca aat ttt cag tct aat ctc att gaa agc cac tca       384
Tyr Arg Cys Ser Ala Asn Phe Gln Ser Asn Leu Ile Glu Ser His Ser
        115                 120                 125

aca act ctt tat gtg aca gga aag caa aat gaa ctc tct gac aca gca       432
Thr Thr Leu Tyr Val Thr Gly Lys Gln Asn Glu Leu Ser Asp Thr Ala
        130                 135                 140

gga agg gaa att aac ctg gtt gat gct cac ctt aag agt gag caa aca       480
Gly Arg Glu Ile Asn Leu Val Asp Ala His Leu Lys Ser Glu Gln Thr
145                 150                 155                 160
```

```
gaa gca agc acc agg caa aat tcc caa gta ctg cta tca gaa act gga    528
Glu Ala Ser Thr Arg Gln Asn Ser Gln Val Leu Leu Ser Glu Thr Gly
            165                 170                 175

att tat gat aat gac cct gac ctt tgt ttc agg atg cag gaa ggg tct    576
Ile Tyr Asp Asn Asp Pro Asp Leu Cys Phe Arg Met Gln Glu Gly Ser
            180                 185                 190

gaa gtt tat tct aat cca tgc ctg gaa gaa aac aaa cca ggc att gtt    624
Glu Val Tyr Ser Asn Pro Cys Leu Glu Glu Asn Lys Pro Gly Ile Val
            195                 200                 205

tat gct tcc ctg aac cat tct gtc att gga ccg aac tca aga ctg gca    672
Tyr Ala Ser Leu Asn His Ser Val Ile Gly Pro Asn Ser Arg Leu Ala
            210                 215                 220

aga aat gta aaa gaa gca cca aca gaa tat gca tcc ata tgt gtg agg    720
Arg Asn Val Lys Glu Ala Pro Thr Glu Tyr Ala Ser Ile Cys Val Arg
225                 230                 235                 240

agt taa                                                            726
Ser
```

<210> 32
<211> 241
<212> PRT
<213> Homo sapiens

<400> 32

```
Met Lys Thr Leu Pro Ala Met Leu Gly Thr Gly Lys Leu Phe Trp Val
1               5               10                  15

Phe Phe Leu Ile Pro Tyr Leu Asp Ile Trp Asn Ile His Gly Lys Glu
            20                  25                  30

Ser Cys Asp Val Gln Leu Tyr Ile Lys Arg Gln Ser Glu His Ser Ile
            35                  40                  45

Leu Ala Gly Asp Pro Phe Glu Leu Glu Cys Pro Val Lys Tyr Cys Ala
            50                  55                  60

Asn Arg Pro His Val Thr Trp Cys Lys Leu Asn Gly Thr Thr Cys Val
65                  70                  75                  80

Lys Leu Glu Asp Arg Gln Thr Ser Trp Lys Glu Glu Lys Asn Ile Ser
                85                  90                  95

Phe Phe Ile Leu His Phe Glu Pro Val Leu Pro Asn Asp Asn Gly Ser
            100                 105                 110

Tyr Arg Cys Ser Ala Asn Phe Gln Ser Asn Leu Ile Glu Ser His Ser
            115                 120                 125
```

```
Thr Thr Leu Tyr Val Thr Gly Lys Gln Asn Glu Leu Ser Asp Thr Ala
    130             135             140

Gly Arg Glu Ile Asn Leu Val Asp Ala His Leu Lys Ser Glu Gln Thr
145             150             155             160

Glu Ala Ser Thr Arg Gln Asn Ser Gln Val Leu Leu Ser Glu Thr Gly
                165             170             175

Ile Tyr Asp Asn Asp Pro Asp Leu Cys Phe Arg Met Gln Glu Gly Ser
            180             185             190

Glu Val Tyr Ser Asn Pro Cys Leu Glu Glu Asn Lys Pro Gly Ile Val
        195             200             205

Tyr Ala Ser Leu Asn His Ser Val Ile Gly Pro Asn Ser Arg Leu Ala
    210             215             220

Arg Asn Val Lys Glu Ala Pro Thr Glu Tyr Ala Ser Ile Cys Val Arg
225             230             235             240

Ser
```

**Description**

Technical Field

Technical Field:

The present invention relates to a medicament for treating cognitive impairment and/or a neurodegenerative disease, comprising an inactivated Sendai virus envelope as an active ingredient and combined application of the medicament and an immune checkpoint inhibitor.

Background Art

Dementia is a cognitive impairment that causes difficulties in daily life and social life due to chronical decline or loss of normally developed mind functions by acquired structural disorder of the brain. There are several kinds of dementia such as Alzheimer-type dementia, vascular dementia, dementia with Lewy bodies, and frontotemporal dementia, but Alzheimer-type dementia among others is most common, being said to account for 60% of the causes of dementia.

While neurodegeneration due to the accumulation of amyloid β is presumed to be most likely, there are various hypotheses about the cause of the Alzheimer-type dementia and the connection with the immune system is also suggested (Non Patent Literatures 1 and 2). Some medical treatments have also been developed for dementia and particularly Alzheimer-type dementia, but no radical treatment has been developed.

Sendai virus (Hemaglutinating virus of Japan (HVJ)) have attracted attention for causing fusion of Erich tumor cells. Its activity of fusing the cell membrane (hereinafter, referred to as the fusing activity) has been analyzed and the use of HVJ as a transfection vector has also been studied. However, HVJ has high immunogenicity and, when NP protein is produced in a large quantity, it is known to induce CTL in particular and there is also concern that the protein synthesis of the host is inhibited. Accordingly, a method for producing fused particles (HVJ-liposome) by fusing liposomes encapsulating a gene or a protein and HVJ inactivated by ultraviolet irradiation has been developed and this has made non-invasive transfection of cells and living bodies possible (Patent Literature 1, Non-Patent Literatures 3 and 4).

The present inventors have developed a novel hybrid transfection vector by combining a virus having high potency (high efficiency) of gene delivery and a nonviral vector having low cytotoxicity and immunogenicity (low toxicity) and constructed fusogenic viral liposome having a fusogenic envelope derived from HVJ. In this delivery system, DNA loaded liposome is fused with UV inactivated HVJ to form HVJ-liposome (400-500 nm in diameter), a fusogenic virus-liposome. The advantage of the fusion-mediated delivery is that transfected DNA is protected from the endosomal degradation and lysosomal degradation in acceptor cells. For example, DNA incorporated in HVJ-liposome can safely be delivered to mammalian cells (Patent Literature 2). Moreover, RNA, oligonucleotides, and drugs can efficiently be introduced into cells in vitro and in vivo. Furthermore, the present inventors have invented a transfection vector encapsulating an exogenous gene by freeze-thawing or mixing the HVJ envelope (HVJ-E) with a surfactant as a transfection vector having a virus envelope, being available for safely and stably transfecting a wide range of tissues in the living body, and having high transfection activity (Patent Literatures 3 and 4). This made it possible to efficiently introduce a substance into the brain or central nerves using HVJ-E (Patent Literatures 5 and 6). Furthermore, the inventors have found that HVJ-E exhibits immunological adjuvant effect by encapsulating a chemotherapeutic agent such as an anticancer agent into HVJ-E and transfecting the HVJ-E into cells or the living body (Patent Literatures 7 and 8).

The inventors have found that HVJ-E itself has the antitumor immunity-activating effect and the cancer cell-specific cell death-inducing effect and is useful as an anticancer agent (Patent Literatures 8 and 9) and have been conducting a doctor-led clinical trial for malignant melanoma patients, prostate cancer patients, and malignant pleural mesothelioma patients.

Citation List

Patent Literature

Patent Literature 1: U.S. Patent No. 5631237
Patent Literature 2: WO2001/057204
Patent Literature 3: Japanese Patent Laid-Open No. 2002-065278
Patent Literature 4: WO2004/035779
Patent Literature 5: WO2006/011600

Patent Literature 6: WO2005/095613
Patent Literature 7: WO2004/039406
Patent Literature 8: WO2005/094878
Patent Literature 9: WO2010/032764

Non Patent Literature

Non Patent Literature 1: Schenk et al., Nature. 1999 Jul 8, 400 (6740), p. 173-177
Non Patent Literature 2: Baruch et al., Nature Medicine, 2016 Feb vol. 22, No. 2, p. 135-137
Non Patent Literature 3: Dzau et al., PNAS 1996, Vol. 93, No. 21, p. 11421-11425
Non Patent Literature 4: Kaneda et al., Mol. Med. Today, 1999, Vol. 5, Issue 7, p. 298-303

Summary of Invention

Technical Problem

An object of the invention is to find a novel effect of HVJ-E and establish its clinical application.

Solution to Problem

The inventors have found that administration of HVJ-E to model mice of Alzheimer's disease created by administration of β amyloid leads to significant improvement of short-term memory. Furthermore, it has been found that combined application of an anti-PD-1 antibody with HVJ-E leads to synergistic improvement of the effect.
The present invention has been completed based on the aforementioned findings and relates to an inactivated Sendai virus envelope for use in a method for preventing and/or treating cognitive impairment and/or a neurodegenerative disease with accumulation of a prionoid, as defined in the claims.

Advantageous Effects of Invention

The present invention provides a new therapeutic for use in a method for treating and/or preventing cognitive impairment including Alzheimer-type dementia and/or a neurodegenerative disease. Cognitive impairment or a neurodegenerative disease with accumulation of a prionoid such as β amyloid, for example, Alzheimer-type dementia, can be treated with HVJ-E more effectively by a combined application with an immune checkpoint inhibitor. The combination effect of HVJ-E and an immune checkpoint inhibitor is synergistic and therefore the combined application can decrease the doses of the agents in comparison with single administration and reduce side effects.

Brief Description of Drawings

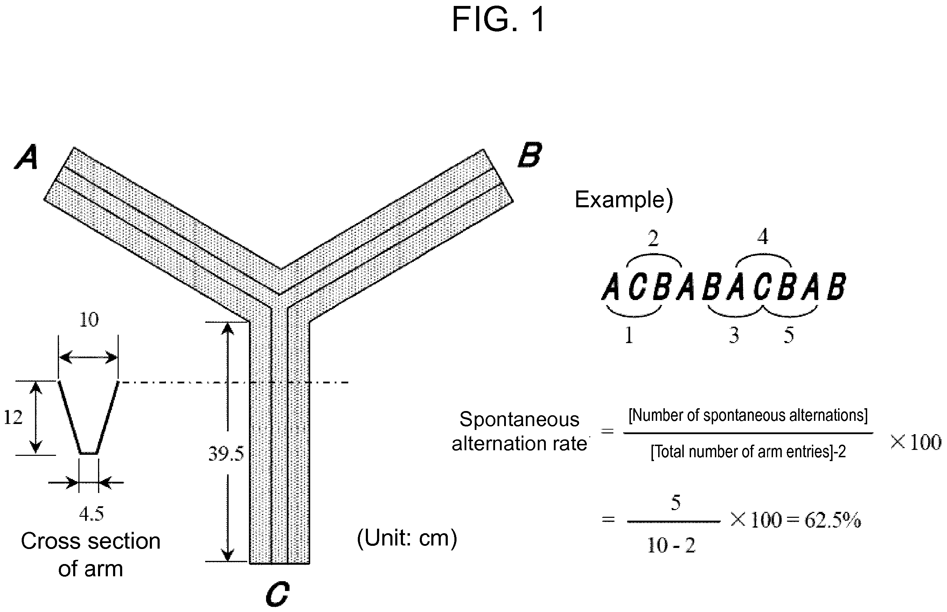

[Figure 1] Figure 1 illustrates an apparatus for the Y-maze test and a method for calculating the spontaneous alternation rate. In the figure, as illustrated in the calculation example, if an animal moves in the order of ACBABACBAB, when the arms of the Y-shaped maze are referred to as A, B, and C, then the number of spontaneous alternation is 5 of ACB, CBA, BAC, ACB, CBA and this is divided by 8, which is the number obtained by subtracting 2 from the total number of entries 10, and multiplied by 100 to obtain a spontaneous alternation rate of 62.5. The spontaneous alternation rate (%) = [5/(10-2)] $\times$ 100 = 62.5%

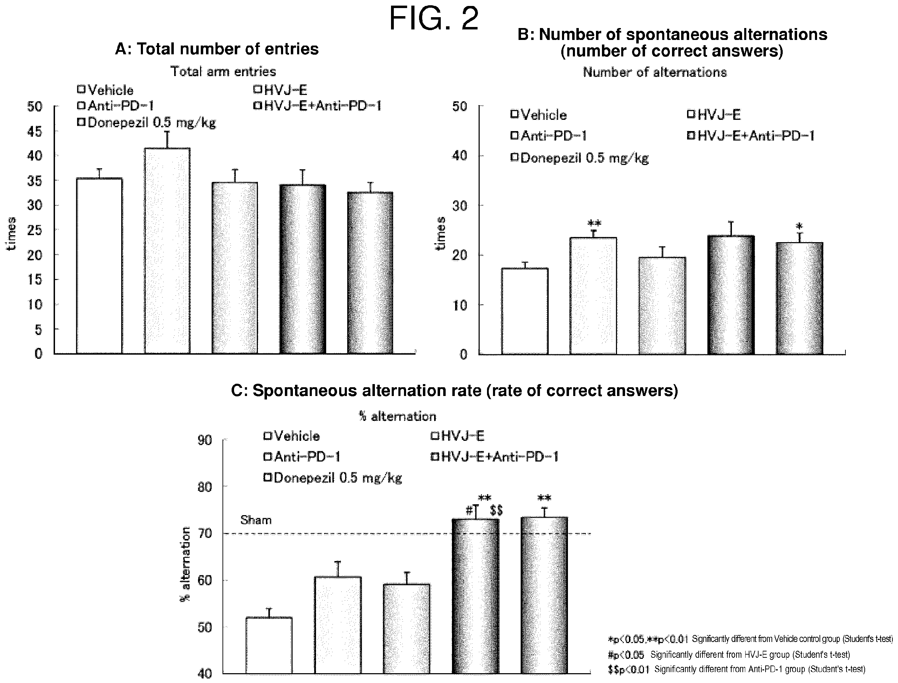

[Figure 2] Figure 2 illustrates the result (HVJ-E is administered subcutaneously) of the Y-maze test. Figure 2A: the number of total entries, Figure 2B: the number of spontaneous alternation (the number of correct answers), and Figure 2C: the spontaneous alternation rate (the rate of correct answer). From the left in all the panels, the negative control group (vehicle administered group), the HVJ-E administered group, the anti-PD-1 antibody administered group, the HVJ-E+ anti-PD-1 antibody combined application group, and the positive control group (donepezil administered group).

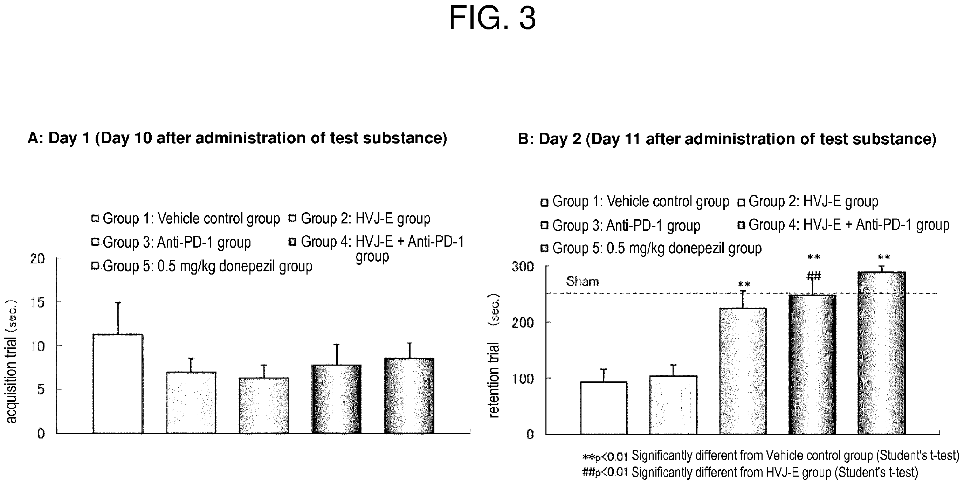

[Figure 3] Figure 3 illustrates the result (HVJ-E is administered subcutaneously) of the passive test (reaction latency). Figure 3A: Day 1 (Day 10 after the administration of the test substance), Figure 3B: Day 2 (Day 11 after the administration of the test substance). From the left in all the panels, the negative control group (vehicle administered group), the HVJ-E administered group, the anti-PD-1 antibody administered group, the HVJ-E + anti-PD-1 antibody combined application group, and the positive control group (donepezil administered group).

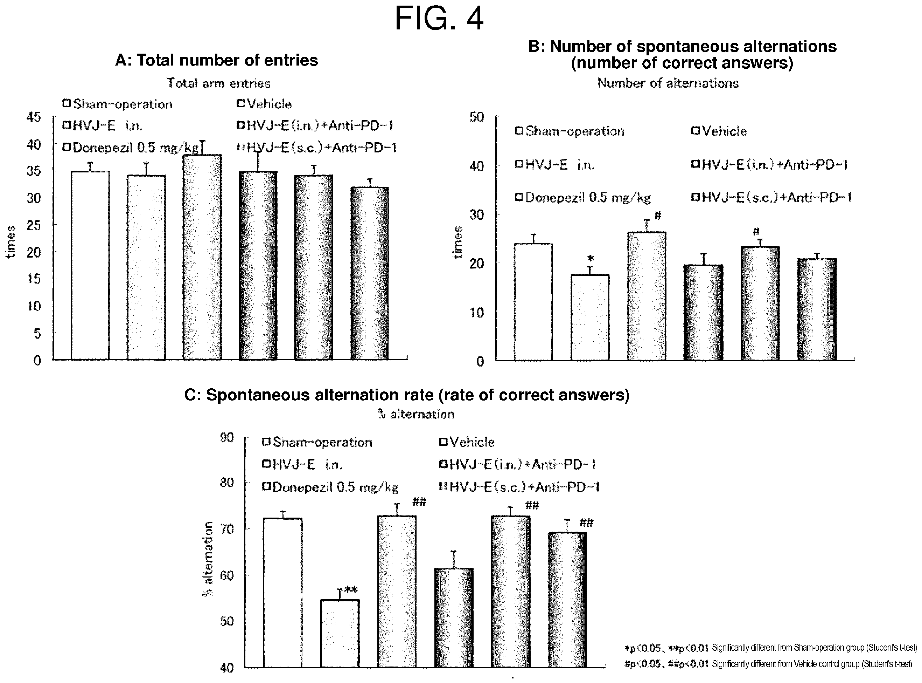

[Figure 4] Figure 4 illustrates the result of the Y-maze test (HVJ-E is administered intranasally). Figure 4A: the number of total entries, Figure 4B: the number of spontaneous alternation (the number of correct answers), and Figure 4C: the spontaneous alternation rate (the rate of correct answer). From the left in all the panels, the negative control group (vehicle administered group), the HVJ-E intranasally administered group, the HVJ-E (intranasal) + anti-PD-1 antibody combined application group, the positive control group (donepezil administered group), and the HVJ-E (subcutaneous) + anti-PD-1 antibody combined application group. In Figure 4, Sham-operation indicates a sham operation group, Vehicle indicates the vehicle administered group, i.n. indicates intranasal administration, and s.c. indicates subcutaneous administration.

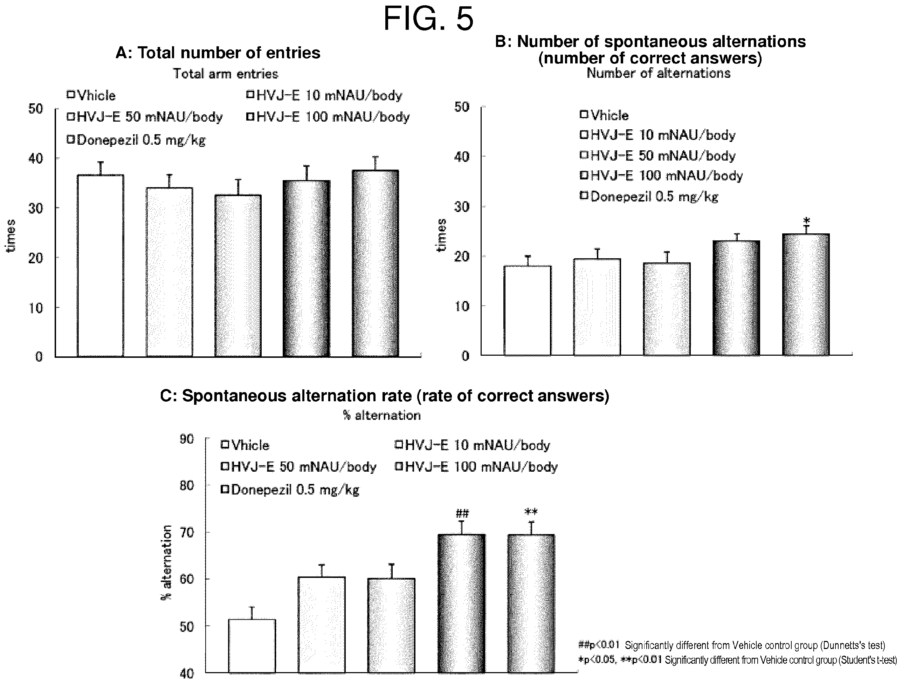

[Figure 5] Figure 5 illustrates the result of the Y-maze test (HVJ-E is administered intranasally). Figure 5A: the number of total entries, Figure 5B: the number of spontaneous alternation (the number of correct answers), and Figure 5C: the spontaneous alternation rate (the rate of correct answer). From the left in all the panels, the negative control group (vehicle administered group), the HVJ-E (10 mNAU/body) nasal administered group, the HVJ-E (50 mNAU/body) nasal administered group, the HVJ-E (100 mNAU/body) nasal administered group, and the positive control group (donepezil administered group). In Figure 5, Vehicle indicates the vehicle administered group.

Description of Embodiments

The present invention relates to a medicament for use in a method for preventing and/or treating cognitive impairment and/or a neurodegenerative disease with accumulation of a prionoid, comprising an inactivated Sendai virus envelope (HVJ-E) as an active ingredient.

1. Medicament comprising Sendai virus envelope "Sendai virus (HVJ)"

The "Sendai virus (HVJ)" is a single strand minus strand RNA virus in the genus Respirovirus of the family Paramyxoviridae, infects mice and rats, and causes a respiratory disease. On the outer side of the virus particle, an envelope, which is a membranous structure, exists, and covers the virus genome and capsid proteins. The envelope proteins are membrane components responsible for the cell fusion activity when the virus adheres to and invades a host cell and play an important role in viral infection, such as the immune evasion.

"Sendai virus envelope (HVJ-E)"

The "Sendai virus envelope (HVJ-E)" according to the present invention is an inactivating Sendai virus which has been inactivated and lost the replication competence (Kaneda et al., Hemagglutinating Virus of Japan (HVJ) Envelope Vector as a Versatile Gene Delivery System. Mol. Ther. 2002, 6, 219-226). HVJ-E does not have the infectivity and the proliferative capacity in the host since the viral genomic RNA has been completely inactivated,

The Hemagglutinin-Neuraminidase (HN) protein and the Fusion (F) protein, which are proteins on the membrane of HVJ-E, maintain the cell membrane fusion ability even after the viral RNA inactivation. Therefore, it is possible to encapsulate a gene or a drug in the inactivated HVJ envelope and deliver to cells of interest. Moreover, HVJ-E can be used for the treatment of cancer alone or encapsulating an anticancer agent since it has the adjuvant effect of increasing tumor immunity (mentioned above, WO2005/094878, WO2010/032764). It has been not known before the present application that HVJ-E has the preventive and therapeutic effect on cognitive impairment and neurodegenerative diseases with the accumulation of a prionoid such as Alzheimer's disease.

"Preparation of HVJ-E"

The HVJ-E according to the present invention can be obtained by inactivating HVJ. The HVJ to be used may be the wild type or a mutant with an appropriate modification as long as it does not cause deteriorating effect on the purpose as a medicament. Examples of the wild type HVJ that can be used include commercially available ATCC (R) VR-105(TM), ATCC (R) VR-907(TM), and the like. The virus is preferably purified prior to the inactivation by a combination of centrifugation or ultrafiltration and an ion exchange column.

Examples of the mutant HVJ include HVJ-E in which HN has been deleted by using an siRNA specific to the mRNA or the like; HVJ-E modified to express a fusion protein of a modified HN protein and a desired protein on the outer surface of the HVJ-E by connecting a desired polypeptide to the C-terminal of the particular region of the HN protein composing the HVJ-E (see Japanese Patent Laid-Open No. 2011-050292); other HVJ-Es in which a membrane protein is modified, and the like.

The inactivation of the virus is not particularly limited as long as it eliminates the replication competence of the virus in host cells, preferably human cells and may be carried out by ultraviolet irradiation, gamma ray irradiation, chemical treatment (alkylating agent treatment), heat-treatment, or the like, but ultraviolet irradiation and gamma beam irradiation are preferred from the viewpoint that the effect on proteins on the envelope membrane is small. In the case of ultraviolet irradiation and gamma ray irradiation, the virus can be inactivated by irradiating a suspension of HVJ with an ultraviolet ray (usually around 90 to 200 millijoules/cm$^2$) or a gamma ray (usually around 5 to 20 grays). Moreover, in the case of the alkylating agent treatment, the virus may be inactivated by adding an alkylating agent such as β propiolactone to a suspension of the HVJ and incubating the suspension. Specific methods for preparing HVJ-E are described in detail in Kaneda et al., 2002, WO01/57204 (Example 8), Japanese Patent Laid-Open No. 2002-065278, and WO03/014338 mentioned above.

"Medicament comprising HVJ-E"

The medicament comprising HVJ-E according to the present invention may comprise a pharmacologically acceptable carrier and/or an additive. Examples of such a carrier and an additive include a filler, a binder, a lubricant, a solvent, a disintegrator, a solubilizing agent, a suspending agent, an emulsifier, an isotonizing agent, a stabilizer, a soothing agent, an antiseptic, an antioxidant, a corrigent, a colorant, a buffer, a superplasticizer, and the like, but are not limited thereto and other commonly used carriers and/or additives may be used.

Specifically, examples of the filler include organic fillers such as saccharides such as lactose, glucose, and D-mannitol, starches, and cellulose such as crystalline cellulose; inorganic fillers such as calcium carbonate and kaolin; and the like.

Examples of the binder include pregelatinized starch, gelatin, Arabian gum, methylcellulose, carboxymethylcellulose, sodium carboxymethylcellulose, microcrystalline cellulose, D-mannitol, trehalose, hydroxypropylcellulose, hydroxypropylmethylcellulose, polyvinylpyrrolidone, polyvinyl alcohol, and the like.

Examples of the lubricant include stearic acid, fatty acid salts such as stearates, talc, silicates, and the like.

Examples of the solvent include purified water, physiological saline solutions, phosphate buffer solutions, and the like.

Examples of the disintegrator include low-substituted hydroxypropyl cellulose, chemically modified cellulose and starches, and the like.

Examples of the solubilizing agent include polyethyleneglycol, propylene glycol, trehalose, benzyl benzoate, ethanol, sodium carbonate, sodium citrate, sodium salicylate, sodium acetate, and the like.

Examples of the suspending agent or emulsifier include sodium lauryl sulfate, Arabian gum, gelatin, lecithin, glyceryl monostearate, polyvinyl alcohol, polyvinylpyrrolidone, cellulose such as sodium carboxymethylcellulose, polysorbate, polyoxyethylene hydrogenated castor oils, and the like.

Examples of the isotonizing agent include sodium chloride, potassium chloride, saccharide, glycerin, urea, and the like.

Examples of the stabilizer include polyethyleneglycol, dextran sodium sulfate, amino acids, and the like.

Examples of the soothing agent include glucose, calcium gluconate, procaine hydrochloride, and the like.

Examples of the antiseptic include p-hydroxybenzoates, chlorobutanol, benzyl alcohol, phenethyl alcohol, dehydroacetic acid, sorbic acid, and the like.

Examples of the antioxidant include water-soluble antioxidants such as ascorbic acid, cysteine hydrochloride, sodium bisulfate, sodium metabisulfite, and sodium sulfite; liposoluble antioxidants such as ascorbyl palmitate, butylated hydroxyanisole (BHA), butylated hydroxytoluene (BHT), lecithin, propyl gallate, and α-tocopherol; and metal chelating agents such as citric acid, ethylenediaminetetraacetic acid (EDTA), sorbitol, tartaric acid, and phosphoric acid.

Examples of the corrigent include sweeteners and flavors commonly used in the field of medicament, and the like and examples of the colorant include coloring agents commonly used in the field of medicament.

HVJ-E may be formulated with a polymer such as hyaluronic acid, polylactic acid, alginic acid, and gelatin and/or a cationization agent such as cationized gelatin (Japanese Patent Laid-Open No. 2008-308,440), besides others.

The route of administration of the medicament according to the present invention is not particularly limited and the administration may be oral or parenteral administration. Specific examples of the parenteral administration include injection, transnasal administration, pulmonary administration, transdermal administration, and the like. Examples of the injection include intravenous injection, intramuscular injection, intraperitoneal injection, subcutaneous injection, intraosseous injection, intraspinal injection, and intradermal injection. The mode of administration can be selected by the age and symptoms of the patient.

The dose of the medicament according to the present invention can determined depending on the purpose of its use and the route of administration. When administered to humans, a dose, for example, in the range of 200 mNAU to 20,000 mNAU in terms of HVJ-E per dosing may be selected. Alternatively, a dose for example, in the range of 3,000 to 60,000 mNAU/body per patient may be selected.

The medicament according to the present invention may be administered in combination with another agent as long as it does not cause deteriorating effect on the purpose of the present invention. As described above, a nucleic acid or an agent may be encapsulated in HVJ-E. Also, in the present invention, an agent or the like useful for the treatment of cognitive impairment or a neurodegenerative disease may be encapsulated in HVJ-E as long as it does not cause deteriorating effect on the purpose of the present invention.

"Cognitive impairment and/or neurodegenerative disease"

The medicament comprising HVJ-E is useful for the treatment and/or prophylaxis of cognitive impairment with the accumulation of a prionoid and/or a neurodegenerative disease with the accumulation of a prionoid.

The "prionoid" is a protein that is considered to cause the expansion of a lesion(s) by the transmission of a released missfolded protein to neighboring cells, and examples of the prionoid include amyloid $\beta$, $\alpha$-synuclein, huntingtin, phosphorylated tau, and the like. Representative examples of the prionoid are "amyloid $\beta$" and "$\alpha$-synuclein" described below.

The "amyloid $\beta$" is a peptide that is a key component of the senile plaque, which is a main pathological change in Alzheimer-type dementia. The amyloid $\beta$ (hereinafter, also referred to as "A$\beta$") is a highly hydrophobic peptide that is produced by cutting out by $\beta$- and $\gamma$-secretase from the precursor protein (APP: amyloid $\beta$ protein precursor) and includes A$\beta_{1-40}$ consisting of 40 amino acids and A$\beta_{1-42}$ consisting of 42 amino acids. A$\beta_{1-42}$ is more fibrillogenic than A$\beta_{1-40}$ and a hypothesis that A$\beta_{1-42}$ forms aggregates and then A$\beta_{1-40}$ aggregates on the A$\beta_{1-42}$ aggregates core to form fibrils has been proposed. However, it is considered that the modification by another risk factor(s) (such as apolipoprotein E and presenilin 1.2) is necessary for the accumulation of amyloid $\beta$. A$\beta$ has the nerve cell toxicity and causes necrosis and the like.

The "$\alpha$-synuclein" is a protein having unknown functions that is mainly found in neural tissues and consists of 140 amino acid residues encoded by the SNCA gene. The $\alpha$-synuclein was first discovered as a component in the amyloid whose fragments are accumulated in Alzheimer disease, but it is considered to be a cause of neurodegenerative diseases including Parkinson disease.

Examples of the "cognitive impairment and/or a neurodegenerative disease with accumulation of a prionoid" include, but are not limited to, Alzheimer-type dementia, mild cognitive impairment (MCI), cerebral amyloid angiopathy, dementia with Lewy bodies, Parkinson's disease, tauopathy, and Creutzfeldt-Jakob disease, and the like.

In the present invention, the term "treatment" means that various symptoms caused by cognitive impairment and/or a neurodegenerative disease with accumulation of a prionoid are improved by administering the medicament according to the present invention to a subject. Moreover, the word "prophylaxis" means that the onset and exacerbation of the cognitive impairment and/or neurodegenerative disease with accumulation of a prionoid is prevented.

2. Combination of HVJ-E and immune checkpoint inhibitor

The inventors have confirmed that administration of an immune checkpoint inhibitor to model mice of Alzheimer's disease leads to a behavioral improvement and also demonstrated that the effect thereof exhibits synergistic effect by a combined application with HVJ-E. More specifically, the present invention also provides a medicament for preventing and/or treating cognitive impairment and/or a neurodegenerative disease with accumulation of a prionoid by combining (using a combination of or mixing) a Sendai virus envelope (HVJ-E) and an immune checkpoint inhibitor.

"Medicament comprising HVJ-E and immune checkpoint inhibitor in combination"

In the present invention, the "medicament for preventing and/or treating cognitive impairment and/or a neurodegenerative disease with accumulation of a prionoid, comprising HVJ-E and an immune checkpoint inhibitor in combination" means a medicament in which HVJ-E and an immune checkpoint inhibitor are combined for administering them simultaneously, separately, or sequentially in prophylaxis and/or treatment of a neurodegenerative disease. In one embodiment, the medicament according to the present invention is provided in a form of a combination drug comprising HVJ-E and an immune checkpoint inhibitor together. Moreover, in another embodiment, the medicament according to the present invention is provided as a combined application of separate agents comprising HVJ-E and an immune checkpoint inhibitor and the agents are used simultaneously or sequentially. Furthermore, in another embodiment, the medicament according to the present invention may be provided as a kit composed of an agent(s) comprising HVJ-E and an immune checkpoint inhibitor.

In the present invention, the "combined application" of HVJ-E and an immune checkpoint inhibitor means administration or use of HVJ-E and an immune checkpoint inhibitor in combination and the order or intervals of the administration thereof is not limited. Moreover, the "combination drug" of HVJ-E and an immune checkpoint inhibitor means a medicament formulated by combining certain amounts of HVJ-E and an immune checkpoint inhibitor (fixed dose combination drug).

The combined administration (use) of HVJ-E and an immune checkpoint inhibitor allows lower doses than the doses in use of either of the drugs alone and therefore can reduce the risk of side effects.

"Immune checkpoint inhibitor"

The "immune checkpoint inhibitor" means to inhibit the immune checkpoint and inhibit the effect of substances and/or molecules that suppress the defense system of acquired immunity. The immune checkpoint is a molecule that suppresses the defense system of acquired immunity to pathogens such as cancer cells, bacteria, and viruses and examples thereof include PD-1, which is expressed on the surface of effector T cells, PD-L1 and PD-L2, which are expressed on the surface of tumor cells, CTLA-4, which is expressed on the surface of activated T cells or the suppressor T cell Treg, and CD80 (B7-1) and CD86 (B7-2), which are expressed on the surface of dendritic cells, as well as B7-H3, B7-H4, B7-H5 (VISTA), KIR, CD137, LAG-3, TIM-3, TIGIT, OX40, BTLA, and the like.

The amino acid sequences of the aforementioned immune checkpoint inhibitors and the nucleotide sequences of the genes encoding them are already known and disclosed in public databases (the nucleic acid sequences: GenBank, DDBJ, and EMBL, the amino acid sequences: SwissProt, PIR, and PDB). For example, the information of the gene of human PD-1 (Homo sapiens programmed cell death 1 (PDCD1), mRNA) is disclosed as GenBank Accession No. NM 005018 and the information of the protein thereof (programmed cell death protein 1 precursor [Homo sapiens]) is disclosed as Accession No. NP 005009. The immune checkpoint inhibitors (for example, antisense nucleic acids, siRNA, shRNA, and antibodies) described below can be designed and obtained based on the disclosed information using a technology known in the field. The information about the immune checkpoint inhibitors are shown together in the following.

[Table 1]

| Immun-Checkpoint | mRNA, GenBank Accession No. | Nucleotide sequence (cDNA) | Amino acid sequence |
|---|---|---|---|
| hPD-1 | NM_005018.2 (Gene ID: 5133) | SEQ ID NO: 1 | SEQ ID NO: 2 |
| hPD-L1 | NM_001267706.1 (Gene ID: 29126) | SEQ ID NO: 3 | SEQ ID NO: 4 |
| hPD-L2 | NM_025239.3 (Gene ID: 80380) | SEQ ID NO: 5 | SEQ ID NO: 6 |
| hCTLA-4 | NM_001037631.2 (Gene ID: 1493) | SEQ ID NO: 7 | SEQ ID NO: 8 |
| hCD80 (B7-1) | NM_005191.3 (Gene ID: 941) | SEQ ID NO: 9 | SEQ ID NO: 10 |
| hB7. 2 (B7-2) | NM_001206924.1 (Gene ID: 942) | SEQ ID NO: 11 | SEQ ID NO: 12 |
| hB7-H3 | NM_001024736.1 (Gene ID: 80381) | SEQ ID NO: 13 | SEQ ID NO: 14 |
| hB7-H4 | NM_001253849.1 (Gene ID: 79679) | SEQ ID NO: 15 | SEQ ID NO: 16 |
| hB7-H5(VISTA) | NM_022153.1 (Gene ID: 64115) | SEQ ID NO: 17 | SEQ ID NO: 18 |
| hKIR: | NM_001322168.1 (Gene ID: 3811) | SEQ ID NO: 19 | SEQ ID NO: 20 |
| hCD137 | NM_001561.5 (Gene ID: 3604) | SEQ ID NO: 21 | SEQ ID NO: 22 |
| hLAG-3 | NM_002286.5 (Gene ID: 3902) | SEQ ID NO: 23 | SEQ ID NO: 24 |
| hTIM-3 | NM_032782.4 (Gene ID: 84868) | SEQ ID NO: 25 | SEQ ID NO: 26 |
| hTIGIT | NM_173799.3 (Gene ID: 201633) | SEQ ID NO: 27 | SEQ ID NO: 28 |
| h0X40 | NM_003327.3 (Gene ID: 7293) | SEQ ID NO: 29 | SEQ ID NO: 30 |
| hBTLA | NM_001085357.1 (Gene ID: 151888) | SEQ ID NO: 31 | SEQ ID NO: 32 |

In the present invention, the "immune checkpoint inhibitor" is not particularly limited, as long as it is a substance that suppresses the expression or activity of the aforementioned immune checkpoint.

The "substance that suppresses the expression of the immune checkpoint" may be one that acts at any level, such as at the level of transcription of the immune checkpoint gene, at the level of posttranscriptional regulation, at the level of translation into protein, and at the level of posttranslational modification. Accordingly, examples of the substance that suppresses the expression of the immune checkpoint include a substance that inhibits the transcription of the gene, a substance that inhibits the processing from the initial transcription product to mRNA, a substance that inhibits the transportation of the mRNA to the cytoplasm, a substance that promotes degradation of the mRNA, a substance that inhibits the translation from the mRNA to the protein, a substance that inhibits the posttranslational modification of the immune checkpoint, and the like. A substance that acts on at any of the levels may be preferably used, but a substance that inhibits the translation from the mRNA to the protein is preferred in terms of direct inhibition of the production of the immune checkpoint.

Examples of the substance that specifically inhibits the translation from the mRNA of the immune checkpoint to the protein include a nucleic acid comprising a nucleotide sequence complementary or substantially complementary to the nucleotide sequence of the mRNA encoding the immune checkpoint or a part thereof.

Examples of the nucleotide sequence complementary or substantially complementary to the nucleotide sequence of the mRNA of the immune checkpoint include

(a) nucleotide sequences complementary or substantially complementary to the nucleotide sequence encoding the immune checkpoint, or
(b) a nucleotide sequence that hybridizes with a complementary strand sequence of the nucleotide sequence encoding the immune checkpoint under highly stringent conditions and that is complementary or substantially complementary to a nucleotide sequence encoding a protein having substantially same activity with the immune checkpoint.

The "substantially complementary" refers to having a complementarity of about 70% or more, preferably about 80% or more, more preferably about 90% or more, most preferably about 95% or more between nucleotide sequences. Moreover, the "activity" refers to the effect of suppressing the anti-tumor activity of T cells to cancer cells. The "substantially same" indicates that the activity thereof is qualitatively (e.g., physiologically or pharmacologically) the same. Therefore, quantitative factors such as the degree of activity (e.g., about 0.1 to about 10 times, preferably about 0.5 to about 2 times) or the molecular weight of the protein may be different. The measurement of the immune checkpoint activity may be conducted according to the methods known per se.

Examples of the highly stringent conditions include hybridization at 45°C in 6 × SSC (sodium chloride/sodium citrate) and then washing at 65°C in 0.2 × SSC/0.1 % SDS once or more.

The "part of a nucleotide sequence complementary or substantially complementary to the nucleotide sequence of the mRNA of the immune checkpoint" is not particularly limited, as long as it can specifically bind to mRNA of the immune checkpoint and not particularly limited in length and position, as long as it can inhibit the translation of the protein from the mRNA, but comprises a part that is at least 10 nucleotides or more, preferably about 15 nucleotides or more, and more preferably about 20 nucleotides or more complementary or substantially complementary to the target sequence in terms of the sequence specificity.

Specifically, preferable example of the nucleic acid comprising a nucleotide sequence complementary or substantially complementary to the nucleotide sequence of the mRNA of the immune checkpoint or a part thereof include any of the following (i) to (iii):

(i) an antisense nucleic acid to the mRNA of the immune checkpoint,
(ii) siRNA to the mRNA of the immune checkpoint,
(iii) a nucleic acid that can generate an siRNA to the mRNA of the immune checkpoint.

(i) Antisense nucleic acid to mRNA of immune checkpoint

The antisense nucleic acid to the mRNA of the immune checkpoint in the present invention (the antisense nucleic acid according to the present invention) is a nucleic acid comprising a nucleotide sequence complementary or substantially complementary to the nucleotide sequence of the mRNA or a part thereof and having a function of suppressing protein synthesis by forming a specific and stable double-strand and binding to the target mRNA and having the function of suppressing the protein synthesis. The antisense nucleic acid may be any of a double-stranded DNA, a single stranded DNA, a double-stranded RNA, a single stranded RNA, a DNA:RNA hybrid.

The target region of the antisense nucleic acid is not particularly limited in length as long as it can inhibit the translation of the immune checkpoint as a result by hybridizing with the antisense nucleic acid and may be the full-length sequence of the mRNA encoding the protein or a partial sequence thereof, and includes a short sequence of about 10 nucleotides to a long sequence of the total sequence of the mRNA or the initial transcription product. An oligonucleotide consisting of about 10 to about 40 nucleotides, and in particular about 15 to about 30 nucleotides is preferred in consideration of the issues such as ease of the synthesis, antigenicity, and intracellular translocation, but it is not limited thereto. Specifically, the sequences such as a 5' terminal hairpin loop, 6 nucleotides pair repeats at 5' terminal, a 5' terminal untranslated region, the translation initiation codon, a protein coding region, the ORF translation stop codon, a 3' terminal untranslated region, a 3' terminal palindromic region, or a 3' terminal hairpin loop of the immune checkpoint can be selected as a preferable target region of the antisense nucleic acid, but it is not particularly limited thereto.

The nucleotide molecule that composes the antisense nucleic acid may be natural DNA or RNA, but may have various kinds of chemical modification to improve stability (chemically and/or anti-enzymatically) and specific activity (affinity to RNA). The antisense nucleic acids may be in a form of antigene. Any of the antisense nucleic acid comprising such modification may be chemically synthesized by a technique known per se.

(ii) siRNA to mRNA of immune checkpoint

As used herein, a double-stranded RNA composed of an oligo-RNA complementary to the mRNA of the immune checkpoint and a complementary strand thereof, so-called siRNA, also comprises a nucleotide sequence of the mRNA of the immune checkpoint and a complementary or substantially complementary nucleotide sequence thereof or a part thereof is also defined to be included in the nucleic acid. The siRNA may be designed using commercially available software (e.g.: RNAi Designer; Invitrogen) based on the nucleotide sequence information about the target mRNA.

The ribonucleotide molecule composing the siRNA may also be modified to improve its stability and specific activity similarly to the above-mentioned antisense nucleic acid. However, the RNAi activity may be lost from siRNA in which all ribonucleotide molecules in the natural type RNA are replaced with modified molecules and therefore the introduction of the minimum modified nucleotides that allows the RISC complex to function is necessary.

siRNA can be prepared by respectively synthesizing a sense strand and an antisense strand of the target sequence of the mRNA with a DNA/RNA automatic synthesizer, denaturing at about 90 to about 95°C for about 1 minute in an appropriate annealing buffer solution, and then annealing at about 30 to about 70°C for about 1 to about 8 hours. Moreover, the siRNA can also be prepared by synthesizing short hair pin RNA (shRNA) to be a precursor of siRNA and cutting with the dicer.

(iii) Nucleic acid capable of generating siRNA to mRNA of immune checkpoint

As used herein, nucleic acids designed such that siRNA to the mRNA of the above-mentioned immune checkpoint is produced in the body is also defined to be included in the nucleic acid comprising a nucleotide sequence complementary or substantially complementary to the nucleotide sequence or a part of the mRNA of the immune checkpoint. Examples of such nucleic acids include expression vectors that have been constructed to express the aforementioned shRNA, and the like. shRNA can be prepared by designing an oligo-RNA comprising a nucleotide sequence in which a sense strand and an antisense strand of the spacer sequence of the target sequence on the mRNA are connected by inserting a spacer sequence capable of forming an appropriate loop structure (e.g., about 15 to 25 nucleotides) and synthesizing this with an automatic DNA/RNA synthesizer. The expression vector comprising an expression cassette of shRNA can be prepared by producing a double-stranded DNA encoding the aforementioned shRNA by a conventional method and inserting it in an appropriate expression vector. As an expression vector of the shRNA, a vector having a Pol III promoter such as U6 or H1 origin can be used.

The inhibitory activity of these nucleic acids on the expression of the immune checkpoint can be examined by using a transformant in which a gene of the immune checkpoint has been introduced, in vivo and in vitro gene expression systems of the immune checkpoint, or an in vivo or in vitro protein translation system of the immune checkpoint.

The substance that suppresses the expression of the immune checkpoint according to the present invention is not limited to the aforementioned nucleic acids and may be another substance such as a low molecular weight compound as long as it directly or indirectly inhibits the production of the immune checkpoint.

In the present invention, the "substance that suppresses activity of the immune checkpoint" may be any substance as long as it suppresses an effect of controlling the anti-tumor activity for the cancer cell of the T cell.

Specifically, the "substance that suppresses the activity of the immune checkpoint" includes an antibody specific to the immune checkpoint. The antibody may be either a polyclonal antibody or a monoclonal antibody. These antibodies can be produced according to the manufacturing process of antibodies or antisera publicly known per se. The isotype of the antibody is not particularly limited, but examples thereof include preferably IgG, IgM, or IgA, and particularly preferably IgG. Moreover, the antibody is not particularly limited as long as it has at least a complementarity-determining region (CDR) that specifically recognizes and bind to a target antigen, and may be a complete antibody molecule as well as a fragment such as Fab, Fab', or F(ab')$_2$, a conjugate molecule produced by genetic engineering, such as scFv, scFv-Fc, a minibody, or a diabody, or a derivative thereof that is modified with a molecule having the protein stabilization effect such as polyethyleneglycol (PEG).

Preferred examples of the "immune checkpoint inhibitor" include an anti-PD-1 antibody, an anti-PD-LI antibody, an anti-PD-L2 antibody, an anti-CTLA-4 antibody, an anti-KIR antibody, an anti-CD137 antibody, an anti-LAG-3 antibody, an anti-OX40 antibody, an anti-CD80 antibody, an anti-CD86 antibody, an anti-B7-H3 antibody, an anti-B7-H4 antibody, an anti-B7-H5 antibody, an anti-TIM-3 antibody, an anti-TIGIT antibody, and an anti-BTLA antibody. Among them, an anti-PD-1 antibody, an anti-PD-L1 antibody, and an anti-CTLA-4 antibody are preferred and an anti-PD-1 antibody is more preferred.

The "anti-PD-1 antibody" used in the present invention is not particularly limited as long as it can bind to PD-1 and inhibit the function as an immune checkpoint. Examples of the anti-PD-1 antibody include those already approved and sold as a medicament and those under development and they can be used suitably. Examples of such an "anti-PD-1 antibody" include, but are not limited to, Nivolumab (Opdivo (GSK/ Ono pharmaceutical)), Pembrolizumab (MK-3475 (Merck)), which is a humanized IgG4 antibody, Pidilizumab (CT-011 (CureTech)), REGN-2810/SAR-439684 (Regeneron), PDR-001 (Novartis), AMP-514/MEDI-0680 (Amplimmune), TSR-042 (AnaptysBio), PF-06801591 (Pfizer), JS-001 (Shanghai Junshi Biosciences), IBI-308 (Innovent Biologics), and BGB-A317 (BeiGene).

The "anti-PD-LI antibody" to be used in the present invention is not particularly limited, as long as it can bind to PD-L1 and inhibit the function as an immune checkpoint. Example of the anti-PD-LI antibody include those already approved (sold) as a medicament and those under development and they can be used suitably. Examples of such an "anti-PD-LI antibody" include, but are not limited to, atezolizumab (MPDL3280A/RG-7446 (Roche/Chugai Pharmaceutical Co., Ltd.), Durvalumab (MEDI4736 (AstraZeneca)), avelumab (MSB0010718C (Merck)), MED10680/AMP-514 (Medimmune), MDX-1105/BMS-936559 (BMS), INCAGN-1876 (Agenus), LY-3300054 (Eli Lilly), and CA-170 (Aurigene Discovery Technology)).

The "anti-CTLA-4 antibody" to be used in the present invention is not particularly limited as long as it can bind to CTLA-4 and inhibit the function as an immune checkpoint. Example of the anti-CTLA-4 antibody include those already approved (sold) as a medicament and those under development and they can be used suitably. Examples of such an "anti-CTLA-4 antibody" include, but are not limited to, Ipilimumab (MDX-010), tremelimumab (CP675/206 (Pfizer)), and AGEN-1884 (4-Antibody).

Besides those described above, Lirilumab (IPH2102/BMS-986015), which is an anti-killer cell immunoglobulin-like receptor (KIR) antibody, Urelumab (BMS-663513) and PF -05082566, which are anti-CD137 antibodies, BMS-986016, which is an anti-LAG-3 antibody, MEDI6469, which is an anti-OX40 antibody, MSB0011359C/M-7824(Merck), which is a bi-functional fusion protein that targets PD-LA and TGF-$\beta$ are under development as an immune checkpoint inhibitor.

The antibodies described above may be produced by a conventional method. In that case, the antibody is preferably a chimeric antibody, a humanized antibody, or a fully human antibody to reduce the xenogeneic antigenicity to humans.

The "immune checkpoint inhibitor" according to the present invention may comprise a pharmacologically acceptable carrier and/or an additive. Examples of such a carrier and an additive include a filler, a binder, a lubricant, a solvent, a disintegrator, a solubilizing agent, a suspending agent, an emulsifier, an isotonizing agent, a stabilizer, a soothing agent, an antiseptic, antioxidant, a corrigent, a colorant, a buffer, a superplasticizer, and the like, but are not limited thereto, and other commonly used carriers and/or additives may be used. The specific examples of the carrier and additive are as described above.

The route of administration of the "immune checkpoint inhibitor" of the present invention is not particularly limited. Parenteral administration is preferred. Specific examples of the parenteral administration include injection, transnasal administration, pulmonary administration, transdermal administration, and the like. Examples of the injection include intravenous injection, intramuscular injection, intraperitoneal injection, and subcutaneous injection. The mode of administration can be selected according to the age and symptoms of the patient.

When an immune checkpoint inhibitor and HVJ-E are contained and provided in separate agents in the medicament according to the present invention, the dosage forms and the routes of administration of these agents may be different or the same. Moreover, one or more different preparations may further be combined.

The dose of the "immune checkpoint inhibitor" according to the present invention can be determined based on the purpose of use and the route of administration thereof. The dose is adjusted to provide an optimal response (for example, therapeutic response) desired. Since the active ingredient is an antibody, the dose is in the range of about 0.0001 to 100 mg/kg and usually 0.01 to 5 mg/kg patient body weight. For example, dose is about 0.3 mg/kg of body weight, 1 mg/kg of body weight, 3 mg/kg of body weight, 5 mg/kg of body weight or 10 mg/kg of body weight, or in the range of 1 to 10 mg/kg. Typical regimens are, for example, once a week administration, once in 2 weeks administration, once in 3 weeks administration, once in 4 weeks administration, once a month administration, once in 3 months administration, or once in 3 to 6 months administration. For example, in the case of administration by IV infusion, the dose is 1 mg/kg body weight or 3 mg/kg body weight. The dose frequency can be determined depending on the symptoms. The inhibitor may be administered in a single bolus or divided into several doses to take time. For example, it may be administered 6 times every 4 weeks and then administered every 3 weeks, or may be administered every 3 weeks, or may be administered once at 3 mg/kg body weight and then 1 mg/kg of body weight every 3 weeks.

When HVJ-E and the immune checkpoint inhibitor according to the present invention are administered in combination, the order of administration is not limited, and HVJ-E may be administered after the administration of the immune checkpoint inhibitor, or both may be administered simultaneously, or the immune checkpoint inhibitor may be administered after the administration of HVJ-E. Preferably, the immune checkpoint inhibitor is administered after the administration of HVJ-E.

When HVJ-E and the immune checkpoint inhibitor are sequentially administered, the interval of the administration of HVJ-E and the immune checkpoint inhibitor is not particularly limited and may be set so that desired combinational effect is obtained, in consideration of the route of administration, the dosage form, the dose, the residual concentration, and the like. For example, the interval of administration is 0 hours to 7 days, preferably 0 hours to 3 days, more preferably 0 to 24 hours, and further preferably 0 hours to 12 hours. The interval of administration may be determined, for example, based on the result of the analysis of the blood concentration of the agent or a metabolite thereof in the patient by a known method.

Examples

Hereinafter, the present invention will be described in detail with reference to Examples, but the present invention is not limited by these Examples.

Example 1: Improving effect of administration of single agent of either HVJ-E or anti-PD-1 antibody and combined administration thereof on learning disability in β amyloid single administered model mice

1. Material and method

Test substance:

(1) HVJ-E (batch number: 140523, GenomIdea, Inc.)
(2) LEAF(TM) Purified anti-mouse CD279 (anti-PD-1 antibody, batch number: B203991, BioLegend Inc.) Positive control substance
5 mg Aricept (R) tablet (donepezil hydrochloride, batch number: 61A53K, Eisai Co., Ltd.)

Vehicle:

(1) Metolose (R) SM -100 (methylcellulose (hereinafter referred to as MC), batch number 5065340, Shin-Etsu Chemical Co., Ltd.)
(2) Water for injection (batch number: 5C74N, Otsuka Pharmaceutical Factory, Inc.)
5% trehalose solution (batch number: 160523, GenomIdea, Inc.)
(3) Phosphate-buffered salt (batch number: 1259290, DS Pharma Biomedical Co., Ltd.)

Substance for model preparation:
Amyloid-β Protein (25-35) (β amyloid, batch number: AW14089, PolyPeptide Laboratories)
Vehicle for substance for model preparation:
Water for injection (batch number: K5F71, Otsuka Pharmaceutical Factory, Inc.)

Administration sample:

Preparation of vehicle (0.5% MC)

A required amount of MC is weighed (electronic balance: either XP205DR or PB3002-S/FACT is used, Mettler Toledo International Inc.) and then stirred and prepared to be at a concentration of 0.5 w/v% with water for injection. The prepared 0.5% MC solution is kept under refrigeration (control temperature: 2.0 to 8.0°C) and used within 7 days after the preparation.

Preparation of vehicle (PBS)

Tablets of phosphate-buffered salt are dissolved with 100 mL per tablet of water for injection and sterilized by filtration with a filter (MILLIPORE) with a pore size of 0.22 μm.

Preparation of test substance (HVJ-E)

A required number of test substances are brought to room temperature and aluminum caps are completely removed from the vials leaving only rubber stoppers. A 1 mL disposable syringe (Terumo Corporation) made of polypropylene with a needle (Terumo Corporation) is filled with 1 mL of water for injection. The outside of the rubber stopper is cleaned with ethanol cotton, then the rubber stopper is penetrated with the aforementioned needle attached to the 1 mL disposable syringe made of polypropylene, and the 1 mL per vial of water for injection is added such as to go down along the wall.

The vial is swirled in circle while avoiding making bubbles in the liquid and attaching the liquid to the rubber stopper to completely dissolve the substance and yield a homogeneous white solution. The concentration of HVJ-E in this preparation is 10,000 mNAU/mL (containing 5% trehalose). The rubber stopper is removed and the whole content is transferred into a tube made of polypropylene using a micropipet while avoiding making bubbles in the liquid.

The tube in which the solution has been transferred is immediately placed on ice and diluted with a 5% trehalose aqueous solution to prepare a dosing solution at a predetermined concentration (2,000 mNAU/mL). It is stored on ice after the preparation and brings back to room temperature just before the administration. Preparation of test substance (anti-PD-1 antibody)

The antibody (1 vial: 1 mg/mL) is used as it is. It is stored on ice till the administration and brings back to room temperature just before the administration.

Preparation of positive control substance

It is prepared in terms of a salt thereof (conversion factor: 1.10).

The required number of donepezil tablets is placed in an agate mortar and sufficiently ground and 0.5% MC is gradually added to a predetermined concentration.

Preparation of substance for generation of model

β amyloid is dissolved to 2 mM with water for injection and the mixture is incubated at 37°C for 4 days to prepare a β amyloid solution. The operation is conducted in a clean bench and instruments and containers to be used are those that have been sterilized.

Frequency of preparation

0.5% MC is prepared once or more every week, the substance for the generation of the model is prepared once 4 days before the administration, and the vehicle (PBS), the test substance, and the positive control substance are prepared every time when needed.

Test system

Animal species and strain
Mouse (SPF): Slc: ddY
Male, 5 weeks of age, 50 animals
Range of body weights one day after the acquisition (23 to 28 g)
Source: Japan SLC, Inc.

Quarantine inspection and acclimation

The animals acquired are put in quarantine for a period of 5 days and acclimated during the following 4 days. During this period, the body weight is measured (an electronic balance: one of PB3002, PG2002-S, PB3002-S/FACT, and MS1602S/02 is used, Mettler Toledo International Inc.) 3 times and general conditions are examined once a day as quarantine inspection and acclimation. Animals confirmed to have no change in body weight and no abnormality in general conditions are assigned to groups. Animals found to have change in body weight and abnormality in general conditions are euthanized using gaseous carbonic acid.

Group assignment

The group assignment is conducted after stratification based on the body weight using a computing system (IBUKI, Nihon Bioresearch Inc.) such that the average body weights of the groups are almost identical by random sampling. Excess animals after the group assignment are euthanized using gaseous carbonic acid on the day of group assignment.

Identification of individual animals

Animals are identified by a combination of marking on the tail with an oil-based ink and application of a dye on a limb on the day of acquisition. After the group assignment, animals are identified by labeling of their tails with the animal numbers with an oil-based ink in the same color as their cage labels (except the sham operation group, which is labeled with a black ink). The cages are labeled with labels on which the test numbers, the acquisition date and the animal numbers for quarantine inspection and acclimation are written during the quarantine inspection and acclimation period and labeled with labels in different colors between the groups on which the test numbers, the group names, the doses, and the animal numbers are written after the group assignment.

Environmental conditions and maintenance of animals

Animals are maintained in a breeding room (Room 9 in Building E except during the quarantine inspection period, in which animals are maintained in Room 10 in Building E) maintained under the following conditions: controlled temperature: 20.0 to 26.0°C, controlled humidity: 40.0 to 70.0%, light and dark for 12 hours each (lighting: 6:00 a.m. to 6:00 p.m.), and the number of ventilation: 12 times/h (with fresh air through a filter). Up to 10 animals per cage or up to 5 animals per cage after the group assignment are maintained in groups in plastic cages (W: 310 × D: 360 × H: 175 mm) in which an autoclaved floor sheet is placed. The feeders are changed once or more in 2 weeks and the water supply bottles and the plastic cages are changed twice or more per week. Cleaning and sterilization of the animal breeding room are conducted every day.

Feeding-stuff

Solid feed (MF, Oriental Yeast Co., Ltd.) containing no milk protein within 5 months after the production was placed in feeders and animals are fed freely. It is confirmed that the concentrations of pollutants, the number of bacteria, and the content of nutritional ingredients in feeding-stuff meet the standards of the test facilities for all lots of the feeding-stuff used.

Analysis organization: Eurofins Scientific Analytics (pollutants) and Oriental Yeast Co., Ltd. (the number of bacteria and nutrition ingredients)

Route of administration

Vehicle (0.5% MC) and positive control substance: oral

Forced oral administration is conducted using a disposable syringe (Terumo Corporation) made of polypropylene with a disposable oral probe (Fuchigami Kikai Co., Ltd.) for mice. At the time of the administration operation, each administration sample is mixed by inversion for each animal and aspirated in a syringe.

Dosing amount of liquid: calculated as 10 mL/kg from the body weight on the day of administration
Dose frequency: total 11 times of once a day Time of administration: between 9:00 a.m. and 12:00 a.m., but administration on the day of β amyloid injection and the day of examination is as follows.

On the day of β amyloid injection, administration is conducted after the β amyloid injection (after emergence).
On the day of examination, administration is conducted about 1 hour (+10 minutes) before the measurement. Reason of selection: it is a usual method used in the test facilities.

Vehicle (5% trehalose solution) and HVJ-E: Subcutaneous (dorsocervical area)

Subcutaneous administration in the dorsocervical area is conducted using a disposable syringe (Terumo Corporation) made of polypropylene with a disposable needle (Terumo Corporation). At the time of the administration operation, each administration sample is mixed by inversion for each animal and aspirated in a syringe.

Dosing amount of liquid: calculated as 5 mL/kg from the body weight on the day of administration
Dose frequency: total 6 times of once in 2 days.
Time of administration: between 9:00 a.m. and 12:00 a.m., but administration on the day of β amyloid injection and the day of examination is as follows.

On the day of β amyloid injection, administration is conducted after the β amyloid injection (after emergence).
On the day of examination, administration is conducted about 1 hour (+10 minutes) before the measurement.

Vehicle (PBS) and anti-PD-1 antibody: intraperitoneal

Intraperitoneal administration is conducted using a disposable syringe (Terumo Corporation) made of polypropylene with a disposable needle (Terumo Corporation). At the time of the administration operation, each administration sample is mixed by inversion for each animal and aspirated in a syringe.

Dosing amount of liquid: 0.25 mL per animal.

Dose frequency: Total twice of once on Day 1 of administration and once on Day 4 of administration. Time of administration: between 9:00 a.m. and 12:00 a.m. Reason of selection: It is a usual method used in the test facilities.

[Table 2]

Group configuration and dose

|  | Name of group | Dose | Route of administration | Number of animals |
|---|---|---|---|---|
| 1. | Vehicle control* | | Oral | 8 |
| 2. | HVJ-E∗∗ | 10,000 mNAU/kg | Subcutaneous + intraperitoneal | 8 |
| 3. | Anti-PD-1 antibody*** | 250 µg/body | Intraperitoneal + subcutaneous | 8 |
| 4. | HVJ-E + Anti-PD-1 antibody | 10,000 mNAU/kg + 250 µg/body | Subcutaneous + intraperitoneal | 8 |
| 5. | Donepezil | 0.5 mg/kg | Oral | 8 |

*0.5% MC is orally administered.
** HVJ-E is subcutaneously administered and the vehicle (PBS) is intraperitoneally administered.
*** The anti-PD-1 antibody is intraperitoneally administered and the vehicle (5% trehalose solution) is subcutaneously administered in the dorsocervical area.

Reason for dose setting

The doses of HVJ-E and the anti-PD-1 antibody were set at doses that were expected to provide sufficient pharmacological effects. The dose of donepezil was set based on the background data at the test facilities at a dose that was expected to provide a sufficient pharmacological effect.

Items of observation and examination

Experimental schedule

The starting day of the administration of the administration sample is referred to as Day 1 of administration and β amyloid is injected on Day 3 of administration. Subsequently, the Y-maze test is conducted on Day 9 of administration, the passive avoidance test is conducted on Day 10 to 11 of administration, the brain is extirpated after the passive avoidance test (retention trial).

General conditions

General conditions are observed before the administration once a day.
When a mouse is dying (a state in which the self-motion is decreased, the breathing and/or the pulse are abnormal, the body temperature is lowered, the animal is in a side-lying position, a prone position, or the like, and the response to external stimulation is decreased), the state is determined as a humanitarian endpoint and the animal is euthanized using gaseous carbonic acid.

Measurement of body weight

The body weight is measured (an electronic balance: one of PB3002, PG2002-S, PB3002-S/FACT, and MS1602S/02 is used, Mettler Toledo International Inc.) every day of administration. The body weight is measured before the administration.

Injection of β amyloid

40 mg/kg of pentobarbital sodium (Tokyo Chemical Industry Co., Ltd.), is intraperitoneally administered (dosing amount of liquid: 10 mL/kg) to animals to anesthetize the animals. After anesthesia, levobupivacaine hydrochloride (Popscaine (R), 0.25% injection, Maruishi Pharmaceutical Co., Ltd.) is subcutaneously administered (0.1 mL) to the scalp. Parietal hairs of the animal are removed and the head is fixed with a brain stereotaxis apparatus. The scalp is cut open after sterilization with a tincture of iodine to expose the skull and the connective tissue on the skull is removed with a cotton swab. The skull is then dried with a blower to find the position of bregma more easily. Using dental drill, a hole for inserting a stainless steel pipe is made on the skull at the position 1 mm side (the right side) and 0.2 mm posterior to bregma. The stainless steel pipe connected to a silicon tube having an outside diameter of 0.5 mm and a microsyringe is perpendicularly inserted to a depth of 2.5 mm from the bone surface. 3 μL of a β amyloid solution (6 μmol/3 μL) is injected into the cerebral ventricle with a microsyringe pump for 3 minutes. After injection, the stainless steel pipe is left inserted for 3 minutes and then taken out slowly. Subsequently, the stainless steel pipe is removed, the cranial hole is plugged with a non-absorbable bone marrow styptic (Nestop (R), Alfresa Pharma Corporation), and the scalp is sutured. The animal is removed from the brain stereotaxis apparatus and returned to the animal cage. The stainless steel pipe and the silicon tube are used after sterilization.

Y-maze test (spontaneous alternation test)

Apparatus (Figure 1).

For the test, a plastic Y-shaped maze (UNICOM) having 3 arms each diverging at an angle of 120° and having a length of 39.5 cm, in which the width of the floor is 4.5 cm and the height of the wall is 12 cm, is used. Before the measurement, the illuminance of the floor of the apparatus is adjusted to 10 to 40 Lux.

Method of measurement

The test is performed about 1 hour after the administration. An animal is placed in one of the arms of the Y-shaped maze and allowed to explore in the maze freely for 8 minutes. The order of the arms in which the animal moves within the measurement time is recorded and the number of times of moving to another arm is counted as the total number of entries. Next, combinations of 3 different arms consecutively selected by the animal are examined in this record and the number of the combinations is expressed as the spontaneous alternation. Furthermore, the spontaneous alternation rate is calculated using the following equation.

```
Spontaneous alternation rate (%) = [the number of
spontaneous alternation / (the total number of entries -
2)] × 100
```

Passive avoidance test

For the test, a step through passive avoidance response apparatus (with dark and light compartments: a product made in company, SHOCK SCRAMBLER: UNICOM) having a light compartment (W: 100 × D: 100 × H: 300 mm) and a dark compartment (W: 240 × D: 245 × H: 300 mm) which provide electrical stimulation from the grid of the floor, partitioned by a central Guillotine door is used. An animal is placed in the light compartment and the Guillotine door is calmly opened 10 seconds later. The time before the animal enters the dark compartment (response latency) is measured.
In the acquisition trial (Day 1), the Guillotine door is closed at the same time as the animal enters the dark compartment and electrical stimulation (0.2 mA, 2 sec, a scramble method) is provided. The presence or absence of cry of the animal upon the electrical stimulation is recorded. The response latency is up to 300 seconds.
The retention trial (Day 2) is terminated when the animal enters the dark compartment or retained in the light compartment for 300 seconds.

Animals that finished the retention trial are euthanized using gaseous carbonic acid.

Analysis of result

For each evaluation category, the average and the standard error of each group are calculated and analyzed as follows.

Statistical analysis

The significance test is conducted by comparison tests between 2 groups on the vehicle control group and the HVJ-E group, the vehicle control group and the anti-PD-1 antibody group, the vehicle control group and the HVJ-E + anti-PD-1 antibody group, the HVJ-E group and the HVJ-E + anti-PD-1 antibody group, the anti-PD-1 antibody group and the HVJ-E + anti-PD-1 antibody group, and the vehicle control group and the donepezil group.

For the comparison tests between 2 groups, the homoscedasticity is tested by the F test and Student's t-test is conducted when homoscedastic and Aspin-Welch test is conducted when heteroscedastic.

The level of significance is set at 5% and the results are described as less than 5% ($p < 0.05$) and less than 1% ($p < 0.01$). The significance test is conducted using a commercially available statistics program (SAS system, SAS Institute Japan Ltd.).

Results

(1) Y-maze test (the number of spontaneous alternation, spontaneous alternation rate)

The result of the Y-maze test is shown in Figure 2. The HVJ-E + anti-PD-1 antibody combined application group exhibited a significantly higher spontaneous alteration rate ($P < 0.01$) in comparison with the vehicle control group and the numerical value was equivalent to the donepezil group. Moreover, the spontaneous alternation rate of the HVJ-E + anti-PD-1 antibody combined application group exhibited a significantly higher spontaneous alternation rate in comparison with the single administration (HVJ-E group or anti-PD-1 antibody group) and the positive interaction was found in Additive interaction and Multiplicative interaction, indicating synergistic effect.

(2) Passive avoidance test (response latency)

The result of the passive test (response latency) is shown in Figure 3. The anti-PD-1 antibody group and the HVJ-E + anti-PD-1 antibody combined application group exhibited a significantly higher effect in comparison with the vehicle control group, although they were slightly inferior to the donepezil group. Moreover, the response latency of the HVJ-E + anti-PD-1 antibody combined application group was significantly different from that of the HVJ-E single administration group.

Discussion

Schenk et al. have reported that immunizing Alzheimer model mice made by overproduction of A$\beta$ with A$\beta_{1-42}$ resulted in pathological improvement (Nature 400: 173-, 1999, listed above) and behavioral improvement (Nature 408:979- and 982-, 2000). Furthermore, they have reported that peripheral administration of an anti-A$\beta$ antibody resulted in pathological improvement (Nature Med 6: 916-, 2000). Moreover, Baruch et al. have reported that administration of a PD-1 antibody to Alzheimer model mice resulted in pathological improvement (Nature Med 22: 135-, 2016, listed above).

Based on the results of this Example and the fact described above, it is considered that both of HVJ-E and the anti-PD-1 antibody (immune checkpoint inhibitor) remove A$\beta$, which is a foreign substance by activating natural immunity as well as acquired immunity, and improve pathological conditions of Alzheimer disease. However, the combined application of HVJ-E and the anti-PD-1 antibody had a significantly higher effect in comparison with single administration, and the fact suggests the possibility that mechanisms of action of both are different. Moreover, HVJ-E exhibited highly improving effect even in single administration, and the fact suggests that HVJ-E contributes to improvement of pathological conditions by a pathway other than the activation of acquired immunity alone. By this experiment, it was shown that more effective treatment of cognitive impairment and neurodegenerative diseases with accumulation of a prionoid such as A$\beta$, including Alzheimer-type dementia, may be possible by use of HVJ-E alone or in combination with an immune checkpoint inhibitor.

Example 2: Improving effect of single and combined administration of HVJ-E and anti-PD-1 antibody on learning disability in β amyloid single administration model mice (comparison between HVJ-E subcutaneous administration and intranasal administration)

The Y-maze test was conducted in the same method described in Example 1, except that a HVJ-E intranasal administration group was created in addition to the HVJ-E subcutaneous administration group. The route of administration and group configuration are shown below.

Route of administration

Vehicle (0.5% MC) and positive control substance: oral The same procedure described in Example 1

Vehicle (5% trehalose solution) and HVJ-E: Intranasal or subcutaneous

The intranasal administration is conducted by administration into a nasal cavity using a micropipet (Eppendorf AG). Subcutaneous administration is conducted using a disposable syringe (Terumo Corporation) made of polypropylene with a disposable needle (Terumo Corporation). At the time of the administration operation, each administration sample is mixed for each animal and aspirated in a syringe.
The intranasal administration is conducted under the following conditions.

Dosing amount of liquid: administration is conducted into both of the nasal cavities (10 $\mu$L/nose) with a liquid amount of 5 $\mu$L per cavity of an animal.
Dose frequency: total 9 times of once a day
The subcutaneous administration is conducted under the following conditions.
Dosing amount of liquid: calculated as 5 mL/kg from the body weight on the day of administration
Dose frequency: total 6 times of once in 2 days.

The time of either of the intranasal administration and the subcutaneous administration is between 9:00 a.m. and 12:00 a.m., but administration on the day of β amyloid injection and on the day of examination is conducted as follows.
On the day of β amyloid injection, administration is conducted after the β amyloid injection (after emergence).
On the day of examination, administration is conducted about 1 hour (+10 minutes) before the measurement.

Vehicle (PBS) and anti-PD-1 antibody: intraperitoneal

The same procedure described in Example 1

[Table 3]

Group configuration and dose

| | Name of group | Dose | Route of administration | Number of animals |
|---|---|---|---|---|
| 1. | Sham-operation | - | Intranasal + intraperitoneal | 8 |
| 2. | Vehicle control | - | Intranasal + intraperitoneal | 8 |
| 3. | HVJ-E*** | 100 mNAU/body | Intranasal + intraperitoneal | 8 |
| 4. | HVJ-E**** + Anti-PD-1 antibody | 100 mNAU/body + 250 $\mu$g/body | Intranasal + intraperitoneal | 8 |
| 5. | Donepezil | 0.5 mg/kg | Oral | 8 |
| 6. | HVJ-E***** + Anti-PD-1 antibody | 10, 000 mNAU/kg + 250 $\mu$g/body | Subcutaneous + intraperitoneal | 8 |

* The vehicle (5% trehalose solution) is intranasally administered and the vehicle (PBS) is intraperitoneally administered.
** 0.5% MC is orally administered.
*** HVJ-E is intranasally administered and the vehicle (PBS) is intraperitoneally administered.
**** HVJ-E is intranasally administered and the anti-PD-1 antibody is intraperitoneally administered.
***** HVJ-E is subcutaneously administered and the anti-PD-1 antibody is intraperitoneally administered.

Results

Y-maze test (the number of spontaneous alternation, spontaneous alternation rate)

The result of the Y-maze test is shown in Figure 4. The HVJ-E intranasal administration group exhibited significantly higher number of spontaneous alternation ($P < 0.05$) and spontaneous alternation rate ($P < 0.01$) in comparison with the vehicle control group and the numerical value was equivalent to the donepezil group. Meanwhile, in the combined application with the anti-PD-1 antibody, the HVJ-E (intranasal administration) + anti-PD-1 antibody administration group was not significantly different from the vehicle control group, but the HVJ-E (subcutaneous administration) + anti-PD-1 antibody administration group had a significantly higher spontaneous alternation rate ($P < 0.01$) in comparison with the vehicle control group.

Discussion

In the experiments described in this Example, it was confirmed that learning disability of mice was markedly improved in the HVJ-E intranasal administration group (the group to which the anti-PD-1 antibody was not administered) in comparison with the vehicle administration group. Moreover, in the experiments described in this Example, it was confirmed that the effect of combined application of HVJ-E and the anti-PD-1 antibody was different between intranasal administration and subcutaneous administration. HVJ-E is considered to attract NK cells, CD4+ T cells, and CD8+ T cells to the tumor lesion by induction of chemokine production from tumor cells, dendritic cells, and macrophages and induce the Th1 dominant transformation to increase antitumor effect of NK cells and Effector T cells (Cancer Res. 2007). Moreover, it has been also reported that HVJ-E acts also on neutrophiles and induces the N1 dominant transformation to increase the antitumor effect (Chang CY, et al., Oncotarget. 2016 Jul 5; 7 (27): 42195-42207). PD-1 is considered to be expressed mainly in lymphocytes such as T cells and B cells as well as bone marrow cells and macrophages (Yuan B, et al., Immunol Lett. 2016 Nov;179: 114-121), and HVJ-E is also considered to act on various kinds of immune cells as described above. It is considered that mucosa immunity and systemic immunity are simultaneously activated by intranasal administration of HVJ-E and HVJ-liposome (Yasuoka E, et al., J Mol Med (Berl). 2007 Mar; 85 (3): 283-92, Sakaue G, et al., J Immunol. 2003 Jan 1, 170 (1) 495-502). It seems that the difference between intranasal administration and subcutaneous administration is related to this mechanism of action of HVJ-E. In either case, HVJ-E is expected to be capable of more effectively treating cognitive impairment and neurodegenerative diseases with accumulation of a prionoid such as A$\beta$, including Alzheimer-type dementia by optimizing the formulation and the route of administration.

Example 3: Improving effect of single intranasal administration of HVJ-E on learning disability in the $\beta$ amyloid single administration model mice (dose-response test)

In Examples 1 and 2, single and combined administration of HVJ-E and an anti-PD-1 antibody to animals in which learning disability has been induced by $\beta$ amyloid single intraventricular administration exhibited more improving effect in the Y-maze test (short-term memory impairment) than the HVJ-E single intranasal administration. In this example, to confirm the dose response of HVJ-E single intranasal administration, the improvement effect of HVJ-E single intranasal administration at various concentrations on the learning disability was evaluated in the Y-maze test using the $\beta$ amyloid single intraventricular administration mice, which is an Alzheimer disease model.

The Y-maze test was conducted in the same method described in Example 1 except that the dose of HVJ-E is changed. The route of administration and group configuration are shown below.

Route of administration

Positive control substance: oral

The same procedure described in Example 1

Vehicle (5% trehalose solution) and HVJ-E: intranasal

The intranasal administration is conducted by administration into a nasal cavity using a micropipet (Eppendorf AG). Subcutaneous administration is conducted using a disposable syringe (Terumo Corporation) made of polypropylene with a disposable needle (Terumo Corporation) as in Example 1. At the time of the administration operation, each administration sample is mixed for each animal and aspirated in a syringe.

The intranasal administration is conducted under the following conditions.

Dosing amount of liquid: administration is conducted into both of the nasal cavities (10 μL/nose) with a liquid amount of 5 μL per cavity of an animal.
Dose frequency: total 9 times of once a day
Time of administration is between 9:00 a.m. and 12:00 a.m., but administration on the day of β amyloid injection and on the day of examination is conducted as follows.

On the day of β amyloid injection, administration is conducted after the β amyloid injection (after emergence).
On the day of examination, administration is conducted about 1 hour (+10 minutes) before the measurement.

[Table 4]

Group configuration and dose

|  | Name of group | Dose | Route of administration | Number of animals |
|---|---|---|---|---|
| 1. | Vehicle control* |  | Intranasal | 8 |
| 2. | HVJ-E | 10 mNAU/body | Intranasal | 8 |
| 3. | HVJ-E | 50 mNAU/body | Intranasal | 8 |
| 4. | HVJ-E | 100 mNAU/body | Intranasal | 8 |
| 5. | Donepezil | 0.5 mg/kg | Oral | 8 |

* The vehicle (5% trehalose solution) is intranasally administered.

Results

Y-maze test (the number of spontaneous alternation, spontaneous alternation rate)

The result of the Y-maze test is shown in Figure 5. Similar to the result in Example 2, the HVJ-E (100 mNAU/body) intranasal administration group exhibited a significantly higher spontaneous alternation rate (P < 0.01) in comparison with the vehicle control group and the numerical value was equivalent to the donepezil group.
The t-test between 2 groups on the HVJ-E administration group and the Vehicle group provided p values of 0.0204 and 0.0515 respectively for 50 mNAU/body and 10 mNAU/body, indicating significant difference for 50 mNAU/body and also the same tendency for 10 mNAU/body (the p value for 100 mNAU/body was 0.0000368). From the foregoing, the dose dependent effect of the HVJ-E single intranasal administration was confirmed. Furthermore, the effect of HVJ-E is expected to be increased by optimizing the dose and the route of administration.

Industrial Availability

The present invention is useful for prevention and/or treatment of cognitive impairment and/or neurodegenerative diseases with accumulation of a prionoid.

**Claims**

1. An inactivated Sendai virus envelope for use in a method for preventing and/or treating cognitive impairment and/or a neurodegenerative disease with accumulation of a prionoid.

2. The Sendai virus envelope for use according to claim 1, wherein the Sendai virus envelope is a wild type Sendai virus envelope.

3. The Sendai virus envelope for use according to claim 1 or 2, wherein the cognitive impairment and/or a neurodegenerative disease with accumulation of a prionoid is any one selected from Alzheimer-type dementia, mild cognitive impairment (MCI), cerebral amyloid angiopathy, dementia with Lewy bodies, Parkinson's disease, tauopathy, and Creutzfeldt-Jakob disease.

4. The Sendai virus envelope for use according to claim 3, wherein the cognitive impairment and/or a neurodegenerative disease with accumulation of a prionoid is Alzheimer-type dementia.

5. The Sendai virus envelope for use according to any one of claims 1 to 4, wherein the prionoid is amyloid β.

6. The Sendai virus envelope for use according to any one of claims 1 to 5, wherein the Sendai virus envelope is applied or administered in combination with an immune checkpoint inhibitor.

7. The Sendai virus envelope for use according to claim 6, wherein the Sendai virus envelope and the immune checkpoint inhibitor are applied or administered simultaneously or sequentially.

8. The Sendai virus envelope for use according to claim 6 or 7, wherein the Sendai virus envelope and the immune checkpoint inhibitor are provided as a combination drug, a combined administration of separate agents, or a kit.

9. The Sendai virus envelope for use according to any one of claims 6 to 8, wherein the immune checkpoint inhibitor is any one or more selected from the group consisting of an anti-PD-1 antibody, an anti-PD-LI antibody, an anti-PD-L2 antibody, an anti-CTLA-4 antibody, an anti-KIR antibody, an anti-CD137 antibody, an anti-LAG-3 antibody, an anti-OX40 antibody, an anti-CD80 antibody, an anti-CD86 antibody, an anti-B7-H3 antibody, an anti-B7-H4 antibody, an anti-B7-H5 antibody, an anti-TIM-3 antibody, an anti-TIGIT antibody, and an anti-BTLA antibody.

10. The Sendai virus envelope for use according to claim 9, wherein the immune checkpoint inhibitor is an anti-PD-1 antibody, an anti-PD-LI antibody, or an anti-CTLA-4 antibody.

**Patentansprüche**

1. Inaktivierte Sendai-Virus-Hülle für die Verwendung in einem Verfahren zum Vorbeugen und/oder Behandeln einer kognitiven Beeinträchtigung und/oder einer neurodegenerativen Krankheit mit Akkumulation eines Prionoids.

2. Sendai-Virus-Hülle für die Verwendung nach Anspruch 1, wobei die Sendai-Virus-Hülle eine Wildtyp-Sendai-Virus-Hülle ist.

3. Sendai-Virus-Hülle für die Verwendung nach Anspruch 1 oder 2, wobei die kognitive Beeinträchtigung und/oder eine neurodegenerative Krankheit mit Akkumulation eines Prionoids eine beliebige ist, die aus Demenz des Alzheimer-Typs, leichter kognitiver Beeinträchtigung (MCI), zerebraler Amyloidangiopathie, Demenz mit Lewy-Körperchen, Parkinson-Krankheit, Tauopathie und Creutzfeldt-Jakob-Krankheit ausgewählt ist.

4. Sendai-Virus-Hülle für die Verwendung nach Anspruch 3, wobei die kognitive Beeinträchtigung und/oder eine neurodegenerative Krankheit mit Akkumulation eines Prionoids eine Demenz des Alzheimer-Typs ist.

5. Sendai-Virus-Hülle für die Verwendung nach einem der Ansprüche 1 bis 4, wobei das Prionoid Amyloid β ist.

6. Sendai-Virus-Hülle für die Verwendung nach einem der Ansprüche 1 bis 5, wobei die Sendai-Virus-Hülle in Kombination mit einem Immun-Checkpoint-Inhibitor angewendet oder verabreicht wird.

7. Sendai-Virus-Hülle für die Verwendung nach Anspruch 6, wobei die Sendai-Virus-Hülle und der Immun-Checkpoint-Inhibitor gleichzeitig oder aufeinanderfolgend angewendet oder verabreicht werden.

8. Sendai-Virus-Hülle für die Verwendung nach Anspruch 6 oder 7, wobei die Sendai-Virus-Hülle und der Immun-Checkpoint-Inhibitor als ein Kombinationsarzneimittel, eine kombinierte Verabreichung separater Mittel oder als ein Kit bereitgestellt werden.

9. Sendai-Virus-Hülle für die Verwendung nach einem der Ansprüche 6 bis 8, wobei der Immun-Checkpoint-Inhibitor ein beliebiger einer oder mehrere ist, die aus der Gruppe ausgewählt sind, die aus einem Anti-PD-1-Antikörper, einem Anti-PD-L1-Antikörper, einem Anti-PD-L2-Antikörper, einem Anti-CTLA-4-Antikörper, einem Anti-KIR-Antikörper, einem Anti-CD137 Antikörper, einem Anti-LAG-3-Antikörper, einem Anti-OX40-Antikörper, einem Anti-CD80-Antikörper, einem Anti-CD86-Antikörper, einem Anti-B7-H3-Antikörper, einem Anti-B7-H4-Antikörper, einem Anti-B7-H5-Antikörper, einem Anti-TIM-3-Antikörper, einem Anti-TIGIT-Antikörper und einem Anti-BTLA-Antikörper besteht.

10. Sendai-Virus-Hülle für die Verwendung nach Anspruch 9, wobei der Immun-Checkpoint-Inhibitor ein Anti-PD-1-Antikörper, ein Anti-PD-L1-Antikörper oder ein Anti-CTLA-4-Antikörper ist.

**Revendications**

1. Enveloppe du virus Sendai inactivée à utiliser dans un procédé de prévention et/ou de traitement de trouble cognitif et/ou d'une maladie neurodégénérative avec accumulation d'un prionoïde.

2. Enveloppe du virus Sendai à utiliser selon la revendication 1, dans laquelle l'enveloppe du virus Sendai est une enveloppe du virus Sendai de type sauvage.

3. Enveloppe du virus Sendai à utiliser selon la revendication 1 ou 2, dans laquelle le trouble cognitif et/ou une maladie neurodégénérative avec accumulation d'un prionoïde est l'un quelconque choisie parmi la démence de type Alzheimer, le trouble cognitif léger (TCL), l'angiopathie amyloïde cérébrale, la démence à corps de Lewy, la maladie de Parkinson, la tauopathie, et la maladie de Creutzfeldt-Jakob.

4. Enveloppe du virus Sendai pour à utiliser selon la revendication 3, dans laquelle le trouble cognitif et/ou une maladie neurodégénérative avec accumulation d'un prionoïde est la démence de type Alzheimer.

5. Enveloppe du virus Sendai à utiliser selon l'une quelconque des revendications 1 à 4, dans laquelle le prionoïde est l'amyloïde $\beta$.

6. Enveloppe du virus Sendai à utiliser selon l'une quelconque des revendications 1 à 5, dans laquelle l'enveloppe du virus Sendai est appliquée ou administrée en combinaison avec un inhibiteur de point de contrôle immunitaire.

7. Enveloppe du virus Sendai à utiliser selon la revendication 6, dans laquelle l'enveloppe du virus Sendai et l'inhibiteur de point de contrôle immunitaire sont appliqués ou administrés simultanément ou séquentiellement.

8. Enveloppe du virus de Sendai à utiliser selon la revendication 6 ou 7, dans laquelle l'enveloppe du virus de Sendai et l'inhibiteur de point de contrôle immunitaire sont fournis sous la forme d'un médicament combiné, d'une administration combinée d'agents séparés ou d'un kit.

9. Enveloppe du virus Sendai à utiliser selon l'une quelconque des revendications 6 à 8, dans laquelle l'inhibiteur de point de contrôle immunitaire est l'un quelconque ou plusieurs choisis dans le groupe constitué par un anticorps anti-PD-1, un anticorps anti-PD-L1, un anticorps anti-PD-L2, un anticorps anti-CTLA-4, un anticorps anti-KIR, un anticorps anti-CD137, un anticorps anti-LAG-3, un anticorps anti-OX40, un anticorps anti-CD80, un anticorps anti-CD86, un anticorps anti-B7-H3, un anticorps anti-B7-H4, un anticorps anti-B7-H5, un anticorps anti-TIM-3, un anticorps anti-TIGIT et un anticorps anti-BTLA.

10. Enveloppe du virus Sendai à utiliser selon la revendication 9, dans laquelle l'inhibiteur de point de contrôle immunitaire est un anticorps anti-PD-1, un anticorps anti-PD-L1 ou un anticorps anti-CTLA-4.

# FIG. 1

10

12

4.5

Cross section
of arm

39.5

(Unit: cm)

A

B

C

Example)

$$\underbrace{AC}_{1}\overset{2}{\overbrace{BA}}\underbrace{BA}\overset{4}{\overbrace{CB}}\underbrace{AB}$$

$$\text{Spontaneous alternation rate} = \frac{[\text{Number of spontaneous alternations}]}{[\text{Total number of arm entries}]-2} \times 100$$

$$= \frac{5}{10-2} \times 100 = 62.5\%$$

EP 3 560 505 B1

# FIG. 2

## A: Total number of entries

### B: Number of spontaneous alternations (number of correct answers)

Total arm entries

□Vehicle     □HVJ-E
□Anti-PD-1     □HVJ-E+Anti-PD-1
□Donepezil 0.5 mg/kg

Number of alternations

□Vehicle     □HVJ-E
□Anti-PD-1     □HVJ-E+Anti-PD-1
□Donepezil 0.5 mg/kg

## C: Spontaneous alternation rate (rate of correct answers)

% alternation

□Vehicle     □HVJ-E
□Anti-PD-1     □HVJ-E+Anti-PD-1
□Donepezil 0.5 mg/kg

Sham

*$p<0.05$,**$p<0.01$ Significantly different from Vehicle control group (Student's t-test)
#$p<0.05$ Significantly different from HVJ-E group (Student's t-test)
$$p<0.01$ Significantly different from Anti-PD-1 group (Student's t-test)

EP 3 560 505 B1

# FIG. 3

**A: Day 1 (Day 10 after administration of test substance)**

**B: Day 2 (Day 11 after administration of test substance)**

□ Group 1: Vehicle control group  □ Group 2: HVJ-E group
□ Group 3: Anti-PD-1 group  □ Group 4: HVJ-E + Anti-PD-1 group
□ Group 5: 0.5 mg/kg donepezil group

□ Group 1: Vehicle control group  □ Group 2: HVJ-E group
□ Group 3: Anti-PD-1 group  □ Group 4: HVJ-E + Anti-PD-1 group
□ Group 5: 0.5 mg/kg donepezil group

**p<0.01 Significantly different from Vehicle control group (Student's t-test)
##p<0.01 Significantly different from HVJ-E group (Student's t-test)

EP 3 560 505 B1

# FIG. 4

**A: Total number of entries**

Total arm entries

□ Sham-operation □ Vehicle
□ HVJ-E i.n. □ HVJ-E(i.n.)+Anti-PD-1
□ Donepezil 0.5 mg/kg ▨ HVJ-E(s.c.)+Anti-PD-1

times

**B: Number of spontaneous alternations (number of correct answers)**

Number of alternations

□ Sham-operation □ Vehicle
□ HVJ-E i.n. □ HVJ-E(i.n.)+Anti-PD-1
□ Donepezil 0.5 mg/kg ▨ HVJ-E(s.c.)+Anti-PD-1

times

**C: Spontaneous alternation rate (rate of correct answers)**

% alternation

□ Sham-operation □ Vehicle
□ HVJ-E i.n. □ HVJ-E(i.n.)+Anti-PD-1
□ Donepezil 0.5 mg/kg ▨ HVJ-E(s.c.)+Anti-PD-1

% alternation

$*p<0.05$, $**p<0.01$ Significantly different from Sham-operation group (Student's t-test)
$\#p<0.05$, $\#\#p<0.01$ Significantly different from Vehicle control group (Student's t-test)

# FIG. 5

## A: Total number of entries
### Total arm entries

□ Vhicle          □ HVJ-E 10 mNAU/body
□ HVJ-E 50 mNAU/body   □ HVJ-E 100 mNAU/body
□ Donepezil 0.5 mg/kg

## B: Number of spontaneous alternations (number of correct answers)
### Number of alternations

□ Vhicle
□ HVJ-E 10 mNAU/body
□ HVJ-E 50 mNAU/body
□ HVJ-E 100 mNAU/body
□ Donepezil 0.5 mg/kg

## C: Spontaneous alternation rate (rate of correct answers)
### % alternation

□ Vhicle          □ HVJ-E 10 mNAU/body
□ HVJ-E 50 mNAU/body   □ HVJ-E 100 mNAU/body
□ Donepezil 0.5 mg/kg

##p<0.01  Significantly different from Vehicle control group (Dunnetts's test)
*p<0.05, **p<0.01 Significantly different from Vehicle control group (Student's t-test)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5631237 A
- WO 2001057204 A
- JP 2002065278 A
- WO 2004035779 A
- WO 2006011600 A
- WO 2005095613 A
- WO 2004039406 A
- WO 2005094878 A
- WO 2010032764 A
- JP 2011050292 A
- WO 0157204 A, Kaneda
- WO 03014338 A
- JP 2008308440 A
- JP 2016236536 A

### Non-patent literature cited in the description

- **SCHENK et al.** *Nature,* 08 July 1999, vol. 400 (6740), 173-177
- **BARUCH et al.** *Nature Medicine,* February 2016, vol. 22 (2), 135-137
- **DZAU et al.** *PNAS,* 1996, vol. 93 (21), 11421-11425
- **KANEDA et al.** *Mol. Med. Today,* 1999, vol. 5 (7), 298-303
- **KANEDA et al.** Hemagglutinating Virus of Japan (HVJ) Envelope Vector as a Versatile Gene Delivery System. *Mol. Ther.,* 2002, vol. 6, 219-226
- *Nature,* 1999, vol. 400, 173
- *Nature,* 2000, vol. 408, 979-982
- *Nature Med,* 2000, vol. 6, 916
- *Nature Med,* 2016, vol. 22, 135
- *Cancer Res.,* 2007
- **CHANG CY et al.** *Oncotarget,* 05 July 2016, vol. 7 (27), 42195-42207
- **YUAN B et al.** *Immunol Lett.,* November 2016, vol. 179, 114-121
- **YASUOKA E et al.** *J Mol Med (Berl),* March 2007, vol. 85 (3), 283-92
- **SAKAUE G et al.** *J Immunol.,* 01 January 2003, vol. 170 (1), 495-502